Europäisches Patentamt

European Patent Office

Office européen des brevets

(19)

(11) Veröffentlichungsnummer: **0 336 446 B1**

(12) **EUROPÄISCHE PATENTSCHRIFT**

(45) Veröffentlichungstag der Patentschrift: **18.05.94**

(21) Anmeldenummer: **89106214.3**

(22) Anmeldetag: **07.04.89**

(51) Int. Cl.⁵: **C12N 15/00**, C12N 9/84, C12N 1/00

(54) **Gen und Genprodukt zur Herstellung von beta-lactamverbindungen.**

(30) Priorität: **08.04.88 AT 922/88**
**13.07.88 AT 1806/88**
**08.09.88 AT 2201/88**

(43) Veröffentlichungstag der Anmeldung:
**11.10.89 Patentblatt 89/41**

(45) Bekanntmachung des Hinweises auf die
Patenterteilung:
**18.05.94 Patentblatt 94/20**

(84) Benannte Vertragsstaaten:
**AT BE CH DE ES FR GB GR IT LI LU NL SE**

(56) Entgegenhaltungen:

**CHEMICAL ABSTRACTS, Band 105, Nr. 3, 21.
Juli 1986, Seite 195, Zusammenfassung Nr.
19557v, Columbus, Ohio, US; W. BRUNS et
al.: "Penicillin acylase: enzyme and gene
structure: a prokaryotic peptide with com-
plex proteolytic processing"**

(73) Patentinhaber: **BIOCHEMIE GESELLSCHAFT
M.B.H.**

**A-6250 Kundl(AT)**

(72) Erfinder: **Knauseder, Franz
Oberndorf 306
A-6322 Kirchbiehl(AT)**
Erfinder: **Leitner, Ernst
Daxerfeld 5
A-6250 Kundl(AT)**
Erfinder: **Palma, Norbert
Kleinsöll 127
A-6250 Breitenbach(AT)**
Erfinder: **Weber, Gerhard**

**A-6322 Unterlangkampfen 437(AT)**

(74) Vertreter: **Kleine Deters, Johannes et al
Sandoz AG
Patentabteilung
CH-4002 Basel (CH)**

EP 0 336 446 B1

CHEMICAL ABSTRACTS, Band 68, Nr. 25, 17. Juni 1968, Seite 10755,Zusammenfassung Nr. 111628j, Columbus, Ohio, US; B. SPENCER: "The biosynthesis of penicillins; acylation of 6-aminopenicillanic acid"

JOURNAL OF BIOTECHNOLOGY, Band 3, 1986, Seiten 187-195, Elsevier Science Publishers B.V., Amsterdam, NL; J.L. GARCIA et al.: "An improved method to clone penicillin acylase genes: Cloning and expression in Escherichia coli of penicillin G acylase from Kluyvera citrophila"

GENE, Band 49, Nr. 1, 1986, Seiten 69-80, Elsevier Science Publishers B.V.,Amsterdam, NL; J.L. BARBERO et al.: "Complete nucleotide sequence of the penicillin acylase gene from Kluyvera citrophila"

CHEMICAL ABSTRACTS, Band 100, Nr. 7, 13. Februar 1984, Seite 245,Zusammenfassung Nr. 47525y, Columbus, Ohio, US; R.G. KOGEKAR et al.:"Biosynthesis of penicillin in vitro. Part II. Purification and properties of 6-aminopenicillanic acid-phenylacetyl-CoA/phenoxyacetyl-CoA transferase"

## Beschreibung

Die Erfindung betrifft Gen und Genprodukt eines in seiner Struktur und multivalenten Funktionalität neuen Schlüsselenzyms, welches die letzte Stufe der Biosynthese von Penicillin G und Penicillin V katalysiert, in gereinigter und isolierter Form.

Bereits 1953 beobachtete Kato (J. Antibiot. Ser. Tokyo 6, 130-136, 1953) in precursorlosen Penicillinfermentationen eine Akkumulierung des Penicillinnukleus. Dieses $\beta$-Lactam wurde später von Batchelor et al. (Nature 183, 257-258, 1959) isoliert und als 6-Aminopenicillansäure (6-APA) identifiziert. 6-APA wird besonders in precursorlosen Penicillinfermentationen angehäuft. Erickson und Bennett (Appl. Microbiol. 13, 738-742, 1965) betrachteten 6-APA als Nebenmetaboliten, der durch emzymatische 7-Desacylierung von Isopenicillin N in Abwesenheit von Phenyl- oder Phenoxyessigsäure als Precursor entsteht bzw. durch enzymatische Spaltung der natürlichen hydrophoben Penicilline gebildet wird. Später konnte nachgewiesen werden, daß die letzte Stufe der Penicillinbiosynthese in einem Austausch der L-$\alpha$-Aminoadipinsäureseitenkette von Isopenicillin N gegen Phenyl- oder Phenoxyessigsäure besteht, wobei die Seitenkettensäuren als Acyl-Coenzym A-Verbindungen in die Reaktion eintreten. Ein Enzym, das diesen Acyltransfer katalysiert, wurde von Loder (Postepy Hig. Med. Dosw. 26, 493-500, 1972) in Rohextrakten von P. chrysogenum nachgewiesen und als Penicillinacyltransferase (PAT) bezeichnet. Dieses Enzym wird in allen penicillinbildenden Pilzen gefunden und ist intrazellulär lokalisiert. Die Arbeiten von Fawcett et al. (Biochem. J. 151, 741-746, 1975) bestätigen die Transacylierung von Isopenicillin N zu Penicillin G mit Enzym-Rohextrakten aus P. chrysogenum in Gegenwart von Phenylacetyl-Coenzym A.

Die Bedeutung der PAT für die Biosynthese von Penicillin G oder V ist durch die Beobachtungen von Brunner et al. (Hoppe-Seyler's Z. Physiol. Chem. 349, 95-103, 1968), Spencer (Biochem. Biophys. Res. Commun. 31, 170-175, 1968), Gatenback und Brunsberg (Acta Chem. Scand. 22, 1059-1061, 1968), Spencer und Maung (Biochem. J. 118, 29-30, 1970), Meesschaert et el. (J. Antibiotics 33, 722-730, 1980), Kogekar et el. (Indian J. of Biochem. and Biophys. 20, 208-212, 1983), Frederiksen et el. (Biotechn. Letters 6, 549-554, 1984), Abraham (in Regulation of Secondary Metabolite Formation, Kleinkauf et al., eds., Proceedings of the 16th Workshop Conferences Hoechst, Gracht Castle, 12.-16.5.85, Vol. 16, Weinheim, p. 115-132, 1986), Luengo et al. (J. Antibiotics 39, 1565-1573 sowie 1754-1759, 1986) sowie Alvarez et al. (Antimicrob. Agents and Chemotherapy 31, 1675-1682, 1987) erhärtet. Das thiolabhängige Enzym kann auch den Acylaustausch innerhalb verschiedener Penicilline katalysieren und ist in Penicillinstämmen mit hoher Syntheseleistung im vermehrten Maße gegenüber niedrig produzierenden Stämmen anzutreffen (Pruess und Johnson, J. Bact. 94, 1502-1508. 1967).

Die vorliegende Erfindung betrifft die Isolierung, Charakterisierung und Aufklärung der Sequenz von Gen und Genprodukt dieses wichtigen Biosyntheseenzyms, womit wesentliche Voraussetzungen für gentechnologische Arbeiten sowohl in Richtung Titersteigerung als auch in Richtung Hybrid- bzw. Mutasynthese bzw. Erschließung neuer enzymatischer $\beta$-Lactam-Transformationswege gegeben sind.

Die Proteinsequenzierung ist nach Laursen, Methods Enzymology 25, 344-359 (1972) bekannt. Ebenfalls ist es heute Stand der Technik, die für Proteine kodierenden Gene zu lokalisieren (Rothstein et al., Cold Spring Harbor Symposia on Quantitative Biology, Vol. XLV, 99-105, 1981) und zu sequenzieren (Maxam and Gilbert, Methods Enzymology 65, 499-560, 1980). Das gezielte Ankoppeln eines Gens an einen starken, gegebenenfalls induzierbaren Promotor ist nach Pribnow, Biolog. Regulation and Development. Bd.1, 231-277, Plenum Press No.4 (Goldberger es al., 1979) bekannt.

Mehrere Enzyme der $\beta$-Lactambiosynthese wurden bereits in reiner Form isoliert und strukturmäßig charakterisiert. Ebenfalls wurden die kodierenden Genregionen mehrerer dieser Enzyme inzwischen isoliert, sequenziert, in entsprechende, für die Transformation geeignete Expressionsvektoren eingebaut und kloniert (z.B. die Isopenicillin N Synthetase aus Cephalosporium acremonium: Samson et al., Nature 318, 191-194, 1985; Baldwin et al., J. Antibiotics 40(5), 652-659, 1987; EP 200 425, Eli Lilly, 1985; die Isopenicillin N Synthetase aus Penicillium chrysogenum: Carr et al., Gene 48, 257-266, 1986; EP 225 128, Eli Lilly, 1985; die Expandase aus Cephalosporium acremonium: Samson et al., Biotechn. 5, 1207-1214, 1987; sowie mehrere. die $\beta$-Lactambiosynthese katalysierende Enzyme aus Streptomyces clavuligerus: EP 233 715, Beecham, 1986). Die erste mit solchen Expressionsvektoren durchgeführte Transformation eines $\beta$-Lactam produzierenden Mikroorganismus ist ebenfalls bereits beschrieben (Skatrud et al., Curr. Genet. 12(5), 337-348, 1987).

Die PAT, welche den letzten Schritt in der Penicillinbiosynthese, nämlich die Transacylierung von Isopenicillin N. bzw. dessen Spaltung zu 6-APA sowie deren Acylierung zu Penicillin nach folgendem Reaktionsschema katalysiert, wurde in penicillinproduzierenden Pilzstämmen bereits früher (siehe oben) nachgewiesen und in ihrer multivalenten katalytischen Funktionalität beschrieben, konnte jedoch bisher infolge ihrer extremen Instebiltität nicht nachweisbar rein isoliert und strukturmäßig charakterisiert werden. Die in penicillinbildenden Stämmen von Penicillium chrysogenum zwischen 40 und 100 Stunden Fermentationsdauer vermehrt gebildete, hoch instabile und leicht saure, thiolabhängige PAT katalysiert einerseits den Acyltransfer von Coenzym A aktivierten fermentativen Seitenketten (z.B. Acyl-Coenzym A-Verbindungen der hydrophoben Precursorsubstanzen Phenoryessigsäure, Phenylessigsäure, Hexansäure, Octansäure usw.) auf natürliche Penicilline (z.B. Isopenicillin N, Penicillin V, Penicillin G, 6-APA usw.), sowie andererseits zwischen natürlichen Penicillinen untereinander, d.h., bei dem über einen Acylenzymkomplex laufenden Acyltransfer fungieren die natürlichen Penicilline sowohl als Acyldonator wie auch als Acylakzeptor, während die Coenzym A aktivierten hydrophoben Precursorverbindungen nur als Acyldonator in die enzymatische Reaktion eintreten können. Da der Acyltransfer im wäßrigen Milieu stattfindet, konkurriert euch Wasser als Nucleophil mit dem Acylenzymkomplex, wobei die energiereichen Acyldonatoren einfach hydrolytisch gespalten werden. Daher findet man im Kulturbrei von Penicillinfermentationen immer auch 6-APA, den $\beta$-Lactamkern der natürlichen Penicilline.

Der Nachweis der PAT kann mit üblichen Analysenverfahren, wie radioaktiven, mikrobiologischen oder chromatographischen Testmethoden, durchgeführt werden. So wurden z.B. für die Bestimmung der 6-APA-Phenoxyacetyl-Coenzym A-Acyltransferaseaktivität der PAT, welche die enzymatische Reaktion 6-APA + Phenoxyacetyl-Coenzym A → Penicillin V + Coenzym A katalysiert, folgende drei verschiedenen Testmethoden ausgearbeitet:

a) Nachweis von Penicillin V über mikrobiologischen Agardiffusionstest mit spezifisch produktsensitiven Teststämmen, z.B. hat Micrococcus luteus ATCC 9341 gegenüber Penicillin eine um mehr als drei Zehnerpotenzen größere antibakterielle Sensitivität als gegenüber 6-APA.

b) Bei Einsatz von S 35-markierter 6-APA (enzymatisch aus S 35-Penicillin G) Nachweis des extrahierbaren S 35-Penicillin V im $\beta$-Counter.

c) Nachweis des extrahierten Penicillin V mittels HPLC.

Die enzymatische Transacylierungsreaktion erfolgt in Mikrotiterplatten mit folgendem Ansatzschema:

4

| 6-APA (2,5 mg/ml im Reaktionspuffer) | 20 $\mu$l |
|---|---|
| PaCoA (ca. 15-20 mg/ml in Wasser bidest.) | 30 $\mu$l |
| Enzym bzw. Reaktionspuffer (0,1 M Phosphatpuffer pH 7,8 inkl. 1 mM DTT) | 150 $\mu$l |

Sofort bzw. zu verschiedenen Zeiten innerhalb einer einstündigen stationären inkubation werden vom Ansatz jeweils 20 $\mu$l Aliquots entnommen, in derselben Mikrotiterplatte 1:5 mit 50% wäßrigem Ethanol ( = Stoppen der Enzymreaktion) bzw. 1:5 mit 5 U/ml Penicillinase (= $\beta$-Lactamkontrolle) verdünnt und im Agardiffusionstest auf mit Micrococcus luteus ATCC 9341 bzw. mit dem lactamsupersensitiven Stamm Pseudomonas aeruginosa BC 248 beimpften Testplatten (Einsaat 0,5%, Antibiotic Medium 1 von DIFCO, 50 $\mu$l pro Näpfchen, Bebrütung 15 Stunden bei 37°C) gegen Penicillin V getestet.

Zu verschiedenen Zeiten werden vom enzymatischen Transacylierungsansatz (gleicher Ansatz wie oben mit 2 ml Gesamtvolumen) Aliquots entnommen, 1:1 mit Diisopropylether bei pH 2,0 (HCl) extrahiert, die organische Phase mit Stickstoff abgedampft, der Rückstand im gleichen Volumen Reaktionspuffer resuspendiert und über HPLC (Bedingungen wie bei der PaCoA Gehaltsbestimmung, nur als Eluens 40% Methanol und 60% 0,01 M TBAS in 0,025 M Phosphatpuffer pH 7,0) auf Penicillin V getestet.

| Retentionszeiten (min) | Phenoxyessigsäure | 1,10 |
|---|---|---|
| | Penicillosäure G | 1,62 |
| | Penicillosäure V | 1,92 |
| | Penicillin G | 3,00 |
| | Penicillin V | 4,92 |
| | 3-Desacetoxycephalosporin V | 2,46 |
| | Cephalosporin V | 3,24 |

Die anderen Aktivitäten des multispezifischen PAT-Enzyms können ebenfalls über HPLC bestimmt werden, wobei als Eluens für die polaren Substrate bzw. Produkte 0,01 M TBAS in 0,025 M Phosphatpuffer pH 7,0 alleine Verwendung findet.

| Retentionszeiten (min) | Isopenicillin N | 1,67 |
|---|---|---|
| | 6-APA | 4,00 |
| | DTT red. | 2,41 |
| | DTT oxid. | 4,60 |

Isopenicillin N kann aus precursorlosen Penicillinfermentetionen über Adsorberharz (DIAION-HP 20) und Reverse Phase Chromatographie (Nucleosil C 18, 10 $\mu$m) gewonnen werden.

Je nach Enzympräparation ist dabei besonders auf die Wahl der Indikatorreaktion zu achten, mit der die eigentliche Aktivität des multispezifischen Biosyntheseenzyms erfaßt werden soll. So ist es beispielsweise nicht besonders zielführend, als Indiketorreaktion für die PAT im Rohextrakt die 6-APA-Phenoxyacetyl-Coenzym A Acyltransferase-Aktivität zu verwenden, da in dieser rohen Enzympräparation anwesende Störaktivitäten sowohl Phenoxyacetyl-Coenzym A ( = PaCoA) und Penicillin V rasch hydrolysieren als euch 6-APA mit dem energiereichen Seitenkettenderivat PaCoA zu Penicillin V acylieren, was z.B. auch bekannte proteolytische Enzyme wie Chymotrypsin katalysieren. Diese interterierenden Aktivitäten unterscheiden sich jedoch deutlich von der eigentlichen thiolabhängigen PAT. Sie zeigen z.B. ein anderes chromatographisches Elutionsverhalten, Stabilitätsprofil und Inhibierungsmuster. Diese interferierenden Störaktivitäten erklären einerseits die bisher in Rohpräparationen gefundenen sehr niedrigen spezifischen PAT-Aktivitäten (um 1-10 $\mu$U/mg Protein) sowie ebenfalls den als paradox erscheinenden Befund, daß die 6-APA-PaCoA-Acyltransferaseaktivität im Rohextrakt mit zunehmender Enzymverdünnung bis zu einem Aktivitätsmaximum stark ansteigt. Ein essentieller Faktor im Nachweis der PAT ist die Anwesenheit von reduzierenden Verbindungen, wie Dithiothreitol oder $\beta$-Mercaptoethenol, welche des hoch oxidationsempfindliche SH-Enzym aktivieren bzw. stabilisieren.

Die hohe Temperaturinstabilität der PAT erschwert die Enzymreinigung ebenfalls wesentlich. So verliert z.B. eine frisch hergestellte Enzympräparation trotz Anwesenheit von verschiedenen Stabilisatoren (z.B. 5 mM Dithiothreitol + 5% Sorbit + 0,1 mM Phenylmethansulfonylfluorid) nach einstündiger stationärer Inkubation bei 37°C die gesamte thiolabhängige 6-APA-PaCoA Acyltransferaseaktivität. Es ist daher angebracht, alle Reinigungsarbeiten in einem Arbeitskühlraum bei 4°C unter Oxidationsschutz durchzuführen. Grob vorgereinigte Enzymlösungen können über mehrere Wochen in Anwesenheit der Stabilisatoren ohne merklichen Aktivitätsverlust eingefroren (-196°C, -20°C) werden, wobei jedoch mehrere Einfrier-Auftauschritte die PAT-Aktivität sofort vollständig desaktivieren. Ammonsulfatpräzipitate sind ebenfalls einige Tage bzw. Wochen bei 4°C in Anwesenheit der genannten Stabilisatormischung mehr oder minder stabil.

Das in der Erfindung angewandte Reinigungsverfahren der PAT liefert, ausgehend von jungem, penicillinpositivem Penicillium-Myzel, nach Myzelaufschluß, Nukleinsäurefällung, Proteinfällung, hydrophober Interaktions- und Affintätschromatographie relativ stabile, mindestens 50%ige, aktive Enzympräparationen, wie sie für die enzymkinetische bzw. proteinchemische Charakterisierung notwendig sind. Die Aufarbeitung bis zur relativ lagerfähigen Proteinpräzipitation kann mit üblichen Reinigungsverfahren durchgeführt werden, z.B. durch Zellaufschluß mit einem Hochdruckhomogenisator oder Glaskugelmühle bzw. die Nukleinsäurefällung mit Cetyltrlmethylammoniumbromid, Streptomycinsulfat oder Protaminsulfat. Für die Proteinfällung eignet sich u.a. Ammonsulfat, wobei eine 50%ige Sättigung ausreicht, um die gesamte nachweisbare PAT-Aktivität zu präzipitieren. Vom Ammonsulfatpräzipitat ausgehend, wird die thiolabhängige PAT an Phenylsepharose Cl 4 B über hydrophobe Interaktion gebunden und nach Entfernung von über 90% des Ballastproteins mit einer wäßrigen Stabilisatorlösung niedriger Ionenstarke eluiert, über eine 10 kD cut off Ultrafiltrationsmembran konzentriert und vorzugsweise über Affinitätschromatographie bzw. alternativ nach einem Gelfiltrationsschritt (Ultrogel Aca 54) über Anionenaustauscher-(DEAE-Sepharose FF) bzw. Adsorptionschromatographie (Hydroxylapatit) bis zu einer mindestens 50%igen aktiven Enzympräparation weitergereinigt, was einem Anreicherungsfaktor von ca. 1000, bezogen auf das Ammonsulfatpräzipitat, entspricht.

Als Affinitätsmatrix können übliche makroporöse Trägermaterialien wie Sepharose, Zellulose, Polymermaterialien, stabilisiertes Kieselgel, Aluminiumoxid usw. eingesetzt werden. Als Affinitätsliganden, welche über einen $C_2$ bis $C_{10}$-Spacer bzw. ohne Spacer kovalent oder durch hydrophobe bzw. ionogene Wechselwirkung an den Träger gebunden werden, eignen sich vorzugsweise Acyl- bzw. Amino-$\beta$-lactamverbindungen. Die Bindung dieser Liganden an den Spacer bzw. die Matrix muß derart erfolgen, daß die Bindungsstelle am Liganden für das zu reinigende Enzym zugänglich bleibt. Erfindungsgemäß zeigen besonders Affinitäsmatrices mit terminalen Amino-Spacern wie AH-Sepharose 4 B bzw. HMD-Ultrogel Aca 34, an die über N-Ethoxycarbonyl-2-ethoxy-1,2-dihydrochinolin (EEDQ) Liganden wie natürliche Penicilline, vorzugsweise 6-APA, Penicillin V oder Penicillin G bzw. analoge stabile 3-Desacetoxycephalosporinverbindungen wie 7-Amino-3-desacetoxycephalosporansäure (7-ADCA) bzw. 7-Phenoxyacetamido- oder 7-Phenylacetamido-3-desacetoxycephalosporansäure (3-Desacetoxycephalosporin V und G) gebunden werden, einen besonders starken Reinigungseffekt. Die Amino-$\beta$-lectamaffinitätsmatrices können dabei ohne aufwendige Schutzgruppentechnologie mit löslicher Penicillin G-Acylase von E. coli aus den entsprechenden Phenylacetamido-$\beta$-lactammatrices hergestellt werden.

Die benötigten Mikroorganismen werden für die Enzymproduktion in üblichen Nährmedien für die Penicillinbildung herangezüchtet. Als Nährmedienbestandteile eignen sich alle Substrate, die ganz allgemein zur Züchtung von Pilzen verwendet werden. Zusätzlich zu diesen Nährstoffen kann man in geeigneter Kombination jene Additive verwenden, die das Wachstum der Mikroorganismen fördern und die PAT-Aktivität steigern. Additive sind z.B. Magnesiumsulfat, Natriumchlorid, Calciumcarbonat, Phosphate und ähnliche anorganische Salze sowie Wuchsstoffe, Vitamine und Spurenelemente. Durch Zusatz verschiedener Induktoren, vorzugsweise Phenoxy- bzw. Phenylessigsäure und derer Derivate oder Analoge. können die erzielten Enzymtiter erheblich gesteigert werden.

Die Anzucht erfolgt vorwiegend submers unter aeroben Bedingungen bei einer Temperatur im Bereich von 15 bis 35°C und einem pH-Wert zwischen 4 und 8. Die Zeit, in der die Kulturbrühe die maximale Enzymaktivität erreicht, hängt von der Art des verwendeten Mikroorganismus ab, und die optimale Zeit für die Kultivierung ist daher vorzugsweise für jeden einzelnen Stamm getrennt zu bestimmen. Im allgemeinen liegt die Kultivierungsdauer im Bereich von vorzugsweise 2 bis 5 Tagen. Für die Transacylierung kann man die Kulturbrühe bzw. ein daraus bereitetes aktives Sekundärpräparat einsetzen. Beispiele für solche aktive Sekundärpräparate sind die aus der Kulturbrühe gewonnenen und gewaschenen, nativen bzw. permeabilisierten Zellen; zeltfreie Extrakte, die man durch physikalische, chemische und enzymatische Behandlung des Myzels erhält (beispielsweise die Zellhomogenate, die man durch Aufschluß oder durch Ultraschallbehandlung des Myzels erhält, und Zellysate, die man durch Behandlung mit oberflächenaktiven Substanzen oder Enzymen bildet); teilweise oder vollständig gereinigte Präparate des gewünschten Enzyms, die man durch Reinigen der zellfreien Extrakte mit Hilfe üblicher Enzymreinigungsmethoden erhält, beispielsweise durch Aussalzen, durch fraktionierte Fällung, durch Dialyse, durch Gelfiltration, durch Ionenaustausch-, Adsorptions- und durch Affinitätschromatographie; stabilisierte PAT-Präparate, die man dadurch erhält, daß man das Enzym bzw. die nativen bzw. permeabilisierten Zellen entweder physikalisch oder chemisch mit wasserunlöslichen hochmolekularen Trägersubstanzen durch Adsorption, Kovalentbildung, Quervernetzen, Einschluß oder Einkepseln immobilisiert.

Die enzymkinetische bzw. physiochemische Charakterisierung der oben resultierenden Enzymkonzentrate kann mit klassischen Methoden wie Chromatographie, Elektrophorese, Untersuchungen hinsichtlich Substratspezifität, N-terminale Sequenzierung` pH- und Temperaturprofil für Aktivität und Stabilität, Aktivierungs-, Inhibierungs- und Stabilisierungsstudien usw. durchgeführt werden.

Für die Isolierung von Genen existieren zahlreiche Methoden. Einen allgemeinen Überblick dazu geben die Bücher von Watson et al. (Rekombinierte DNA - Eine Einfuhrung, Heidelberg, 1983) und Winnacker (Gene und Klone - Eine Einführung in die Gentechnologie, Weinheim, 1984). Im Falle des pat-Gens (Gen für das Enzym PAT) scheint es am sinnvollsten, synthetische DNA-Sonden zu verwenden, da die dazu notwendigen Aminosäuresequenzen vorliegen. Dazu kann man aus der Aminosäuresequenz des Polypeptides 1 der PAT eine DNA-Sequenz auf Grund des genetischen Kodes ableiten und die entsprechenden Oligonukleotide chemisch synthetisieren. Abbildung 5 zeigt die Anordnung der Aminosäureresten 2 bis 22 des Polypeptides 1 der PAT. Da einigen Aminosäuren 2, 3, 4 oder 6 Kodons zugeordnet werden, ergibt sich eine Nukleotidsequenz, wie sie unter der Aminosäuresequenz angegeben ist. Der Abschnitt, der den Aminosäureresten 16 bis 21 entspricht, wäre z.B. gut geeignet, da hier nur 16 verschiedene Gensequenzen möglich sind. Diese 16 Oligonukleotide, welche der RNA komplementär sind, kann man als vier Gemische mit je vier Oligonukleotiden synthetisieren (Oligonukleotidgemische 1, 2, 3 und 4 in Abbildung 5). Eine sichere Identifizierung des Gens ist mit diesen Oligonukleotiden vermutlich nicht möglich, da im Genom von Penicillium chrysogenum weitere DNA-Sequenzen vorkommen, die diesen DNA-Sequenzen sehr ähnlich sind. Eine Unterscheidung dieser unterschiedlichen DNA-Sequenzen kann methodisch sehr aufwendig sein. Deshalb ist es ratsam, über ein weiteres Oligonukleotid als Suchsequenz zu verfügen

Die Strategie, Gene mit Hilfe von DNA-Sonden zu isolieren, wird von Lathe (J. Mol. Biol. 183, 1-12, 1985) diskutiert. Sind z.B. Informationen über die Häufigkeit bestimmter Kodons oder bestimmter Dinukleotidfolgen bekannt, kann eine längere DNA-Sequenz synthetisiert werden. Für Penicillium chrysogenum sind solche Informationen nicht verfügbar. Um trotzdem ein längeres Oligonukleotid als Sonde zu erhalten, kann man die vorhandenen Oligonukleotide als Sequenzierprimer verwenden: Das Enzym Reverse Transcriptase kann einen DNA-Strang sysnthetisieren, der zu einer vorgegebenen RNA komplementär ist, aber nur dann, wenn ein geeignetes Startermolekül (= Primer) vorhanden ist. Im beschriebenen Fall können als Primer die Oligonukleotidgemische 1 bis 4 eingesetzt werden. Dadurch wird es möglich, nur die pat mRNA zu sequenzieren, obwohl zahlreiche andere mRNAs in der Präparation vorhanden sind. Die Sequenzierreaktion wird in Gegenwart von basenspeziflschen Kettenabbruch-Reagentien durchgeführt (Dideoxynucleosid-Triphosphate). Dadurch entstehen zahlreiche verlängerte Oligonukleotide, deren gelelektrophoretische Analyse es erlaubt, die Basenabfolge einer DNA zu ermitteln, die zur pat mRNA komplementär ist. Der Vorteil dieser Methode ist, daß die erhaltene Sequenzinformation erlaubt, direkt neue Oligonukleotide zu synthetisieren. Zusätzlich ist es sehr bedeutsam, daß diese Sequenz mit der Sequenz, die aus der Aminosäuresequenz abgeleitet wird, übereinstimmen muß und so absichert, daß tatsächlich eine pat-spezifische Sequenz vorliegt.

Die eigentliche Isolierung des Gens kann dann aus einer Genbank erfolgen. Eine Genbank ist eine Sammlung rekombinanter DNA-Vektoren, die jeweils einen kleinen Teil der DNA von Penicillium chrysogenum enthalten (im Falle einer λ-EMBL3-Genbank sind dies je rekombinantem Molekül ca. 0.05%). Mittels Plaquehybridisierung kann man λ-Klone isolieren, die mit der radioaktiv markierten DNA-Sonde hybridisieren. Von der DNA dieser Klone kann eine physikalische Karte angefertigt werden. Eine solche Restriktionskarte gibt die Anordnung von Restriktionsendonucleas-Schnittstellen an, die als Orientierungshilfe dienen können. Durch DNA-Hybridisierungstechniken läßt sich ermitteln, mit welchem Teilbereich der DNA die DNA-Sonden hybridisieren. Die entsprechenden Tellfragmente werden in Plasmid- oder Bakteriophagen-Vektormoleküle eingebaut und mit molekulargenetischen Methoden weiter charakterisiert. Die Einklonierung eines DNA-Segmentes in den Vektor M13mp19 erlaubt dann die Bestimmung der DNA-Sequenz, wobei die pat-spezifische DNA-Sonde als Primer verwendet werden kann. Eine weitere Sequenzierung der DNA erlaubt es, den kodierenden Anteil des Gens und die daraus abgeleitete Aminosäuresequenz der PAT zu ermitteln. Die bereits bestimmten Aminosäure-Teilsequenzen können die dazu erforderliche information liefern.

Das pat-Gen aus Penicillium chrysogenum kann auf Grund der DNA-Sequenzinformation in einen Expressionsvektor eingebaut werden, der eine gute Expression der PAT in gut bekannten Expressionsorganismen erlaubt (Reznikoff und Gold, Meximizing Gene Expression, Boston, 1986). Um eine präzise Konstruktion zu ermöglichen, bei der des ATG genau in die Position des E.coli Startkodons kommt, kann man durch ortsspezifische ("site directed") Mutagenese eine NcoI-Schnittstelle einführen. Vorher muß jedoch ein vollständiger cDNA-Klon aus einer entsprechenden Genbank isoliert werden. Beispiel 19 (siehe auch Abbildung 7) beschreibt die Details der Konstruktion des Plasmides pBC2001. Dazu wird zunächst das DNA-Fragment aus einem pat-cDNA-Klon in M13mp19 kloniert. Damit steht eine einzelsträngige DNA zur Verfügung, die für die ortsspezifische Mutagenese zur Einführung der NcoI-Schnittstelle verwendet wird; außerdem eine HindIII-Schnittstelle, die für die Klonierung nötig ist. Das Plasmid pBC2001 entsteht, wenn man das NcoI-HindIII-Fragment in den Vektor pKK233-2 einbaut. Es enthält effektive Signale für die Expression in E.coli (ptrc, rrnBT1T2) und ein Ampicillin-Resistenzgen (bla), als E.coli-Selektionsmarke.

Die DNA des pat-Gens kann in Vektoren eingebaut werden, die eine Transformation von Penicillium chrysogenum oder von anderen $\beta$-lactamproduzierenden Mikroorganismen erlauben. Da Transformanten häufig die eingebrachte DNA in mehreren Kopien enthalten (z.B. Kolar et al.), ist eine stärkere Expression des Gens möglich, die zu einer gesteigerten Penicillinbildung führen kann. Im Beispiel 20 ist die Konstruktion des Plasmides pBC2002 beschrieben, das zur Transformation von Penicillium chrysogenum verwendet werden kann. pBC2002 (siehe auch Abbildung 8) enthält das vollständige Penicillium chrysogenum pat Gen auf einem 4,8kb SalI Restriktionsfragment, das in den modifizierten Vektor pHS103 eingebaut wurde. Das Phleomycin-Resistenzgen (ble) erlaubt die Selektion in Penicillium chrysogenum; das Ampicillin-Resistenzgen (bla) die Selektion in E.coli.

Weiterhin ist die Manipulation der DNA möglich. So kann der Austausch von Promotorabschnitten ebenfalls die Transkription in Penicillium chrysogenum verbessern. Eine ortsspezifische ("site directed") Mutagenese des ATGs kann die Translation in Penicillium chrysogenum steigern. Der Austausch spezieller Aminosäurereste durch ortsspezifische oder fragmentspezifische - dann jedoch ungerichtet, aber genspezifisch - Mutagenese kann es ermöglichen, spezielle Mutanten zu isolieren, welche die Stabilität des Enzyms oder seine Aktivität oder Spezifität verändern.

Auch ein Screening von zufällig oder nicht-zufällig ausgewählten Mikroorganismen - bei dem die DNA solcher Organismen auf die spezifische Hybridisierung mit radioaktiver pat-DNA untersucht wird - ist eine naheliegende Nutzung des isolierten DNA-Abschnittes. Das Ergebnis eines solchen Screenings können Stämme sein, die bisher nicht bekannte $\beta$-Lactame produzieren, oder Stämme, die PAT-ähnliche Enzyme enthalten, die aber andere Eigenschaften aufweisen.

Die DNA der vorliegenden Erfindung wurde aus Penicillium chrysogenum isoliert. Die Vorgangsweise ist in den Beispielen 10 bis 13 beschrieben und durch die Abbildungen 4,5 und 6 illustriert. Ausgehend von der Aminosäuresequenz des Polypeptides 1 der PAT (Abb. 5) wurden vier Oligonukleotide synthetisiert (Abb. 5: Oligonukleotidgemische 1 bis 4, Abb. 6 C), die als Sequenzierprimer verwendet werden (Beispiel 11). Die ersten 29 Basen der so ermittelten Sequenz (Abb. 6 D) stimmen mit der postulierten pat mRNA-Sequenz überein (Abb. 6 B). Auf Grunde dieser Information wurde ein 30mer synthetisiert (Abb. 6 E), das als radioaktive Sonde zur Isolierung eines λ-Klons aus der Penicillium chrysogenum Genbank (Beispiele 12 und 13) verwendet wurde. Nach einer Subklonierung in M13-Vektoren wurde die DNA sequenziert (Abb. 6 F). Die ersten 77 Basen dieser Sequenz stimmen mit den Basen 35 bis 111 der zur mRNA komplementären Sequenz (Abb. 6 D) überein. Die Zusammenhänge zwischen diesen einzelnen Sequenzen demonstrieren, daß tatsächlich das pat-Gen isoliert wurde.

Die PAT hat ein Molekulargewicht von ca. 38.000 D. Aus einem mittleren Aminosäure-Molekulargewicht von 110 D läßt sich für den kodierenden Anteil des Gens eine Größe von 1100 bp schätzen. Da Gene von filamentösen Pilzen meist nur wenige und wenn, denn kleine Introns enthalten (Ballance, Yeast 2,229, 1986), kann man davon ausgehen, daß das vollständige Gen auf dem beschriebenen DNA-Molekül liegt und daß Kontrollregionen ebenfalls vollständig vorhanden sind.

Die folgenden Beispiele erläutern die Erfindung, ohne sie jedoch zu beschränken. In den Beispielen stehen die Abkürzungen ml, l, mg, g, Upm, TS, GV% und U für Milliliter, Liter, Milligramm, Gramm, Umdrehungen pro Minute, Trockensubstanz, Gewicht/Volumsprozent und Internationale Enzymeinheit (1 Internationale Enzymeinheit = 1 µMol Substrat/min). Gewichtsteile verhalten sich zu Volumsteilen wie g zu ml, die Temperatur wird in Celsiusgraden angegeben. Die Charakterisierung der Produkte wurde unter Verwendung der folgenden Techniken durchgeführt: Hochleistungs-Dünnschichtchromatographie (HPTLC), Hochdruck-Flüssigchromatographie (HPLC), Ultraviolett-Spektrophotometrie (UV), Infrarotspektrometrie (IR) und magnetische Kernresonanz (NMR). Die den in den Beispielen angeführten Stammnamen beigefügten Nummern entsprechen den eingetragenen Stammbezeichnungen jener Kultursammlungen, bei denen die Stämme als solche deponiert sind, z.B. bei ATCC (American Type Culture Collection, Rockville, Maryland, USA).

**Herstellung und Gehaltsbestimmung von Phenoxyacetyl-Coenzym A (PaCoA):**

Prinzip:

Coenzym A + Phenoxyessigsäurechlorid → PaCoA

901 mg CoA werden unter Eiskühlung in 15 ml bidestilliertem Wasser gelöst und mit Natronlauge auf pH 7,0 (Verbrauch 2,56 ml 1 N NaOH und 1,60 ml 0,5 N NaOH) gestellt. Anschließend werden in Portionen insgesamt 235 mg Phenoxyessigsäurechlorid unter kräftigem Rühren zugesetzt und der pH-Wert auf 7,0 gehalten (Verbrauch 1,8 ml 1 N NaOH). Nach einstündigem Rühren unter Eiskühlung wird dreimal mit je 40 ml Diethylether bei pH 2,0 (6 bzw. 1 N HCl) extrahiert, dann die wäßrige Phase mit NaOH neutralisiert, im Rotationsverdampfer bei Raumtemperatur vom restlichen Ether befreit, kurz mit Stickstoff begast, anschließend 1:50 mit bidestilliertem Wasser verdünnt, in Portionen zu je 500 $\mu$l ampulliert und in Flüssigstickstoff eingefroren. Die Gehaltsbestimmung erfolgt über HPLC, wobei als Standard Phenylacetyl-Coenzym A (PeCoA) verwendet wird.

| HPLC-System: | HP 1090 Liquid Chromatogram (Hewlett Packard) |
|---|---|
| Säule: | Hypersil ODS 5 $\mu$m, C 18, 150x200 mm |
| Integrator: | Modell 3392 (Hewlett Packard) / ATT 3 |
| Fluß: | 0,5 ml/min |
| Detektion: | 230 nm |
| Eluens: | 35% Methanol |
| | 65% 0,01 M Tetrabutylammoniumsulfat (TBAS) in 0,025 M Phosphatpuffer pH 7,0 |

| Auswertung: | | | |
|---|---|---|---|
| Probe | Retentionszeit (min) | Fläche | Höhe |
| 400 $\mu$g/ml PeCoA | 9,41 | 8466900 | 210692 |
| 200 -"- | 9,68 | 4105400 | 107689 |
| 100 -"- | 9,88 | 2092100 | 54990 |
| PaCoA Stlsg. 1:200 | 10,53 | 1966600 | 47737 |
| PaCoA Stlsg. 1:50 | 10,68 | 7850700 | 172983 |
| PaCoA Stlsg. 1:40 | 10,47 | 10389000 | 222475 |
| Flächenintegration → 19,2 mg PaCoA/ml | | | |
| Höhenauswertung → 16,8 mg PaCoA/ml | | | |
| Mittelwert → 18,0 mg PaCoA/ml | | | |

**Beispiel 1: Heranführung der enzymatisch aktiven Biomasse:**

Der Inhalt einer Ampulle mit lyophilisierten Sporen Von Penicillium chrysogenum P2 / ATCC 48271 wird in 8 ml Medium A (Zusammensetzung pro Liter: 15 g Lactose, 0,11 g Stickstoff aus Cornsteep Liquor, 5 g Witte Pepton, 4 g NaCl, 0,5 g $MgSO_4.7H_2O$, 0,6 g $KH_2PO_4$, 5 mg $FeCl_3.6H_2O$, 2 mg $CuSO_4.5H_2O$, alles ad 1000 ml mit $H_2O$ destilliert, pH 4,85, Sterilisation: 20 min bei 120°) suspendiert. Mit 2 ml dieser Sporensuspension wird ein 2 l Erlenmeyerkolben mit 225 ml Gerste beimpft. Die Gerste wird vor dem Abfüllen mit Wasser klar gewaschen, 20 bis 30 Minuten in Medium A gequollen, über ein Sieb filtriert und ca. eine Stunde auf Filterpapier getrocknet, anschließend in 2 l Erlenmeyerkolben zu je 225 ml abgefüllt, dreimal bei 100°, jeweils ca. eine Stunde, mit eintägigen Intervallen sterilisiert und vor dem Animpfen noch 2 Tage bie 40 bis 45° getrocknet. Nach der Sporeneinsaat werden die Erlenmeyerkolben kurz geschüttelt, dann ungefähr 8 Tage bei 24 ± 1° und 60 ± 10% relativer Luftfeuchtigkeit inkubiert.

Nach dieser stationären Bebrütungszeit werden die Pilzsporen mit 250 ml 0,9% NaCl + 0,1% Tween 80 auf einem vertikalen Schüttler für 3 bis 5 Minuten bei 140 Upm resuspendiert. Nach erneutem Zusatz von 250 ml 0,9%iger NaCl-Lösung (ohne Tween 80) wird die Sporensuspension in eine 1 l Impfkanne steril dekantiert. Mit je 8 ml von diesem bei 4° ca. einen Monat lagerfähigem Impfgut werden jeweils 50 Stück 2 l Erlenmeyerkolben zu je 200 ml Medium B (Zusammensetzung pro Liter: 9 g Kaliumphenoxyacetat, 150 g Lactose Monohydrat, 2,25 g Stickstoff als Pharmamedia, 10,5 g $(NH_4)_2SO_4$, 4 g $Na_2SO_4$, 0,5 g $KH_2PO_4$, 10 ml tierisches Öl (Lardöl), 25 g $CaCO_3$, alles ad 1000 ml mit $H_2O$ destilliert; pH 6,5, Sterilisation: 20 min bei 120°) beimpft und drei Tage bei 25 ± 1° bei 260 Upm geschüttelt. Nach dieser Schüttelinkubation werden 1820 g an enzymatisch aktiver Biomasse geerntet. Der Penicillintiter beträgt 1,9 g Penicillin V pro Liter Brei.

**Beispiel 2: Herstellung einer enzymatisch aktiven Enzymrohpäparation:**

1786 g feuchtes Myzel (= 220 g TS), erhalten gemäß Beispiel 1, werden in 3,5 l 0,1 M Phosphatpuffer pH 7,5 inklusive 5 mM Dithiothreitol (DTT), 0,1% Triton X 100 sowie 0,1 mM Phenylmethansulfonylfluorid (PMSF) resuspendiert und unter Kühlung bei 2/3° kontinuierlich in der Glaskugelmühle (600 ml Stahimahlbehälter mit 510 ml 500-750 $\mu$m Glasperlen, Drehzahl 2000 Upm, 0,1 mm Reibspaltbreite, Durchfluß 30 l/Stunde, Vorlauf 2 bis 3°, Rücklauf 5°, Solekühlung mit Vorlauf -20/8° und Rücklauf -5/-1°) homogenisiert. Das Homogenat wird anschließend 30 Minuten bei 15.000 Upm und 4° zentrifigiert, der Überstand mit 0,5 GV % Cetyltrimethylammoniumbromid (CTAB) bei 0° versetzt und 30 Minuten gerührt. Die präzipitierte Nukleinsäure wird durch Zentrifugation (10 Minuten, 15.000 Upm, 4°) abgetrennt, der Überstand durch Zugabe von festem Ammonsulfat auf 50 GV % Sättigung gebracht und zur Vervollständigung der Proteinfällung über Nacht bei 4° gehalten. Der Niederschlag wird anschließend zentrifugiert, mit kalter gesättigter Ammonsulfatlösung gewaschen und nach Zusatz von 5 GV % Sorbit, 5 mM DTT, 2 mM EDTA und 0,1 mM PMSF bis zum nächsten Chromatographieschritt bei 4° gelagert. Der Aufschluß bzw. die beiden Fällungsstufen werden mit Protein- (Bradford) bzw. Aktivitätsbestimmung (mikrobiologische Erfassung der 6-APA-PaCoA-Acyltransferaseaktivität mit dem lactamsupersensitiven Stamm Pseudomonas aeruginosa BC 248) verfolgt. Die gefundenen Werte sind in der Tabelle 1 angeführt.

**Beispiel 3: Mikrobiologischer Nachweis der Transacylierungs- bzw. Penicillin V-Spaltungsaktivität in der Enzympräparätion AS-Präzipitat mit bzw. ohne Zusatz von Inhibitoren:**

Die, wie in Beispiel 2 beschrieben, hergestelite Enzymrohpräzipitation wird im mikrobiologischen Agardiffusionstest hinsichtlich Transacylierung von 6-APA bzw. Isopenicillin N mit PaCoA und Spaltung von Kalium Penicillin V mit bzw. ohne Inhibitorzusatz folgendermaßen getestet:

| | |
|---|---|
| Testkeim: | Micrococcus luteus ATCC 9341/BC 85, 0,5% Einsaat |
| Testmedium | Antibiotic Medium 1 (DIFCO), 110 ml |
| Testplatte: | Nunc 243x243x18 mm |
| Probe: | 50 $\mu$l pro Näpfchen (6 mm Durchmesser) |
| Inkubation: | 37°, 15 Stunden |
| Enzym: | AS-Präzipitat (hergestellt wie in Beispiel 2 beschrieben): 1 ml zentrifugiert (20.000 Upm/10 min), Überstand verworfen, Pellet in 20 ml RP + Z resuspendiert. |
| RP + Z: | 0,1 M Phosphatpuffer pH 7,5 + 1 mM DTT + 10 mM MgCl$_2$ |

**A. Transcylierung/Acylierung**

Ansatz in Mikrotiterplatten:

| | |
|---|---|
| Substrat 6-APA bzw. Isopenicillin N (2,5 mg/ml) | 10 $\mu$l |
| PaCoA (ca. 15 mg/ml) | 15 $\mu$l |
| Enzym bzw. RP + Z mit bzw. ohne Inhibitor | 75 $\mu$l |

Inhibitor:

a) ohne Inhibitor (= Kontrolle)

b) 2 mM Jodacetamid

c) 2 mM PMSF

Sofort bzw. nach 7, 30 und 60 Minuten Inkubationszeit werden 20 $\mu$l Aliquots der Ansätze entnommen, 1:5 mit 50% Ethanol (Stoppen der Enzymreaktion) verdünnt und im Agardiffusionstest auf Penicillingehalt getestet.

**Transacylierung von Isopenicillin N  (# 28 u. 36) mit PaCoA (HH ⊘ mm)**

| Isopeni- cillin N # | a) | | | | b) | | | | c) | | | |
|---|---|---|---|---|---|---|---|---|---|---|---|---|
| | s | 7' | 30' | 60' | s | 7' | 30' | 60' | s | 7' | 30' | 60' |
| Nr. 28 | 22.5 | 25.5 | 26.0 | 23.0 | 21.1 | 20.8 | 18.3 | 14.7 | 22.7 | 24.7 | 25.7 | 20.7 |
| Nr. 36 | 27.0 | 28.3 | 30.8 | 28.7 | 25.2 | 25.0 | 23.6 | 22.1 | 26.3 | 27.2 | 27.7 | 26.7 |
| | Isopenicillin N  Std.  µg/ml | | | | Penicillin V Std.  µg/ml | | | | | | | |
| | 50 | 25 | 12.5 | | 2.0 | 1.5 | 1.0 | 0.5 | 0.25 | | | |
| Nr. 28 | | 20 | 16.5 | 12.4 | 25.3 | 24.0 | 21.7 | 17.8 | 9.7 | | | |
| Nr. 36 | | 24.5 | 22.1 | 18.4 | | | | | | | | |

**Acylierung von 6–APA mit PaCoA in Abhängigkeit von der Enzymverdünnung**

| 1:5 | | | | 1:10 | | | | 1:50 | | | | 1:100 | | | |
|---|---|---|---|---|---|---|---|---|---|---|---|---|---|---|---|
| s | 7' | 30' | 60' | s | 7' | 30' | 60' | s | 7' | 30' | 60' | s | 7' | 30' | 60' |
| 18.3 | 24.4 | 24.3 | 15.7 | 18.6 | 25.4 | 28.5 | 25.4 | 16.0 | 24.2 | 30.7 | 30.7 | 14.5 | 21.3 | 26.7 | 27.0 |

| 1:500 | | | | 1:1000 | | | | Penicillin V  Std.  µg/ml | | | | |
|---|---|---|---|---|---|---|---|---|---|---|---|---|
| s | 7' | 30' | 60' | s | 7' | 30' | 60' | 2.0 | 1.5 | 1.0 | 0.5 | 0.25 |
| 14.8 | 17.8 | 20.6 | 22.8 | 0 | 9.6 | 10.5 | 11.1 | 25.3 | 24.0 | 21.7 | 17.8 | 9.7 |

## B. Penicillin V-Spaltung

Ansatz in Mikrotiterplatten:

| | |
|---|---|
| Kalium Penicillin V 100 µg/ml | 10 µl |
| 0,1 M Phosphatpuffer pH 7,5 | 15 µl |
| Enzym bzw. RP + Z ( + Inhibitor) | 75 µl |
| a) ohne Inhibitor ( = Kontrolle) | |
| b) + 2 mM Jodacetamid | |
| c) + 2 mM Ethylmaleinimid | |
| d) + 2 mM PMSF | |
| e) RP + Z | |

**Spaltung von Kalium Penicillin V (HH ⊘ mm) in Anwesenheit verschiedener Inhibitoren**

| a) | | | | b) | | | | c) | | | | d) | | | |
|---|---|---|---|---|---|---|---|---|---|---|---|---|---|---|---|
| s | 7' | 30' | 60' | s | 7' | 30' | 60' | s | 7' | 30' | 60' | s | 7' | 30' | 60' |
| 25.7 | 21.8 | 0 | 0 | 25.7 | 23.1 | 0 | 0 | 25.0 | 23.0 | 0 | 0 | 25.3 | 0 | 0 | 0 |

| e) | | | | Kalium Penicillin V Std.  µg/ml | | | | | Als Vergleich Transacylierung 6–APA + PaCoA → Penicillin V | | | |
|---|---|---|---|---|---|---|---|---|---|---|---|---|
| s | 7' | 30' | 60' | 2.0 | 1.5 | 1.0 | 0.5 | 0.25 | | s | 7' | 30' | 60' |
| 26.8 | 25.8 | 26.2 | 26.4 | 27.4 | 24.7 | 23.4 | 18.4 | 11.9 | | 17.0 | 23.2 | 26.9 | 25.9 |

**Beispiel 4: Aktivierungs-, Inhibierungs-, Stabilisierungs- und pH-Profil der PAT:**

Das, wie in Beispiel 2 beschrieben, hergestellte PAT-Ammonsulfatpräzipitat wird 1:10 mit 0,1 M Reaktionspuffer, in Anwesenheit verschiedener Aktivatoren bzw. Inhibitoren oder Stabilisatoren verdünnt und sofort bzw. nach eintägiger Inkubation bei 4° bzw. -20° im mikrobiologischen Testmodell auf 6-APA-PaCoA-Acyltransferaseaktivität getestet. Die Ergebnisse sind in Tabelle 2 zusammengefaßt.

**Beispiel 5: Temperaturstabilität der PAT:**

Das, wie in Beispiel 2 beschrieben, hergestellte PAT-Ammonsulfatpräzipitat wird 1:20 mit 0,1 M Phophatpuffer pH 7,5 inklusive 1 bzw. 10 mM DTT verdünnt, scharf zentrifugiert, der Überstand bei -196°, -20°, +4°, +20° und +37° stationär inkubiert und sofort bzw. nach 1, 4, 26 und 120 Stunden im mikrobiologischen Testmodell auf 6-APA-PaCoA-Acyltransferaseaktivität getestet. Die Ergebnisse sind in Tabelle 3 angeführt.

**Beispiel 6: Reinigung der PAT über hydrophobe Interaktionschromatographie auf Phenylsepharose Cl-4B (HIC-Lauf) und Affinitätschromatographie auf 6-APA-AH-Sepharose 4 B (Affinitätslauf):**

240 g der, wie in Beispiel 2 beschrieben, gewonnenen Ammonsulfatpräzipitation werden in 2000 ml 50 mM Phosphatpuffer pH 7,5 inklusive 1 M (NH$_4$)$_2$SO$_4$ sowie 1 mM DTT gelöst und im Arbeitskühlraum bei +4° auf eine BP 113-Säule mit 2000 ml äquilibrierter Phenylsepharose Cl-4B aufgegeben. Anschließend wird die Säule mit einem Fluß von 50 ml/min mit 3 Bettvolumina an 50 mM Phosphatpuffer pH 7,5 inklusive 1 M (NH$_4$)$_2$SO$_4$ und 1 mM DTT gewaschen, dann mit weiteren 2 Bettvolumina 50 mM Phosphatpuffer pH 7,5 inklusive 1 mM DTT das Ballastprotein entfernt, sodann mit 3 Bettvolumina entionisiertem Wasser (E-H$_2$O) inklusive 1 mM DTT das PAT-Enzym eluiert, die aktive Fraktion im Pellicon-Kassettensystem über eine Polysulfonultrafiltrationsmembran mit einem cut off von 10 kD auf ein Zehntel Volumen konzentriert, mit 1 GV % Sorbit, 5 mM DTT, 2 mM EDTA und 0,1 mM PMSF stabilisiert und bei -20° bis zum nächsten Chromatographieschritt eingefroren. Die mikrobiologisch erfaßte 6-APA-PaCoA-Acyltransferaseaktivität sowie der Gesamtproteingehalt der einzelnen, je ein Bettvolumen umfassenden Fraktionen sind in der folgenden Tabelle zusammengefaßt:

| Probe | Mikrobiologische PAT-Aktivität (mm HH Ø gegen Pseudomonas aeruginosa BC 248) | | | Gesamtprotein/Bradford (mg) |
|---|---|---|---|---|
| | sofort | 30 min | 30 min + Penase | |
| Einsatz | 19 | 24 | 0 | 5483 |
| PL 150/1 | 17 | 18 | 0 | 230 |
| PL 150/2 | 16 | 15 | 0 | 1o2 |
| PL 150/3 | 14 | 15 | 0 | 98 |
| PL 150/4 | 0 | 0 | 0 | 2750 |
| PL 150/5 | 0 | 0 | 0 | 390 |
| PL 150/6 | Spur | 15 | 0 | 52 |
| PL 150/7 | 18 | 31 | 0 | 389 |
| PL 150/8 | 0 | 0 | 0 | 14 |

Das aktive Retentat der HIC-Fraktion wird am nächsten Tag unter schonenden Bedingungen aufgetaut und auf eine K 26/40 Pharmaciasäule mit 50 ml Affinitätsmatrix 6-APA-AH-Sepharose 4 B, welche durch Kuppen von Penicillin G an AH-Sepharose 4B (Pharmacia) mit EEDQ nach dem LKB-Protokoll "Practicai guide for use in affinity chromatography and related techniques", Reactifs IBF-Societe Chimique Pointet-Girard, France 1933, S. 133, und durch anschließende Abspaltung der Phenylessigsäure mit löslicher Penicillin G-Amidase von E. coli hergestellt wurde, aufgegeben. Die Säule wird darauf mit einem Fluß von 3 ml/min mit folgenden Medien bei 4° eluiert:

AL 278/1:12 Bettvolumina 50 mM Phosphatpuffer pH 7,5 + 1 mM DTT + 0,1 M NaCl

AL 278/2: 3 Bettvolumina gleicher Puffer + 1 mM DTT + 0,1 M NaCl + 1 mM 6-APA

AL 278/3: 6 Bettvolumina gleicher Puffer + 1 mM DTT + 1 M NaCl + 1 mM 6-APA

Die mit dem spezifischen Eluenten 6-APA in der Fraktion 278/2 eluierte PAT-Aktivität wird im Minitan-Kassettensystem über eine Polysulfon-UF-Membran mit einem cut off von 10 kD auf ein Zehntel Volumen aufkonzentriert, erneut mit der Stabilitätsmischung 1 GV % Sorbit, 5 mM DTT, 2 mM EDTA und 0,1 mM PMSF versetzt und in Portionen tiefgefroren. Die über HPLC bestimmte Substratspezifität dieser zumindest 50% reinen Enzympräparation ist in Tabelle 4 festgehalten.

**Beispiel 7: Chromatofokussierung der affinitätschromatographisch hergestellten PAT-Enzympräparation auf Mono P/Pharmacia (Chromatofocussing AL 310):**

Eine 1 ml Portion der, wie in Beispiel 6 beschrieben, über Affinitätschromatographie gereinigten und konzentrierten PAT-Enzympräparation wird unter schonenden Bedingungen aufgetaut, über eine äquilibrierte PD 10 Sephadex G 25 Fertigsäule gegen 25 mM bis Tris/HCl Puffer pH 6,3 pufferausgetauscht, dann auf eine mit dem gleichen Puffer äquilibrierte 4 ml FPLC Mono P-Fertigsäule aufgegeben, anschließend sofort mit einem Fluß von 60 ml/Stunde mit 100 ml eines 1:10 verdünnten und auf pH 4,0 gestellten Polypuffers 74 eluiert, peakweise fraktioniert und die Fraktionen original bzw. über Centricon 10 zehnfach konzentriert über RPC, SDS-Gradientenpolyacrylamidgelelektrophorese, isoelektrische Fokussierung auf immobiline Basis und Gelfiltration analysiert.

Abbildung 1 zeigt das Elutionsprofil des PAT-Enzyms auf der Mono P. Das multifunktionelle Enzym wird in mindestens drei isoelektrische Formen (PAT 310/3, PAT 310/4 und PAT 310/5) aufgespalten. Diese verschieden geladenen Enzymvarianten können entweder echte Isoenzyme oder verschiedene Redoxformen desselben Enzyms darstellen.

Wie der Abbildung 2 zu entnehmen ist, zeffällt die thiolabhängige PAT aus Penicillium chrysogenum unter den denaturierenden Bedingungen der RPC (Säule = Biorad Hi Pore RP 304, 250x4,6 mm; HPLC-Parameter: 0,5 ml/min, 45 bar, 280 nm, 40°, 25 $\mu$l Injektionsvolumen; linearer Gradient zwischen Eluens A: 35%iges wäßriges Acetonitril mit 0,01% Trifluoressigsäure und Eluens B: 80%iges wäßriges Acetonitril mit 0,01% Trifluoressigsäure, 30 Minuten) in ein Polypeptid 1 und je nach Iso- bzw. Redoxvariante in ein bzw. zwei unterschiedlich hydrophobe Polypeptidvarianten 2a und 2b.

In der SDS-Gradientenpolyacrylamidgelelektrophorese (siehe Abbildung 3a, Bedingungen nach Laemmli, Nature 227, 680- 685, 1970; aber Gradientengel mit 8-20% T) zerfällt das PAT-Enzym in zwei verschieden große Polypeptide mit einem MG um 30 und 8 kD. Durch RPC und anschließende SDS-Gradienten-PAGE kann gezeigt werden, daß das in der RPC zuerst eluierende Polypeptid 1 mit der ca. 30 kD Komponente und die beiden stärker retentierten, hydrophoben Polypeptide 2a und 2b mit der 8 kD Komponente identisch sind. Weiters kann durch solche multidimensionalen Analysen gezeigt werden, daß die kleinen Polypeptide 2a und 2b auf der SDS-Gradienten-PAGE dieselbe Wanderungsstrecke besitzen, während das große, ca. 30 kD Polypeptid 1 der basischen Iso- bzw. Redoxvariante etwas schneller wandert als das entsprechende Polypeptid 1 der mehr sauren Form.

Bei der isoelektrischen Fokussierung (IEF) auf Ampholinebasis (siehe Abb. 3b, methodische Durchführung nach LKB Application Note 1804) zeigt der aktive Affinitätspool drei eng beisammenliegende Banden mit einem isoelektrischen Punkt um 5,1.

Im engen pH-Bereich der hochauflösenden IEF auf Immobilinebasis (sieh Abb. 3c, methodische Durchführung nach LKB Application Note 1819) zeigen die über Chromatofokussierung getrennten PAT-Varianten pl-Werte um 5,15, 5,06 und 5,32.

Bei der MG-Bestimmung über Gelfiltration wird auf Superose 12 von Pharmacia (Eluens: 0,1 M Phosphatpuffer, pH 7,5 inklusive 1 mM DTT, 0,4 ml/min) ein MG von 36,5 kD bzw. auf der TSK-G2000 SW von LKB (das gleiche Eluens mit 0,5 ml/min) ein MG von 38 kD bestimmt, was mit der Summe der beiden denaturierten Polypeptide 1 und 2 gut korreliert.

**Beispiel 8: N-terminale Sequenzierung der Polypeptide 1 und 2a sowie 2b:**

Eine weitere Portion (2 mg Protein) der, wie in Beispiel 6 beschrieben, über Affinitätschromatographie gereinigten PAT-Enzympräparation wird nach schonendem Auftauen zweimal über RPC (dieselbe Säule und Bedingungen wie in Beispiel 7 angeführt, aber mit einem flacher ansteigenden Gradienten: min/% B = 0/0, 8/0, 20/12, 25/12, 30/100, 33/100, 36/0, 40/0) getrennt, die Fraktionen mit den Polypeptiden 1 sowie 2a und 2b lyophilisiert und im Protein-Sequenator N-terminal sequenziert.

**N-terminale Aminosäuresequenz:**

**30 kD Einheit / Polypeptid 1: Teilsequenz 1**

```
1           5                   10                  15                  20
__-Thr-Thr-Ala-Tyr-Cys-Gln-Leu-Pro-Asn-Gly-Ala-Leu-Gln-Gly-Gln-Asn-Trp-Asp-Phe-
                    25                  30                  35      37
Phe-Ser-Ala-Thr-Lys-Glu-Asn-Leu-Ile-Arg-__-__-__-__-Gln-__-Gly-
```

**8 kD Einheit I / Polypeptid 2a: Teilsequenz 6**

```
1           5                   10                  15                  20
Met-Leu-His-Ile-Leu-Cys-Gln-Gly-Thr-Pro-Phe-Glu-Ile-Gly-Tyr-Glu-His-Gly-Ser-Ala-
                    25                  30                  34
Ala-Lys-Ala-Val-Ile-Ala-Arg-Ser-Ile-Asp-Phe-Ala-Val-Asp-
```

**8 kD Einheit II / Polypeptid 2b: Teilsequenz 6**

```
1           5                   10                  15                  20
Met-Leu-His-Ile-Leu-Cys-Gln-Gly-Thr-Pro-Phe-Glu-Ile-Gly-Tyr-Glu-His-Gly-Ser-Ala-
                    25                  30                  35                  40
Ala-Lys-__-Val-Ile-Ala-Arg-__-Ile-Asp-Phe-Ala-Val-Asp-Leu-__-__-Gly-__-Thr-
```

Wie in der SDS-Gradienten-PAGE unterscheiden sich die in der RPC unterschiedlich retentierten kleinen Polypeptide 2a und 2b im sequenzierten Molekülschnitt nicht.

**Beispiel 9: Sequenzierung proteolytischer Peptidfragmente von Polypeptid 1 und 2:**

Eine, wie in Beispiel 8 beschrieben, hergestellte lyophilisierte Fraktion von Polypeptid 1 bzw. 2 wird nach J.M.Wilkinson (Practical Protein Chemistry - A Handbook, edited by A. Darbre, 1986) mit Trypsin, bzw. Lysylendopeptidase enzymatisch gespalten, einzelne Peptidfragmente werden über RPC isoliert und im Protein-Sequenator sequenziert. Folgende Aminosäure-Teilsequenzen werden u.a. erhalten:

AS-Teilsequenz 2 (tryptisches Peptidfragment von Polypeptid 1):

```
1           5                   10          13
Gly-Ala-Thr-Leu-Phe-Asn-Ile-Ile-Tyr-Asp-His-Ala-Arg-
```

AS-Teilsequenz 3 (tryptisches Peptidfragment von Polypeptid 1):

```
1           5                   10
Pro-Thr-Asn-Pro-Asp-Glu-Met-Phe-Val-Met-Arg
```

AS-Teilsequenz 4 (tryptisches Peptidfragment von Polypeptid 1):

```
1           5                   10
Glu-Leu-Asp-Pro-Leu-Pro-Asp-Ser-Trp-Asn-Arg
```

AS-Teilsequenz 5 (tryptisches Peptidfragment von Polypeptid 1):

```
1           5                   10      12
Met-Glu-Phe-Leu-__-Asp-Gly-Phe-Asp-Gly-Thr-Lys
```

AS-Teilsequenz 7 (tryptisches Peptidfragment von Polypeptid 1):

```
1           5                   10                  14
Ile-Ala-Leu-Glu-Ser-Thr-Ser-Pro-Ser-Gln-Ala-Tyr-Asp-Arg
```

AS-Teilsequenz 8 (tryptisches Peptidfragment von Polypeptid 1):

Val-Gly-Phe-Asn-Ser-Ala-Gly-Val-Ala-Val-Asn-Tyr-Asn-Ala-Leu-His-Leu-Gln-Gly-Leu-Arg-Pro-Thr-Gly-Val-Pro-Ser-His-Ile-Ala-Leu-Arg

AS-Teilsequenz 9 (tryptisches Peptidfragment von Polypeptid 2):

Thr-Glu-Phe-Ala-Tyr-Gly-Leu-Lys

AS-Teilsequenz 10 (tryptisches Peptidfragment von Polypeptid 2):

Tyr-Tyr-__-Glu-Ile-Arg

AS-Teilsequenz 11 (tryptisches Peptidfragment von Polypeptid 2):

Trp-Pro-Lys

AS-Teilsequenz 12 (tryptisches Peptidfragment von Polypeptid 2):

Ser-Ile-Asp-Phe-Ala-Val-Asp-Leu-Ile-Arg

AS-Teilsequenz 13 (tryptisches Peptidfragment von Polypeptid 2):

Asp-Val-Ser-Glu-Ile-Val-Met-Leu-Asn-Thr-Arg

AS-Teilsequenz 14 (tryptisches Peptidfragment von Polypeptid 2):

Gln-Val-Leu-Ser-Gln-Leu-Gly-Arg

AS-Teilsequenz 15 (Peptidfragment von Polypeptid 2 mit Lysylendopeptidase):

Cys-Gln-Gly-Thr-Pro-Phe-Glu

AS-Teilsequenz 16 (Peptidfragment von Polypeptid 2 mit Lysylendopeptidase):

Tyr-Tyr-Glu-Glu-Ile-Arg-Gly-Ile-Ala-Lys

AS-Teilsequenz 17 (Peptidfragment von Polypeptid 2 mit Lysylendopeptidase):

Gln-Val-Leu-Ser-Gln-Leu-Gly-Arg-Val-Ile-Glu-Glu-Arg-__-Pro-Lys

AS-Teilsequenz 18 (Peptidfragment von Polypeptid 2 mit Lysylendopeptidase):

Gly-Ala-Glu-Arg-Asp-Val-Ser-Glu-Ile-Val-Met-Leu-Asn-Thr-Arg

AS-Teilsequenz 19 (tryptisches Peptidfragment von Polypeptid 2):

Ala-Val-Ile-Ala-Arg

AS-Teilsequenz 20 (tryptisches Peptidfragment von Polypeptid 2):

Lys-Thr-Asp-Glu-Glu-Leu-Lys

AS–Teilsequenz 21 (tryptisches Peptidfragment von Polypeptid 2):

1        4

Gly–Ile–Ala–Lys


AS–Teilsequenz 22 (tryptisches Peptidfragment von Polypeptid 2):

1         5

Val–Ile–Glu–Glu–Arg


AS–Teilsequenz 23 (Peptidfragment von Polypeptid 2 mit Lysylendopeptidase):

1        5           10

Asn–Thr–__–Thr–Glu–Phe–Ala–Tyr–Gly–Leu–Lys


AS–Teilsequenz 24 (tryptisches Peptidfragment von Polypeptid 2):

1      3

Ala–Ala–Arg


**Beispiel 10: Isolierung von poly(A)$^+$ RNA aus Myzel von Penicillium chrysogenum:**

Eine Sporensuspension von Penicillium chrysogenum P2/ATCC 48271 wird in 5 Erlenmeyerkolben (zu je 1000 ml) mit je 100 ml CDS-Medium (für 1 l CDS-Medium werden nach dem Autoklavieren gemischt: 900 ml Lösung A: 3g NaNO$_3$, 0,5g MgSO$_4$.7H$_2$O, 0,5g KCl, 0,1 g FeSO$_4$.2H$_2$O, 5 g Hefeextrakt, 10 g Caseinpepton, 20 g Saccharose, pH 5,8 und 100 ml Lösung B: 250 mM K$_2$HPO$_4$/KH$_2$PO$_4$, pH 5,8) etwa 70 Stunden bei 25° und 250 Upm in einem Schüttler inkubiert. 12 Erlenmeyerkolben (zu je 1000 ml) mit je 300 ml CDLP-Medium (Zusammensetzung wie CDS-Medium, an Stelle von 20 g Saccharose jedoch 150 g Lactose/l und 50 ml 10% Phenoxyessigsäure [gelöst in Wasser, pH 7,5 mit KOH eingestellt]) werden mit je 30 ml der Vorkultur beimpft und 40 Stunden bei 25° und 250 Upm im Schüttler inkubiert. Das Myzel wird mittels eines Büchner-Trichters abfiltriert, kurz mit TE gewaschen (10 mM Tris/HCl, pH 8,1, 1 mM EDTA) und in flüssigem Stickstoff zu einem feinen Pulver zerrieben. Dieses Pulver wird in Lysepuffer (5 M Guanidinmonothiocyanat, 10 mM EDTA, 50 mM Tris/HCl, pH 7,5, 8% $\beta$-Mercaptoethanol) suspendiert (3 ml Lysepuffer pro g Myzelfeuchtmesse) und 1 Stunde bei Raumtemperatur gerührt. Die Isolierung der Gesamt-RNA erfolgt nach einer beschriebenen Methode (Cathala et al., DNA 2, 329-335, 1983). 60 g Myzelfeuchtmasse erlauben es, etwa 18 mg Roh-RNA zu isolieren, die mit Ethanol gefällt und bei -70° aufbewahrt wird, Die Anreicherung der poly(A)$^+$ RNA geschieht durch eine Oligo (dT) Cellulose-Affinitätschromatographie, wie sie in Maniatis et al., Molecular Cloning, Cold Spring Harbor, 1982, beschrieben ist. Dazu wird die Roh-RNA durch Zentrifugation und Trocknung in Wasser gelöst. Nach der Chromatographie werden Fraktionen, die poly(A)$^+$ RNA enthalten, erneut mit Ethanol gefällt und bei -70° aufbewahrt. Die Integrität der poly(A)$^+$ RNA wird durch Gelelektrophorese in Gegenwart von Glyoxal (Maniatis et al.) überprüft, die Konzentration durch UV-Absorption.

**Beispiel 11: Partielle Sequenzierung einer RNA, die für das Enzym PAT kodiert:**

Aus der Aminosäuresequenz des Polypeptides 1 der PAT (Beispiel 8) läßt sich auf Grund des genetischen Kodes eine DNA-Sequenz ableiten (Abb. 5). Ein Bereich von 17 bp, der eine minimale Anzahl von unterschiedlichen Sequenzen zuläßt, wird für die Synthese von Oligonukleotidgemischen ausgewählt. 4 Gemische mit 4 unterschiedlichen Oligonukleotiden, die zu dem Sinn-Strang und damit auch zur RNA komplementär sind, werden synthetisiert (Oligonukleotidgemische 1, 2, 3 und 4 in Abb. 5). Diese Oligonukleotide werden enzymatisch phosphoryliert und dadurch radioaktiv markiert (Maniatis et al.). 150 ng Oligonukleotide werden in 12 $\mu$l Reaktionsansatz (50 mM Tris/HCl, pH 9,5, 10 mM MgCl$_2$, 5 mM DTE, 5% Glycerin) mit 250 $\mu$Ci ATP (Amersham PB 10218) mit 16 Einheiten Polynukleotidkinase für 30 Minuten bei 37° inkubiert. 10 $\mu$g der poly(A)$^+$ RNA (Beispiel 10) werden in 250 mM KCl, 10 mM Tris/HCl, pH 8,3 mit 2 $\mu$l 32-P-markiertem Oligonukleotidgemisch in einem 0,5 ml Reaktionsgefäß vermischt. Die Mischung wird 2 Minuten auf 75° erhitzt und dann 45 Minuten bei 50° erwärmt. In 4 Eppendorfgefäße werden je 3,3 $\mu$l Reaktionspuffer (24 mM Tris/HCl, pH 8,3, 16 mM MgCl$_2$, 8 mM DTE, 0,4 mM dATP, 0,8 mM dGTP, 0,4 mM dCTP, 0,4 mM dTTP, 6 Einheiten Reverse Transcriptase und außerdem je 1 $\mu$l 1 mM dATP oder 1 $\mu$l 1

mM dGTP oder 1 µl 1 mM dCTP oder 1 µl 1 mM dTTP gegeben. Nach der Zugabe von 2 µl poly(A)[+] RNA-Oligonukleotidgemisch wird der inhalt des Eppendorfgefäßes kurz zentrifugiert und 45 Minuten bei 50° erwärmt. Die Reaktion wird durch 2 µl Stop-Puffer (99% Formamid, 0,3% Bromphenolblau, 0,3% Xylencyanol) und dreiminütiges Kochen beendet. Je 4 µl der Proben werden auf ein 8%iges Polyacrylamld/Harnstoffgel aufgetragen und 2,5 Stunden bei 40 W aufgetrennt (Sequl Gen, Biorad). Nach dem Lauf wird das Gel für 20 Minuten in 5% Methanol, 5% Essigsäure gelegt und 1 Stunde bei 80° getrocknet. Das abgekühlte Gel wird in Haushaltsfolie eingeschlagen. Ein Röntgenfilm (Kodak XOmat AR) wird für 20 Stunden aufgelegt. Die Sequenz, die sich von diesem Röntgenfilm ablesen läßt, ist wiedergegeben:

```
              10          20          30          40          50          60
    5´—CCATTTGGAAGTTGACAATAGGCAGTGGTGCAGCCATCACGGGCTGCCTTGAGCCCGTAT
              70          80          90          100         110
    GCAAATTCCGTGCGGGTATTAAGCATGACAATCTCGGAGACATCGCGTTCA—3´
```

Die Positionen 1 bis 29 sind komplementär zur DNA-Sequenz, die sich aus der Aminosäuresequenz des Polypeptides 1 der PAT ableiten läßt (Abbildung 5, 6D). Ein Oligonukleotid, das die ersten 30 Nukleotide dieser Sequenz umfaßt, wird synthetisiert.

**Beispiel 12: Konstruktion einer genomischen Genbank von Penicillium chrysogenum:**

Aus dem Myzel einer in CDS-Medium gewachsenen Penicillium chrysogenum Kultur (Beispiel 10) wird DNA isoliert. Das Myzel wird in flüssigem Stickstoff zerrieben, dann in 1% Sarkosyl, 0,1 M EDTA, pH 8, 100 µg Proteinase K/ml gegeben (1 g Myzel/25 ml) und 48 Stunden bei 37° geschüttelt. Die Mischung wird dreimal mit Phenol extrahiert und nach Zugabe von 0,1 Volumsanteilen 3 M Natriumazetat, pH 5,2, mit Ethanol gefällt. Anschließend wird eine CsCl-EtBr-Zentrifugation durchgeführt (Maniatis et al.) und nach Extraktion mit Isoamylalkohol und Dialyse gegen TE die Konzentration durch UV-Absorption bestimmt. Die DNA wird außerdem durch Agarose-Gelelektrophorese überprüft. 300 µg Penicillium chrysogenum DNA werden in 5 U Sau3A (BRL) 60 Minuten bei 37° in 10 mM Tris/HCl, pH 7,5, 10 mM MgCl$_2$, 1 mM DTE, 50 mM NaCl gespalten. Das Ausmaß der Spaltung wird auf einem Agarose-Gel überprüft. Erwünscht ist, daß ein großer Teil der DNA zwischen 10 und 20 kb groß ist. Die DNA wird dann auf zwei NaCl-Gradienten (hergestellt durch Einfrieren und Wiederauftauen von 2 Beckmann-SW28.1-Ultrazentrifugenröhrchen mit 20% NaCl in TE [10 mM Tris/HCl, pH 8,0, 1 mM ETDA]) aufgetragen und in der Ultrazentrifuge im Rotor SW 28.1 16 Stunden bei 14.000 Upm zentrifugiert. Der Inhalt der Röhrchen wird fraktioniert. Fraktionen mit DNA größer als 10 kb werden vereinigt und gegen TE dialysiert. Nach einer Konzentrierung der DNA (500 µg/ml) wird sie mit λ-EMBL3-DNA (Frischhauf et al., J.Mol.Biol. 170, 827-832, 1983), die mit BamHI und EcoRI gespalten worden ist, vereinigt. In 30 mM Tris/HCl, pH 7,5, 10 mM MgCl$_2$, 10 mM DTE, 2,5 mM ATP wird nach Zugabe von 0,5 U T4-DNA-Ligase über Nacht bei 16° ligiert (DNA-Konzentration 200 µg/ml, Vektor zu Penicillium chrysogenum-DNA im molaren Verhältnis 1:1). Das Ligationsgemisch wird mit Hilfe von Proteinextrakten ("packaging mixes", Maniatis et al.) in vitro verpackt. Die entstandenen λ-Lysate werden auf dem Indikatorstamm NM539 (Frischhauf et al.) getitert. Erhalten werden etwa 10[6] pfu.

**Beispiel 13: Isolierung von λ-Klonen, die mit einem PAT-spezifischen Oligonukleotid hybridisieren:**

40.000 rekombinante Phagen der Penicillium chrysogenum Genbank in λ-EMBL3 (Beispiel 12) werden mit dem Stamm NM538 (Frischhauf et al.) auf 90 mm TB-Platten in 0,7% Agarose ausgegossen (TB Medium enthält 10 g Bacto Trypton und 5 g NaCl pro Liter, der pH 7,5 wird mit NaOH eingestellt). Von diesen Platten werden je zwei Abdrücke auf Nylon-Filter hergestellt (Amersham Hybond N-Filter). Nach der UV-Fixierung der DNA werden diese Filter für die Hybridisierung eingesetzt. Das in Beispiel 11 beschriebene 30mer, dessen Sequenz komplementär zu der DNA-Sequenz ist, die aus der Aminosäuresequenz des Polypeptides 1 der PAT ist, wird radioaktiv markiert (T4-Polynukleotid-Kinase-Reaktion wie in Beispiel 11). Die Hybridisierung wird bei 37° in 6xSSPE (1xSSPE ist 0,15 M NaCl, 10 mM NaH$_2$PO$_4$, 1 mM EDTA, pH 7,4), 30% Formamid, 5x Denhard, 0,1% SDS, 100 µg/ml Heringssperma-DNA, 0,1 mM ATP, 3 ng/ml 32-P-markiertes Oligonukleotid für etwa 20 Stunden durchgeführt. Die Filter werden dann dreimal 10 Minuten bei Raumtemperatur in 2xSSC (1xSSC ist 0,3 M NaCl, 0,03 M Natriumcitrat, pH 7,0), 0,1% SDS und anschließend dreimal 20 Minuten bei 56° in 1xSSC, 0,1% SDS gewaschen und nach dem Trocknen autoradiographiert. Regionen in der Agaroseschicht der ursprünglichen Platte, die positiven Hybridisierungssignalen auf dem Röntgenfilm entsprechen, werden mit einer sterilen Pasteurpipette ausgestochen und in SM-Puffer (5,8 g NaCl, 2 g MgSO$_4$.7H$_2$O und 50 ml 1 M Tris/HCl, pH 7,5) resuspendiert. Eine geeignete Verdünnung wird mit NM538 als Indikatorstamm erneut auf TB-Platten ausgegossen. Die Phagen auf diesen Platten werden auf Nylonfilter übertragen und wie oben beschrieben hybridisiert. Aus Plaques, die zum zweitenmal positive Signale ergeben, werden die entsprechenden rekombinanten λ-Phagen isoliert. Die gereinigte DNA dieser Phagen wird für Restriktionsanalysen und Southernhybridisierungen verwendet. Außerdem werden Subklonierungen in Plasmide (pUC 12, Messing, Methods Enzymol. 101, 20, 1983) und M13-Vektoren durchgeführt (M13mp18, M13mp19, Norrander et al., Gene 26, 101, 1983). Die Sequenzierung eines M13mp19-Subklons mit dem Oligonukleotid ergibt folgende Sequenz:

```
              10         20        30        40         50
      5´-CATCACGGGCTGCCTTGAGCCCGTATGCAAATTCCGTGCGGGTATTAAGC
              60         70        80        90        100
      ATGACAATCTCGGAGACATCGCGTTCAGCGCCCTTTGCAATACCTGGTTT
             110        120       130       140        150
      TTCGGGGGTCATCGGTCAGCATTGGTGCAGAAAATGTAGGAAAGACCGAA
             160        170       180       190        200
      GTGGCACTCACCGCGAATCTCCTCGTAGTATTTGGGCCATCTTTCCTCGATC-3´
```

Die Nukleotide 35 bis 111 der DNA-Sequenz, die mit Hilfe der RNA bestimmt werden (Beispiel 11), stimmen mit den Nukleotiden 1 bis 77 dieser DNA-Sequenz überein (Abb. 6F).

**Beispiel 14: DNA-Sequenz des PAT-Gens:**

In Abbildung 4 ist die Lage dieser DNA-Sequenz durch zwei waagrechte Striche eingezeichnet (A), 1 entspricht dabei dem ersten Nukleotid, 1972 dem letzten.

```
        10        20        30        40        50        60        70
GTTGATGTCCCATCAGTGTCATGCTATGGTCCCAGATTGGTGGCTACGGCAATATAAATCTCAGCATGCA

        80        90       100       110       120       130       140
GTTCCGCCTGCATGATCATCCCCAGGACGCCGTTTGTCATCTCCGTCAGCCAGGTCTCAGTTGTTTACCC

       150       160       170       180       190       200       210
ATCTTCCGACCCGCAGCAGAAATGCTTCACATCCTCTGTCAAGGCACTCCCTTTGAAGTAAGTGCTGCAC

       220       230       240       250       260       270       280
TGAATACCAGATTTTTTCCTTCTGAATCTTCCGAGTTCTGACCTGATCCAGATCGGCTACGAACATGGCT

       290       300       310       320       330       340       350
CTGCTGCCAAAGCCGTGATAGCCAGAAGCATTGACTTCGCCGTCGATCTCATCCGAGGGAAAACGAAGAA

       360       370       380       390       400       410       420
GACGGACGAAGAGCTTAAACAGGTACTCTCGCAACTGGGGCGCGTGATCGAGGAAAGATGGCCCAAATAC

       430       440       450       460       470       480       490
TACGAGGAGATTCGCGGTGAGTGCCACTTCGGTCTTTCCTACATTTTCTGCACCAATGCTGACCGATGAC

       500       510       520       530       540       550       560
CCCCGAAAAACCAGGTATTGCAAAGGGCGCTGAACGCGATGTCTCCGAGATTGTCATGCTTAATACCCGC

       570       580       590       600       610       620       630
ACGGAATTTGCATACGGGCTCAAGGCAGCCCGTGATGGCTGCACCACTGCCTATTGTCAACTTCCAAATG

       640       650       660       670       680       690       700
GAGCCCTCCAGGGCCAAAACTGGGATGTACGTTAAGAGATTTTACCTCCTCATTTTATTCCATCGAATTT

       710       720       730       740       750       760       770
GCGCCGACTAATTTGGTTGTTCAAGTTCTTTTCTGCCACCAAAGAGAACCTGATCCGGTTAACGATCCGT

       780       790       800       810       820       830       840
CAGGCCGGACTTCCCACCATCAAATTCATAACCGAGGCTGGAATCATCGGGAAGGTTGGATTTAACAGTG

       850       860       870       880       890       900       910
CGGGGGTCGCCGTCAATTACAACGCCCTTCACCTTCAAGGTCTTCGACCCACCGGAGTTCCTTCGCATAT

       920       930       940       950       960       970       980
TGCCCTCCGCATAGCGCTCGAAAGCACTTCTCCTTCCCAGGCCTATGACCGGATCGTGGAGCAAGGCGGA

       990      1000      1010      1020      1030      1040      1050
ATGGCCGCCAGCGCTTTTATCATGGTGGGCAATGGGCACGAGGCATTTGGTTTGGAATTCTCCCCCACCA
```

```
      1060      1070      1080      1090      1100      1110      1120
GCATCCGAAAGCAGGTGCTCGACGCGAATGGTAGGATGGTGCACACCAACCACTGCTTGCTTCAGCACGG

      1130      1140      1150      1160      1170      1180      1190
CAAAAATGAGAAAGAGCTCGATCCCTTACCGGACTCATGGAATCGCCACCAGCGTATGGAGTTCCTCCTC

      1200      1210      1220      1230      1240      1250      1260
GACGGGTTCGACGGCACCAAACAGGCATTTGCCCAGCTCTGGGCCGACGAAGACAATTATCCCTTTAGCA

      1270      1280      1290      1300      1310      1320      1330
TCTGCCGCGCTTACGAGGAGGGCAAGAGCAGAGGCGCGACTCTGTTCAATATCATCTACGACCATGCCCG

      1340      1350      1360      1370      1380      1390      1400
TAGAGAGGCAACGGTGCGGCTTGGCCGGCCGACCAACCCTGATGAGATGTTTGTCATGCGGTTTGACGAG

      1410      1420      1430      1440      1450      1460      1470
GAGGACGAGAGGTCTGCGCTCAACGCCAGGCTTTGAAGGCTCTTCATGACGAGCCAATGCATCTTTTGTA

      1480      1490      1500      1510      1520      1530      1540
TGTAGCTTCAACCGACTCCGTCTTCACTTCTTCGCCCGCACTGCCTACCGTTTGTACCATCTGACTCATA

      1550      1560      1570      1580      1590      1600      1610
TAAATGTCTAGCCCCTACCTACACTATACCTAAGGGAGAGAAGCGTAGAGTGATTAACGTACGGGCCTAT

      1620      1630      1640      1650      1660      1670      1680
AGTACCCCGATCTCTAGATAGAACATTTAGTAGAGATTAGGATGCCTAACTAATTTAACTTGAGCATTGT

      1690      1700      1710      1720      1730      1740      1750
CCCGTTCATATTGATTTTCATCCATTATACACTCTTAATCGTTTCCCGGTAGAAGCCGNATATATACGAC

      1760      1770      1780      1790      1800      1810      1820
CATAGGGTGTGGAGAACAGGGCTTCCCGTCTGCTTGGCCGTACTTAAGCTATATATTCTACACGGCCAAT

      1830      1840      1850      1860      1870      1880      1890
ACTCAATGTGCCCTTAGCACCTAAGCGGCACTCTAGGGTAAGTGCGGGTGATATAGGTGAGAAGTCTTAA

      1900      1910      1920      1930      1940      1950      1960
GACTGAAGACAGGATATCACGCGTTACCCTGCACCGTACCTACTACCTTCAATATCACTCTTTCAGGATG

      1970
GACAGGGTCGAC
```

**Beispiel 15: DNA-Sequenz des PAT-Gens:**

In dieser DNA-Sequenz wurde der kodierende Bereich durch einen Vergleich der experimentell bestimmten Aminosäureteilsequenzen mit der Kodierkapazität dieser DNA-Sequenz festgelegt. Dabei zeigt es sich, daß in diesem Gen drei Introns vorkommen, die zusätzlich durch Intron-Exon-Konsensus-Sequenzen (Rambosek und Leach, Critical Reviews in Biotechnology 6, 357-393, 1987) identifiziert wurden.

```
        10        20        30        40        50        60        70
GTTGATGTCCCATCAGTGTCATGCTATGGTCCCAGATTGGTGGCTACGGCAATATAAATCTCAGCATGCA
```

```
            80        90       100       110       120       130       140
GTTCCGCCTGCATGATCATCCCCAGGACGCCGTTTGTCATCTCCGTCAGCCAGGTCTCAGTTGTTTACCC

           150       160       170       180       190       200       210
ATCTTCCGACCCGCAGCAGAAATGCTTCACATCCTCTGTCAAGGCACTCCCTTTGAAGTAAGTGCTGCAC
                     MetLeuHisIleLeuCisGlnGlyThrProPheGlu

           220       230       240       250       260       270       280
TGAATACCAGATTTTTTCCTTCTGAATCTTCCGAGTTCTGACCTGATCCAGATCGGCTACGAACATGGCT
                                                       IleGlyTyrGluHisGlyS

           290       300       310       320       330       340       350
CTGCTGCCAAAGCCGTGATAGCCAGAAGCATTGACTTCGCCGTCGATCTCATCCGAGGGAAAAACGAAGAA
erAlaAlaLysAlaValIleAlaArgSerIleAspPheAlaValAspLeuIleArgGlyLysThrLysLy

           360       370       380       390       400       410       420
GACGGACGAAGAGCTTAAACAGGTACTCTCGCAACTGGGGCGCGTGATCGAGGAAAGATGGCCCAAATAC
sThrAspGluGluLeuLysGlnValLeuSerGlnLeuGlyArgValIleGluGluArgTrpProLysTyr

           430       440       450       460       470       480       490
TACGAGGAGATTCGCGGTGAGTGCCACTTCGGTCTTTCCTACATTTTCTGCACCAATGCTGACCGATGAC
TyrGluGluIleArgG

           500       510       520       530       540       550       560
CCCCGAAAAACCAGGTATTGCAAAGGGCGCTGAACGCGATGTCTCCGAGATTGTCATGCTTAATACCCGC
                 lyIleAlaLysGlyAlaGluArgAspValSerGluIleValMetLeuAsnThrArg

           570       580       590       600       610       620    ·   630
ACGGAATTTGCATACGGGCTCAAGGCAGCCCGTGATGGCTGCACCACTGCCTATTGTCAACTTCCAAATG
ThrGluPheAlaTyrGlyLeuLysAlaAlaArgAspGlyCysThrThrAlaTyrCysGlnLeuProAsnG

           640       650       660       670       680       690       700
GAGCCCTCCAGGGCCAAAACTGGGATGTACGTTAAGAGATTTTACCTCCTCATTTTATTCCATCGAATTT
lyAlaLeuGlnGlyGlnAsnTrpAsp

           710       720       730       740       750       760       770
GCGCCGACTAATTTGGTTGTTCAAGTTCTTTTCTGCCACCAAAGAGAACCTGATCCGGTTAACGATCCGT
                       PhePheSerAlaThrLysGluAsnLeuIleArgLeuThrIleArg

           780       790       800       810       820       830       840
CAGGCCGGACTTCCCACCATCAAATTCATAACCGAGGCTGGAATCATCGGGAAGGTTGGATTTAACAGTG
GlnAlaGlyLeuProThrIleLysPheIleThrGluAlaGlyIleIleGlyLysValGlyPheAsnSerA

           850       860       870       880       890       900       910
CGGGGGTCGCCGTCAATTACAACGCCCTTCACCTTCAAGGTCTTCGACCCACCGGAGTTCCTTCGCATAT
laGlyValAlaValAsnTyrAsnAlaLeuHisLeuGlnGlyLeuArgProThrGlyValProSerHisIl

           920       930       940       950       960       970       980
TGCCCTCCGCATAGCGCTCGAAAGCACTTCTCCTTCCCAGGCCTATGACCGGATCGTGGAGCAAGGCGGA
eAlaLeuArgIleAlaLeuGluSerThrSerProSerGlnAlaTyrAspArgIleValGluGlnGlyGly

           990      1000      1010      1020      1030      1040      1050
ATGGCCGCCAGCGCTTTTATCATGGTGGGCAATGGGCACGAGGCATTTGGTTTGGAATTCTCCCCCCACCA
MetAlaAlaSerAlaPheIleMetValGlyAsnGlyHisGluAlaPheGlyLeuGluPheSerProThrS
```

21

```
        1060      1070      1080      1090      1100      1110      1120
GCATCCGAAAGCAGGTGCTCGACGCGAATGGTAGGATGGTGCACACCAACCACTGCTTGCTTCAGCACGG
erIleArgLysGlnValLeuAspAlaAsnGlyArgMetValHisThrAsnHisCysLeuLeuGlnHisGl
```

```
        1130      1140      1150      1160      1170      1180      1190
CAAAAATGAGAAAGAGCTCGATCCCTTACCGGACTCATGGAATCGCCACCAGCGTATGGAGTTCCTCCTC
yLysAsnGluLysGluLeuAspProLeuProAspSerTrpAsnArgHisGlnArgMetGluPheLeuLeu
```

```
        1200      1210      1220      1230      1240      1250      1260
GACGGGTTCGACGGCACCAAACAGGCATTTGCCCAGCTCTGGGCCGACGAAGACAATTATCCCTTTAGCA
AspGlyPheAspGlyThrLysGlnAlaPheAlaGlnLeuTrpAlaAspGluAspAsnTyrProPheSerI
```

```
        1270      1280      1290      1300      1310      1320      1330
TCTGCCGCGCTTACGAGGAGGGCAAGAGCAGAGGCGCGACTCTGTTCAATATCATCTACGACCATGCCCG
leCysArgAlaTyrGluGluGlyLysSerArgGlyAlaThrLeuPheAsnIleIleTyrAspHisAlaAr
```

```
        1340      1350      1360      1370      1380      1390      1400
TAGAGAGGCAACGGTGCGGCTTGGCCGGCCGACCAACCCTGATGAGATGTTTGTCATGCGGTTTGACGAG
gArgGluAlaThrValArgLeuGlyArgProThrAsnProAspGluMetPheValMetArgPheAspGlu
```

```
        1410      1420      1430      1440      1450      1460      1470
GAGGACGAGAGGTCTGCGCTCAACGCCAGGCTTTGAAGGCTCTTCATGACGAGCCAATGCATCTTTTGTA
GluAspGluArgSerAlaLeuAsnAlaArgLeu***
```

```
        1480      1490      1500      1510      1520      1530      1540
TGTAGCTTCAACCGACTCCGTCTTCACTTCTTCGCCCGCACTGCCTACCGTTTGTACCATCTGACTCATA
```

```
        1550      1560      1570      1580      1590      1600      1610
TAAATGTCTAGCCCCTACCTACACTATACCTAAGGGAGAGAAGCGTAGAGTGATTAACGTACGGGCCTAT
```

```
        1620      1630      1640      1650      1660      1670      1680
AGTACCCCGATCTCTAGATAGAACATTTAGTAGAGATTAGGATGCCTAACTAATTTAACTTGAGCATTGT
```

```
        1690      1700      1710      1720      1730      1740      1750
CCCGTTCATATTGATTTTCATCCATTATACACTCTTAATCGTTTCCCGGTAGAAGCCGNATATATACGAC
```

```
        1760      1770      1780      1790      1800      1810      1820
CATAGGGTGTGGAGAACAGGGCTTCCCGTCTGCTTGGCCGTACTTAAGCTATATATTCTACACGGCCAAT
```

```
        1830      1840      1850      1860      1870      1880      1890
ACTCAATGTGCCCTTAGCACCTAAGCGGCACTCTAGGGTAAGTGCGGGTGATATAGGTGAGAAGTCTTAA
```

```
        1900      1910      1920      1930      1940      1950      1960
GACTGAAGACAGGATATCACGCGTTACCCTGCACCGTACCTACTACCTTCAATATCACTCTTTCAGGATG
```

```
        1970
GACACGGGTCGAC
```

## Beispiel 16: Aminosäuresequenz der PAT, abgeleitet aus der DNA-Sequenz:

Dieses Polypeptid hat ein Molekulargewicht von 40.000 D. Wenn es zwischen Aminosäurerest 102 und 103 gespalten wird, erhält man zwei Polypeptide: Ein Polypeptid mit 11.500 D und der N-terminalen Aminosäuresequenz des Polypeptides 2; sämtliche Peptidfragmente lassen sich in dieser Aminosäuresequenz wiederfinden (AS-Teilsequenzen 9 bis 24). Es handelt sich also um die "8kD-Komponente der PAT".

Ein Polypeptid mit 28.500 D und der N-terminalen Aminosäuresequenz des Polypeptides 1; sämtliche Peptidfragmente lassen sich in dieser Aminosäuresequenz wiederfinden (AS-Teilsequenzen 2, 3, 4, 5, 7, 8).

Es handelt sich also um die "30kD-Komponente der PAT"

```
                              10                              20
         MetLeuHisIleLeuCysGlnGlyThrProPheGluIleGlyTyrGluHisGlySerAla

                              30                              40
         AlaLysAlaValIleAlaArgSerIleAspPheAlaValAspLeuIleArgGlyLysThr

                              50                              60
         LysLysThrAspGluGluLeuLysGlnValLeuSerGlnLeuGlyArgValIleGluGlu

                              70                              80
         ArgTrpProLysTyrTyrGluGluIleArgGlyIleAlaLysGlyAlaGluArgAspVal

                              90                              100
         SerGluIleValMetLeuAsnThrArgThrGluPheAlaTyrGlyLeuLysAlaAlaArg

                              110                             120
         AspGlyCysThrThrAlaTyrCysGlnLeuProAsnGlyAlaLeuGlnGlyGlnAsnTrp

                              130                             140
         AspPhePheSerAlaThrLysGluAsnLeuIleArgLeuThrIleArgGlnAlaGlyLeu

                              150                             160
         ProThrIleLysPheIleThrGluAlaGlyIleIleGlyLysValGlyPheAsnSerAla

                              170                             180
         GlyValAlaValAsnTyrAsnAlaLeuHisLeuGlnGlyLeuArgProThrGlyValPro

                              190                             200
         SerHisIleAlaLeuArgIleAlaLeuGluSerThrSerProSerGlnAlaTyrAspArg

                              210                             220
         IleValGluGlnGlyGlyMetAlaAlaSerAlaPheIleMetValGlyAsnGlyHisGlu

                              230                             240
         AlaPheGlyLeuGluPheSerProThrSerIleArgLysGlnValLeuAspAlaAsnGly

                              250                             260
         ArgMetValHisThrAsnHisCysLeuLeuGlnHisGlyLysAsnGluLysGluLeuAsp

                              270                             280
         ProLeuProAspSerTrpAsnArgHisGlnArgMetGluPheLeuLeuAspGlyPheAsp

                              290                             300
         GlyThrLysGlnAlaPheAlaGlnLeuTrpAlaAspGluAspAsnTyrProPheSerIle

                              310                             320
         CysArgAlaTyrGluGluGlyLysSerArgGlyAlaThrLeuPheAsnIleIleTyrAsp

                              330                             340
         HisAlaArgArgGluAlaThrValArgLeuGlyArgProThrAsnProAspGluMetPhe

                              350
         ValMetArgPheAspGluGluAspGluArgSerAlaLeuAsnAlaArgLeu
```

**Beispiel 17:** Vergleich der aus der DNA abgeleiteten Aminosäuresequenz der PAT mit den experimentell ermittelten Aminosäuresequenzen:

30kD Einheit/Polypeptid 1 (N–terminale Aminosäuresequenz)

| 2 | ThrThrAlaTyrCysGlnLeuProAsnGlyAlaLeuGlnGlyGlnAsnTrpAspPhePhe | |
| 104 | ThrThrAlaTyrCysGlnLeuProAsnGlyAlaLeuGlnGlyGlnAsnTrpAspPhePhe | |

| 22 | SerAlaThrLysGluAsnLeuIleArg | 30 |
| 124 | SerAlaThrLysGluAsnLeuIleArg | 132 |

AS–Teilsequenz 2 (tryptisches Peptidfragment von Polypeptid 1):

| 1 | GlyAlaThrLeuPheAsnIleIleTyrAspHisAlaArg | 13 |
| 311 | GlyAlaThrLeuPheAsnIleIleTyrAspHisAlaArg | 323 |

AS–Teilsequenz 3 (tryptisches Peptidfragment von Polypeptid 1):

| 1 | ProThrAsnProAspGluMetPheValMetArg | 11 |
| 333 | ProThrAsnProAspGluMetPheValMetArg | 343 |

AS–Teilsequenz 4 (tryptisches Peptidfragment von Polypeptid 1):

| 1 | GluLeuAspProLeuProAspSerTrpAsnArg | 11 |
| 258 | GluLeuAspProLeuProAspSerTrpAsnArg | 268 |

AS–Teilsequenz 5 (tryptisches Peptidfragment von Polypeptid 1):

| 1 | MetGluPheLeu——AspGlyPheAspGlyThrLys | 12 |
| 272 | MetGluPheLeuLeuAspGlyPheAspGlyThrLys | 283 |

AS–Teilsequenz 7 (tryptisches Peptidfragment von Polypeptid 1):

| 1 | IleAlaLeuGluSerThrSerProSerGlnAlaTyrAspArg | 14 |
| 187 | IleAlaLeuGluSerThrSerProSerGlnAlaTyrAspArg | 200 |

AS–Teilsequenz 8 (tryptisches Peptidfragment von Polypeptid 1):

| 1 | ValGlyPheAsnSerAlaGlyValAlaValAsnTyrAsnAlaLeuHisLeuGlnGlyLeu | |
| 155 | ValGlyPheAsnSerAlaGlyValAlaValAsnTyrAsnAlaLeuHisLeuGlnGlyLeu | |
| 21 | ArgProThrGlyValProSerHisIleAlaLeuArg | 32 |
| 175 | ArgProThrGlyValProSerHisIleAlaLeuArg | 186 |

8kD Einheit I/Polypeptid 2a

1        MetLeuHisIleLeuCysGlnGlyThrProPheGluIleGlyTyrGluHisGlySerAla
1        MetLeuHisIleLeuCysGlnGlyThrProPheGluIleGlyTyrGluHisGlySerAla

        AlaLysAlaValIleAlaArgSerIleAspPheAlaValAsp   34
        AlaLysAlaValIleAlaArgSerIleAspPheAlaValAsp   34

8kD Einheit II/Polypeptid 2b

1        MetLeuHisIleLeuCysGlnGlyThrProPheGluIleGlyTyrGluHisGlySerAla
1        MetLeuHisIleLeuCysGlnGlyThrProPheGluIleGlyTyrGluHisGlySerAla

        AlaLysAlaValIleAlaArgSerIleAspPheAlaValAspLeuIleArgGlyLysThr   40
        AlaLys__ ValIleAlaArg__ IleAspPheAlaValAspLeu__ __Gly__ Thr   40

AS–Teilsequenz 9 (tryptisches Peptidfragment von Polypeptid 2):

1        ThrGluPheAlaTyrGlyLeuLys        8
90       ThrGluPheAlaTyrGlyLeuLys        97

AS–Teilsequenz 10 (tryptisches Peptidfragment von Polypeptid 2):

1        TyrTyrAnyGluIleArg     6
65       TyrTyrGluGluIleArg     70

AS–Teilsequenz 11 (tryptisches Peptidfragment von Polypeptid 2):

1        TrpProLys   3
62       TrpProLys   64

AS–Teilsequenz 12 (tryptisches Peptidfragment von Polypeptid 2):

1        SerIleAspPheAlaValAspLeuIleArg   10
28       SerIleAspPheAlaValAspLeuIleArg   37

AS–Teilsequenz 13 (tryptisches Peptidfragment von Polypeptid 2):

1        AspValSerGluIleValMetLeuAsnThrArg      11
79       AspValSerGluIleValMetLeuAsnThrArg      89

AS–Teilsequenz 14 (tryptisches Peptidfragment von Polypeptid 2):

1        GlnValLeuSerGlnLeuGlyArg       8
49       GlnValLeuSerGlnLeuGlyArg      56

AS–Teilsequenz 15 (Peptidfragment von Polypeptid 2 mit Lysylendopeptidase):

6        CysGlnGlyThrProPheGlu      12
1        CysGlnGlyThrProPheGlu      7

AS–Teilsequenz 16 (Peptidfragment von Polypeptid 2 mit Lysylendopeptidase):

65       TyrTyrGluGluIleArgGlyIleAlaLys     74
1        TyrTyrGluGluIleArgGlyIleAlaLys     10

AS–Teilsequenz 17 (Peptidfragment von Polypeptid 2 mit Lysylendopeptidase):

49       GlnValLeuSerGlnLeuGlyArgValIleGluGluArgTrpProLys     64
1        GlnValLeuSerGlnLeuGlyArgValIleGluGluArg__ ProLys     16

AS–Teilsequenz 18 (Peptidfragment von Polypeptid 2 mit Lysylendopeptidase):

75       GlyAlaGluArgAspValSerGluIleValMetLeuAsnThrArg     89
1        GlyAlaGluArgAspValSerGluIleValMetLeuAsnThrArg     15

AS–Teilsequenz 19 (tryptisches Peptidfragment von Polypeptid 2):

23       AlaValIleAlaArg     27
1        AlaValIleAlaArg     5

AS–Teilsequenz 20 (tryptisches Peptidfragment von Polypeptid 2):

42       LysThrAspGluGluLeuLys     48
1        Lys__ AspGluGluLeuLys     7

AS–Teilsequenz 21 (tryptisches Peptidfragment von Polypeptid 2):

71       GlyIleAlaLys     74
1        GlyIleAlaLys     4

AS–Teilsequenz 22 (tryptisches Peptidfragment von Polypeptid 2):

57       ValIleGluGluArg     61
1        ValIleGluGluArg '     5

AS–Teilsequenz 23 (Peptidfragment von Polypeptid 2 mit Lysylendopeptidase):

87       AsnThrArgThrGluPheAlaTyrGlyLeuLys     97
1        AsnThr__ ThrGluPheAlaTyrGlyLeuLys     11

**Zusammenfassung:**

| AS-Teilsequenz | Position der AS in der aus der DNA abgeleiteten AS Sequenz |
|---|---|
| N-terminale AS-Sequenz 30 kD Einheit/Polypeptid 1 | 104 - 113 |
| N-terminale AS-Sequenz 8 kD Einheit/ Polypeptid 2a | 1 - 34 |
| N-terminale AS-Sequenz 8 kD Einheit/Polypeptid 2b | 1 - 40 |
| AS-Teilsequenz 2 (tryptisches Peptidfragment von Polypeptid 1): | 311 - 323 |
| AS-Teilsequenz 3 (tryptisches Peptidfragment von Polypeptid 1): | 333 - 343 |
| AS-Teilsequenz 4 (tryptisches Peptidfragment von Polypeptid 1): | 258 - 268 |
| AS-Teilsequenz 5 (tryptisches Peptidfragment von Polypeptid 1): | 272 - 283 |
| AS-Teilsequenz 7 (tryptisches Peptidfragment von Polypeptid 1): | 187 - 200 |
| AS-Teilsequenz 8 (tryptisches Peptidfragment von Polypeptid 1): | 155 - 186 |
| AS-Teilsequenz 9 (tryptisches Fragment von Polypeptid 2): | 90 - 97 |
| AS-Teilsequenz 10 (tryptisches Fragment von Polypeptid 2): | 65 - 70 |
| AS-Teilsequenz 11 (tryptisches Fragment von Polypeptid 2): | 62 - 64 |
| AS-Teilsequenz 12 (tryptisches Fragment von Polypeptid 2): | 28 - 37 |
| AS-Teilsequenz 13 (tryptisches Fragment von Polypeptid 2): | 79 - 89 |
| AS-Teilsequenz 14 (tryptisches Fragment von Polypeptid 2): | 49 - 56 |
| AS-Teilsequenz 15 (Lysyl-E. von Polypeptid 2): | 6 - 12 |
| AS-Teilsequenz 16 (Lysyl-E. von Polypeptid 2): | 65 - 74 |
| AS-Teilsequenz 17 (Lysyl-E. von Polypeptid 2): | 49 - 64 |
| AS-Teilsequenz 18 (Lysyl-E. von Polypeptid 2): | 75 - 89 |
| AS-Teilsequenz 19 (tryptisches Fragment von Polypeptid 2): | 23 - 27 |
| AS-Teilsequenz 20 (tryptisches Fragment von Polypeptid 2): | 42 - 48 |
| AS-Teilsequenz 21 (tryptisches Fragment von Polypeptid 2): | 71 - 74 |
| AS-Teilsequenz 22 (tryptisches Fragment von Polypeptid 2): | 57 - 61 |
| AS-Teilsequenz 23 (Lysyl-E. von Polypeptid 2): | 87 - 97 |

**Beispiel 18: Teilsequenz eines DNA-Fragmentes, das zwischen dem Gen für die Isopenicillin N-Synthetase (ips) und dem Gen für PAT liegt:**

Diese Sequenz beginnt mit "GGATCC", der Erkennungs-Sequenz des Restriktionsenzyms BamHI, die im ips-Gen liegt. Die 34 letzten (C-terminalen) Codons dieses Gens sind gezeigt; sie wurden durch einen Vergleich mit der veröffentlichten DNA-Sequenz identifiziert (Carr. et al., Gene 48, 257-266, 1986). Nach einem Zwischenbereich folgt bei 1442 die Position 1 der pat-DNA-Sequenz. Diese DNA-Teilsequenz ist in der Abb. 4 eingezeichnet (B).

Diese DNA-Teilsequenz ist wichtig, da sie die enge Koppelung zwischen ips- und pat-Gen belegt, und weil alle wesentlichen Kontrollelemente für Transkription und Translation des pat-Gens auf diesem Fragment liegen.

```
            10          20          30          40          50
GGATCCTAGCAAGGAAGACGGCAAGACCGATCAGCGGCCAATCTCGTACG
   AspProSerLysGluAspGlyLysThrAspGlnArgProIleSerTyrG

            60          70          80          90          100
GGGACTATCTGCAGAACGGATTAGTTAGTCTAATCAACAAGAACGGCCAG
   lyAspTyrLeuGlnAsnGlyLeuValSerLeuIleAsnLysAsnGlyGln

            110         120         130         140         150
ACATGAAAGGGCCCATGGATGGGACCGGGATGGAAATCCCGGACTCTGAG
ThrEnd

                                                       850
NNNNNNNNNNNNNNNNNNN   1250 bp   NNNNNNNNNNNNNNNNNNNNN

            1410       1420       1430       1440        1450
CAAGACTAGGCGGATGCAGCAGGGATACTCGAGGTGCCCCAGTTGATGTC

            1460       1470       1480       1490        1500
CCATCAGTGTCATGCTATGGTCCCAGATTGGTGGCTACGGCCAATATAAA

            1510       1520       1530       1540        1550
TCTCAGCATGCAGTTCCGCCTGCATGATCATCCCCAGGACGCCCGCAGCA

            1560       1570       1580       1590
GAAATGCTTCACATCCTCTGTCAAGGCACTCCCTTTGAAGTA
   MetLeuHisIleLeuCysGlnGlyThrProPheGlu
```

**Beispiel 19: Konstruktion des Plasmides pBC2001 und Expression des pat-Gens in E.coli:**

a) Konstruktion einer cDNA-Genbank von Penicillium chrysogenum:

5 $\mu$g der poly(A)$^+$-RNA, deren Isolierung und Reinigung in Beispiel 10 beschrieben ist, wird mit Hilfe von Reverser Transkriptase mit einem oligo dT-Primer in Gegenwart der vier Desoxynukleosidtriphosphate in eine komplementäre einzelsträngige DNA umgesetzt. Durch die Enzyme RNaseH und DNA-Polymerase wird daraus ein doppelsträngiges Molekül gebildet (Gubler und Hoffman, Gene 25, 263, 1983). Nach dem Anfügen von geeigneten EcoRI-Adaptoren, wozu die Enzyme Polynukleotidkinase und T4-DNA-Ligase verwendet werden, erhält man eine lineare doppelsträngige cDNA, die in Kloniervektoren eingebaut werden kann. Für diese Umsetzungen läßt sich günstigerweise ein käuflicher cDNA-Synthese-Kit (Fa. Pharmacia) verwenden, der die wichtigsten Enzyme und das Adapter-Oligonukleotid enthält. Die Durchführung der Reaktionen erfolgt nach den Angaben des Herstellers. Die so synthetisierte doppelsträngige cDNA mit EcoRI-Enden wird in den Vektor gt10 (Huynh et al., DNA cloning, Glover, D.M. ed., Oxford, 1, 49, 1985) einkloniert. 80 $\mu$l der cDNA-Präparation werden dazu mit 16 $\mu$l gt10-DNA (8 $\mu$g) vermischt, die vorher mit EcoRI gespalten und mit alkalischer Phosphatase behandelt wurde (Maniatis et al.). Nach Zugabe von 3 $\mu$l 3 M Natriumazetat (pH 5,2) und 250 $\mu$l Ethanol wird für 20 Stunden bei -20° gefällt und anschließend in 60 $\mu$l 10 mM Tris-HCl (pH 7,5), 1 mM EDTA gelöst. Die Ligation erfolgt in 66 mM Tris-HCl (pH 7,5), 1 mM Spermidin, 10 mM MgCl$_2$, 15 mM Dithiothreitol, 0,2 mg/ml BSA, 0,5 mM ATP durch die Zugabe von 6 U T4-DNA-Ligase bei 12° für 20 Stunden. Das Ligationsgemisch wird mit Proteinextrakten ("packaging mixe") in vitro verpackt und mit dem E.coli-Stamm C600hfl (Huynh et al.) ausplattiert. Die erforderlichen Methoden sind beschrieben (Maniatis et al.). In einem solchen Versuch können mehr als 5.10$^5$ Plaques erhalten werden.

b) Isolierung von pat-spezifischen cDNA-Klonen und Suklonierung in M13mp19.

Mit etwa 40000 Plaques der Penicillium chrysogenum cDNA-Genbank wird eine Plaquehybridisierung durchgeführt, wie sie in Beispiel 13 beschrieben ist. Als Indikatorstamm wird der E.coli-Stamm C600hfl (Huynh et al.) verwendet. Die DNA der rekombinanten gt10-Phagen wird nach einer partiellen Spaltung mit EcoRI mit EcoRI-gespaltener M13mp19-RF-DNA ligiert und transfiziert. Einzelne Klone rekombinanter M13mp19-RF-DNA werden durch Restriktionskartierung identifiziert und bestätigt.

c) Ortsspezifische Mutagenese der DNA des rekombinanten M13mp19 Klones zur Einfügung einer NcoI-Schnittstelle.

Es wird ein 41mer Oligonukleotid mit folgender Sequenz synthetisiert:

**5´-TCCGACCCGCAGCAGCCATGGTTCACATCCTCTGTCAACGC-3´**

Die DNA-Sequenz ist gegenüber der DNA-Sequenz der pat-cDNA so verändert, daß eine NcoI-Schnittstelle entsteht, die das ATG einschließt, das mit dem N-terminalen Methioninrest korrespondiert. Rechts davon sind 20, links davon 15 Nukleotide identisch mit der pat-cDNA-Sequenz. 5 pmol des phosphorylierten Oligonukleotides und 0,5 pmol der einzelsträngigen M13mp19-DNA werden gemischt und in 10 $\mu$l 20 mM Tris-HCl(pH7,5), 10 mM MgCl$_2$, 50 mM NaCl für 5 Minuten auf 65° und für 20 Minuten auf 42° erwärmt Die Behandlung mit Klenow Polymerase, T4-DNA-Ligase und S1-Nuklease erfolgt nach Eghtedarzadeh und Henikoff (Nucl. Acids Res. 14, 5115,1986): 10$\mu$l Lösung mit 2 Einheiten Klenow Polymerase und 3 Einheiten T4-DNA-Ligase in 20 mM Tris-Cl (pH7,5), 10 mM MgCl$_2$, 10 mM Dithiothreit, 0,8 mM von jedem der vier dNTPs, 1 mM ATP, 1 Stunde bei 42°. Die Reaktion wird durch die Zugabe von EDTA abgestoppt (Endkonzentration 25mM) und 10 Minuten auf 70° erhitzt. Nach einer Ethanolfällung wird das Pellet in 10 $\mu$l Tris-HCl (pH 8), 1 mM EDTA gelöst. Nach der Zugabe von 15 $\mu$l 30 mM Kaliumazetat (pH 4,6), 0,25 M NaCl, 1 mM ZnCl$_2$, 5% Glycerin, 7 Einheiten S1 Nuklease wird 20 Minuten bei Raumtemperatur inkubiert. Nach einer Transfektion eines geeigneten E.coli-Stammes (z.B. JM101; Yanisch-Perron et al., Gene 33, 103, 1985) mit dem Reaktionsansatz und der Isolierung von RF-DNA können die veränderten DNAs durch Spaltung mit NcoI identifiziert werden.

d) Klonierung des NcoI-HindIII-Fragmentes mit der pat cDNA in das Plasmid pKK233-2.

Die RF-DNA des rekombinanten M13-Klones, der die pat cDNA mit der eingeführten NcoI-Schnittstelle enthält, wird mit NcoI und HindIII gespalten. Das Plasmid pKK233-2 (Amann und Brosius, Gene 40, 183, 1985) wird ebenfalls mit NcoI und HindIII gespalten. Die Ligation der beiden DNAs erfolgt in 66 mM Tris-HCl (pH 7,5), 1 mM Spermidin, 10 mM MgCl$_2$, 15 mM Dithiothreitol, 0,2 mg/ml BSA, 0,5 mM ATP durch die Zugabe von 2 U T4-DNA-Ligase bei 14° für 20 Stunden. Nach einer Transformation eines geeigneten E.coli-Stammes (z.B. JM83; Yanisch-Perron et al., Gene 33, 103, 1985) werden einzelne Plasmid-DNAs isoliert und durch Restriktionskartierung charakterisiert. Das Plasmid, das das NcoI-HindIII-Restriktionsfragment mit der pat cDNA im Plasmid pKK233-2 enthält, wird pBC2001 genannt.

c) Expression des pat-Gens in E. coli

E.coli RB791 (Amann und Brosius, Gene 40, 183, 1985) wird mit pBC2001 transformiert. 50 ml LB Medium (pro Liter: 10 g Bacto Tryptone, 8 g NaCl, 5 g Hefe Extrakt; pH 7,5 wird mit NaOH eingestellt) werden mit einer Einzelkolonie des Stammes RB791 (pBC2001) angeimpft und bei 37° bei 200 Upm geschüttelt, bis eine optische Dichte von 0,5 erreicht ist (gemessen bei 600 nm). 5 ml einer 0,1 M IPTG (Isopropyl-$\beta$-D-thiogalactosid) werden zugegeben. Anschließend wird weitere 3 Stunden bei 37° und 200 Upm geschüttelt. Die Zellen werden dann abzentrifugiert (10 Minuten, 5000 upm, 20° Beckman JA20) und für einen Nachweis des in E.coli exprimierten Proteins aufgearbeitet. Dieser Nachweis kann dann durch eine SDS Polyacrylamidgelelektrophorese des E.coli Gesamtproteins, durch einen Western-Blot mit Hilfe von PAT-spezifischen Antikörpern oder durch einen enzymatischen Nachweis erfolgen.

**Beispiel 20: Transformation des pat-Gens nach Penicillium chrysogenum**

a) Konstruktion des Plasmides pBC2002:

Des Plasmid pHS103 (Kolar et al., Gene 62, 127, 1988) wird mit EcoRI vollständig gespalten und in Gegenwart von EcoRI-SalI gespaltener M13mp19-RF-DNA wieder ligiert (66-mM Tris-HCl (pH 7,5), 1 mM Spermidin, 10 mM MgCl$_2$, 15 mM Dithiothreitol, 0,2 mg/ml BSA, 0,5 mM ATP durch die Zugabe von 2 U T4-DNA-Ligase bei 14° für 20 Stunden). Nach einer Transformation steht damit ein Plasmid zur Verfügung, das sich für die Klonierung von SalI-Fragmenten eignet. Dieses Plasmid wird mit SalI gespalten, ebenso ein rekombinantes Plasmid mit einem 4,8 kb großen SalI Fragment mit dem vollständigen pat-Gen (siehe Beispiel 13). Nach Ligation und Transformation läßt sich ein Plasmid identifizieren, das aus dem modifizierten pHS103 und dem 4.8 kb SalI-Fragment mit dem pat-Gen besteht; dieses Plasmid wird pBC2002 genannt.

b) Isolierung von Penicillium chrysogenum Protoplesten und Transformation:

2 ml einer dichten Sporensuspension von Penicillium chrysogenum P2/ATCC werden zu 200 ml sterilem Minimalmedium für Penicillium chrysogenum in einem 1 l Erlenmeyerkolben gegeben (pro Liter: 3 g $NaNO_3$, 0,5 g $MgSO_4$.$7H_2O$, 0,5 g KCl, 10 mg $FeSO_4$.$7H_2O$, 20 g Saccharose, 13 g $KH_2PO_4$ und 1 ml Spurenelementmischung (für 100 ml: 0,1 g $FeSO_4$.$7H_2O$, 0,9 g $ZnSO_4$.$7H_2O$, 0,04 g $CuSO_4$.$5H_2O$, 0,01 g $MnSO_4$.$H_2O$, 0,01 g $H_3BO_3$, 0,01 g $Na_2HPO_4$.$2H_2O$)) und 20 Stunden bei 25° und 250 Upm geschüttelt. Die Isolierung und Reinigung der Protoplasten erfolgt nach Yelton et al. (Proc. Natl. Acad. Sci. U.S.A., 81, 1470, 1984).

Das Mycel wird abzentrifugiert und zweimal in 0,9 M NaCl gewaschen und in 20 ml 0,9 M NaCl mit 5 mg/ml Novozyme 234 resuspendiert. Inkubation bei 30° für 1,5 Stunden. Der Reaktionsansatz wird zentrifugiert (Beckman Kühlzentrifuge JS 7.5, 1500 Upm, 20°, 5 Minuten). Das Protoplastenpellet wird zweimal mit 0,9 M NaCl und anschließend einmal in 1,0 M Sorbitol, 50 mM $CaCl_2$ gewaschen. Die Protoplesten werden in 0,2 ml 1,0 M Sorbitol, 50 mM $CaCl_2$ resuspendiert (etwa 0,5 bis $5.10^8$ Protoplasten/ml).

5 $\mu$g pBC2002 in 10 mM Tris-HCl (pH 7,5), 1 mM EDTA werden für die Transformation eingesetzt und zur Protoplastensuspension gegeben. 12,5 $\mu$l 25 % Polyethylenglycol (BDH), 10mM Tris-HCl pH 7,5, 50mM $CaCl_2$ (sterilfiltriert) werden zugegeben. Inkubation für 20 Minuten im Eis. 0,5 ml 12,5 $\mu$l 25 % Polyethylenglycol (BDH), 10 mM Tris-HCl (pH 7,5), 50 mM $CaCl_2$ (sterilfiltriert) werden zugegeben: die Mischung wird für 5 Minuten bei 20° inkubiert. Nach der Zugabe von 1 ml 0,9 M NaCl, 50 mM $CaCl_2$ wird durchmischt und zu 3 ml Minimalmedium mit 0,5 % Agar und 20 $\mu$g Phleomycin/ml (48°) gegeben. Ausplettiert werden die Transformationsansätze auf Minimelmedium-Platten (Minimalmedium wie oben beschrieben, mit 1,6 % Agar und 20 $\mu$g Phleomycin/ml).

DNA, die aus verschiedenen Kolonien isoliert wird, kann durch Southernhybridisierung mit radioaktiv markierter pat-DNA charakterisiert werden. Unter den Transformanten lassen sich so diejenigen herausfinden, die auf Grund von multiplen Integrationsereignissen mehrere Kopien des pat-Gens enthalten. Solche Stämme werden anschließend durch Testfermentationen auf eine erhöhte Penicillinbildung getestet.

**Tabelle 1:** Aufschluß und Fällung der thiolabhängigen PAT aus Penicillium chrysogenum P2/ATCC 48271

| Reinigung | Aufschluß-dauer (min) | Gesamt-volumen (ml) | Protein (mg ges.) | Protein (mg/ml) | ÜST unverdünnt [1] sofort | 30' | 30'+ Penase | ÜST 1:10 sofort | 30' | 30'+ Penase | ÜST 1:50 sofort | 30' | 30'+ Penase |
|---|---|---|---|---|---|---|---|---|---|---|---|---|---|
| Zellaufschluß mit Dyno mill Type KDL (1786 g Bfm = 220 g TS in 3.5 l) | 0 | 4900 | 676 | 0.138 | 0 | 0 | 0 | 0 | 0 | 0 | 0 | 0 | 0 |
| 0.1 M Phosphatpuffer pH 7.5 + 5 mM DTT + 0.1% Triton X 100 + 0.1 mM PMSF 600 ml Stahlmahlbehälter | 30 | | 20776 | 4.24 | Spur | 18.0 | 0 | 0 | 24.4 | 0 | 0 | Spur | 0 |
| 510 ml 500-750 µm Glasperlen | 60 | | 25431 | 5.19 | Spur | 17.9 | 0 | Spur | 27.0 | 0 | 0 | 15.7 | 0 |
| Im kont. Betrieb: Drehzahl: 2000 Upm (= 6.7 m/sek) Reibspaltbreite: 0.1 mm Flußrate Mahlgut = 30 l/h Temp. Mahlgut VL 2/3°C RL 5°C | 90 | | 29204 | 5.96 | 15.2 | 22.0 | 0 | 13.8 | 29.9 | 0 | 0 | 16.5 | 0 |
| Temp. Kühlung VL –20/–8°C RL –5/–1°C | 120 | | 29302 | 5.98 | Spur | 17.1 | 0 | 0 | 24.8 | 0 | 0 | 0 | 0 |
| ÜST nach Nukleinsäurefällung mit 0.5% CTAB | | 4650 | 12927 | 2.78 | 14.4 | 26.0 | 0 | 0 | 25.8 | 0 | 0 | 0 | 0 |
| Proteinpräzipitat mit 50 GV % Ammonsulfat | | 480 (= 544 g) | 10965 | 22.8 | 27.4 [2] | 28.8 | 0 | 18.4 | 28.2 | 0 | 16.1 | 27.1 | 0 |

1) Die unverdünnten Aufschlußproben wurden vor der Aktivitätstestung über PD 10 Säulen pufferausgetauscht (Abtrennung des Restgehalts an Penicillin V. 2.5 ml ÜST-Probe mit 3.5 ml 0.1 M Phosphatpuffer pH 7.5 + 1 mM DTT) )

2) $(NH_4)_2SO_4$-Präzipitat vor Aktivitätstestung mit 0.1 M Phosphatpuffer pH 7.5 + 1 mM DTT 1:5 verdünnt

Penase = Penicillinase

## Tabelle 2: Stabilität bzw. Aktivität der thiolabhängigen PAT aus Penicillium chrysogenum P2/ATCC 48271 in Abhängigkeit von der Temperatur, Zeit, pH-Wert und Stabilisatorzusätzen

| Testung | sofort | | | 24 h bei +4°C | | | 24 h bei -20°C | | |
|---|---|---|---|---|---|---|---|---|---|
| Stabilisierungskonditionen | Mikrobiologische 6-APA-PaCoA Acylierungsaktivität (mm HH ⊘ gegen Micrococcus luteus ATCC 9341/BC 85) | | | | | | | | |
| | sofort | 30' | 30'+ Penase | sofort | 30' | 30'+ Penase | sofort | 30' | 30'+ Penase |
| **Aktivierung** | | | | | | | | | |
| ohne Zusatz | 11 | 15.5 | 0 | – | – | 0 | – | – | 0 |
| + 1 mM DTT | 23.5 | 37.1 | 0 | 0 | Spur | 0 | 15 | 38 | 0 |
| + 10 mM DTT | 22.7 | 39.0 | 0 | 16.6 | 36 | 0 | 16 | 33 | 0 |
| + 10 mM ß-ME | 15.4 | 27.8 | 0 | 0 | Spur | 0 | Spur | 28.3 | 0 |
| + 10 mM MgCl$_2$ | 9.8 | 17.1 | 0 | 0 | Spur | 0 | 18.5 | 15 | 0 |
| + 2 mM EDTA | 0 | 16.0 | 0 | – | – | 0 | – | – | 0 |
| + 1 mM DTT + 10 mM MgCl$_2$ | 26.9 | 36.7 | 0 | 0 | Spur | 0 | 12.2 | 37 | 0 |
| + 2 mM EDTA + 10 mM MgCl$_2$ | 16.3 | 25.4 | 0 | 0 | 14.8 | 0 | Spur | 23 | 0 |
| + 5% Sorbit + 1 mM DTT + 2 mM EDTA + 1 mM PMSF | 26.0 | 38.6 | 0 | 0 | Spur | 0 | 11.3 | 33 | 0 |
| + 5% Sorbit + 1 mM DTT + 1 mM PMSF | 25.5 | 36.6 | 0 | 0 | Spur | 0 | 16.8 | 36 | 0 |
| **pH-Profil** | | | | | | | | | |
| 0.1 M HEPES          pH 7.5 + 1 mM DTT | 26.6 | 37.3 | 0 | 0 | Spur | 0 | 12.5 | 33.5 | 0 |
| 0.1 M NaH$_2$PO$_4$ / Na$_2$HPO$_4$   pH 6.0 + 1 mM DTT | 0 | 17.3 | 0 | – | – | 0 | – | – | 0 |
| 0.1 M NaH$_2$PO$_4$ / Na$_2$HPO$_4$   pH 7.0 + 1 mM DTT | 22.1 | 32.7 | 0 | 0 | 11.2 | 0 | 0 | 29.3 | 0 |
| 0.1 M NaH$_2$PO$_4$ / Na$_2$HPO$_4$   pH 8.0 + 1 mM DTT | 33.8 | 35.0 | 0 | 0 | 13 | 0 | 17 | 36.5 | 0 |
| 0.1 M TRIS/HCl          pH 8.0 + 1 mM DTT | 14.3 | 19.6 | 0 | 0 | 0 | 0 | 0 | 16 | 0 |
| 0.1 M TRIS/HCl          pH 9.0 + 1 mM DTT | 0 | Spur | 0 | – | – | 0 | – | – | 0 |
| **Inhibierung** | | | | | | | | | |
| ohne Zusatz | 22.6 | 33.6 | 0 | Spur | Spur | 0 | 11.6 | 34 | 0 |
| + 2 mM PMSF | 22.1 | 32.2 | 0 | 0 | Spur | 0 | 13.8 | 27 | 0 |
| + 2 mM DIPFP | 22.6 | 34.5 | 0 | 0 | Spur | 0 | 10 | 35 | 0 |
| + 2 mM 2.2'-Bipyridyl | 0 | 0 | 0 | – | – | 0 | – | – | 0 |
| + 2 mM Hydroxychinolin | n. | 34.5 | 0 | 0 | 12 | 0 | 13.5 | 38 | 0 |
| + 2 mM o-Phenanthrolin | 18.9 | 29.9 | 0 | Spur | Spur | 0 | 11 | 33 | 0 |
| + 2 mM N-Ethylmaleinimid | 0 | 0 | 0 | – | – | 0 | – | – | 0 |
| + 2 mM Jodacetamid | 0 | 0 | 0 | – | – | 0 | – | – | 0 |
| + 2 mM 4-Hydroxymercuri-Na-benzoat | 19.4 | 32.3 | 0 | 0 | 11.6 | 0 | 12.2 | 39 | 0 |

**Tabelle 2: Fortsetzung**

| Testung | sofort | | | 24 h bei +4°C | | | 24 h bei −20°C | | |
|---|---|---|---|---|---|---|---|---|---|
| Stabilisierungskonditionen | Mikrobiologische 6-APA-PaCoA Transacylierungsaktivität (mm HH ⊘ gegen Micrococcus luteus ATCC 9341/BC 85) | | | | | | | | |
| | sofort | 30' | 30'+ Penase | sofort | 30' | 30'+ Penase | sofort | 30' | 30'+ Penase |
| **Stabilisierung** | | | | | | | | | |
| ohne Zusatz | 21.5 | 33.4 | 0 | 0 | Spur | 0 | 15.2 | 38 | 0 |
| + 5% Sorbit | 23.3 | 34.1 | 0 | 0 | 12.2 | 0 | 16.3 | 38 | 0 |
| + 20% Sorbit | 21.9 | 34.2 | 0 | 0 | 12 | 0 | 15.2 | 37 | 0 |
| + 1 mM PMSF | 22.0 | 33.7 | 0 | 0 | Spur | 0 | 15 | 40 | 0 |
| + 1% BSA | Spur | 20.0 | 0 | 0 | 0 | 0 | 0 | 18 | 0 |
| + 10% Glycerin | 22.0 | 33.1 | 0 | 0 | 11.5 | 0 | 15.2 | 38 | 0 |
| + 5% Mannit | 24.0 | 36.2 | 0 | 0 | Spur | 0 | 16.8 | 38 | 0 |
| + 100 µg 6-APA/ml | 26.8 | 35.4 | 0 | 15 | 24 | 0 | 17.2 | 38 | 0 |

| µg/ml Penicillin V Standard | mm HH ⊘ gegen BC 85 |
|---|---|
| 2 | 27.4 |
| 1.5 | 24.7 |
| 1.0 | 23.4 |
| 0.5 | 18.4 |
| 0.25 | 11.9 |

Enzym: PAT-Ammonsulfatpräzipitat

Lagerung und Testung → 1 + 10 mit 0.1 M HEPES Reaktionspuffer pH 7.5 unter verschiedenen Stabilisierungskonditionen

EP 0 336 446 B1

**Tabelle 3: Temperaturstabilität der PaCoA-6-APA-Acyltransferaseaktivität aus Penicillium chrysogenum**

| DTT (mM) | Inkubations-dauer | -196°C | | | -20°C | | | +4°C | | | RT | | | 37°C | | |
|---|---|---|---|---|---|---|---|---|---|---|---|---|---|---|---|---|
| | | Mikrobiologische 6-APA-PaCoA Transacylierungsaktivität (mm HH ⊘ gegen Micrococcus luteus ATCC 9341/BC 85) | | | | | | | | | | | | | | |
| | | sofort | 30' | 30'+Penase | sofort | 30' | 30'+Penase | sofort | 30' | 30'+Penase | sofort | 30' | 30'+Penase | sofort | 30' | 30'+Penase |
| 1 | sofort | 14 | 40 | .0 | 14 | 40 | 0 | 14 | 40 | 0 | 14 | 40 | 0 | 14 | 40 | 0 |
| | 1 h | 14 | 36 | 0 | Spur | 38 | 0 | Spur | 38 | 0 | 10 | 37 | 0 | 0 | Spur | 0 |
| | 4 h | 12 | 37 | 0 | 17 | 36 | 0 | 12 | 36 | 0 | 0 | 16 | 0 | 0 | 0 | 0 |
| | 26 h | 17 | 33 | 0 | 17 | 35 | 0 | 10 | 32 | 0 | Spur | 14 | 0 | 0 | 0 | 0 |
| | 120 h | 26 | 32 | 0 | 23 | 34 | 0 | 0 | Spur | 0 | 0 | 0 | 0 | 0 | 0 | 0 |
| 10 | sofort | 24 | 40 | 0 | 24 | 40 | 0 | 24 | 40 | 0 | 24 | 40 | 0 | 24 | 40 | 0 |
| | 1 h | 15 | 35 | 0 | 11 | 35 | 0 | 14 | 34 | 0 | 13 | 34 | 0 | 0 | 17 | 0 |
| | 4 h | – | – | – | 12 | 34 | 0 | 11 | 32 | 0 | 11 | 28 | 0 | 0 | 0 | 0 |
| | 26 h | 18 | 32 | 0 | 17 | 34 | 0 | 11 | 27 | 0 | 0 | Spur | 0 | 0 | 0 | 0 |
| | 120 h | – | – | – | 28 | 32 | 0 | 0 | Spur | 0 | 0 | 0 | 0 | 0 | 0 | 0 |

Enzym: AS–Präzipitat / 1:20 mit RP verdünnt, zentrifugiert, ÜST → Test

Lagerung: 0,1 m PP pH 7,5 inklusive 1 bzw. 10 mM DTT

EP 0 336 446 B1

Tabelle 4: Substratspezifität der gereinigten thiolabhängigen PAT aus Penicillium chrysogenum P2/ATCC 48271
(aktive Fraktion AL 278/2 nach Affinitätschromatographie)

| Substrat | | Produkt | mittels HPLC bestimmte spezifische Enzymaktivität (mU/mg Protein) A1 278/2 |
|---|---|---|---|
| Acylakzeptor | Acyldonator | | |
| Isopenicillin N | PaCoA | Penicillin V | 146 |
| Isopenicillin N | PeCoA | Penicillin G | 80 |
| 6-APA | PaCoA | Penicillin V | 393 |
| 6-APA | PeCoA | Penicillin G | 329 |
| 7-ADCA | PaCoA | 3-Desacetoxy-cephalosporin V | 49 |
| 7-ACA | PaCoA | Cephalosporin V | 0 |
| Cephalosporin C | PaCoA | Cephalosporin V | 0 |
| Penicillin G | PaCoA | Penicillin V | 25 |
| Penicillin V | PeCoA | Penicillin G | 99 |
| $H_2O$ | Isopenicillin N | 6-APA | 150 |
| $H_2O$ | Penicillin V | 6-APA | 75 |
| $H_2O$ | Penicillin G | 6-APA | 17 |
| $H_2O$ | 7-Glutaryl-APA | 6-APA | 106 |

EP 0 336 446 B1

Abbildung 1: Reinigung der thiolabhängigen PAT aus Penicillium chrysogenum P2/ATCC 48271

Chromatofokussierung der affinitätschromatographisch hergestellten PAT–Enzympräparation AL 278/2 auf Mono P = AL 310

| | |
|---|---|
| Säule: | Pharmacia Mono P |
| Bettvolumen: | 4 ml |
| Laufmittelpuffer: | A:  25 mM bis TRIS–Puffer pH 6,3; HCl |
| | B:  10 ml Polypuffer 74/100 ml pH 4,0; HCl |
| Probe: | Affinitätsprobe AL 278/2 |
| Probenvolumen: | 1 ml gegen Puffer a pufferausgetauscht, |
| | davon 2 ml aufgegeben |
| Papiervorschub: | 30 cm/h |
| Fluß: | 60 ml/h |
| OD: | 0,1; 280 nm; HR–10–Zelle |
| Fraktionen: | siehe Markierungen |

pH 7,0
pH 6,0
pH 5,0
pH 4,0

310/4
310/5
310/3

0,07 OD

Optische Dichte 280 nm

pH–Kurve

OD 280 nm

Elutionsvolumen ⟶

**Abbildung 2: Reinigung der thiolabhängigen PAT aus P. chrysogenum P2/ATCC 48271**

RPC (MN Nucleosil 300–5/C4) von Fraktionen 3–5 aus AL 310/Chromatofokussierung des aktiven Pools AL 278/2 (nach Affinitätschromatographie) auf Mono P

neutrale PAT-Isovariante
pI 5,15 – 5,17 (Immobiline)                                    AL 310/4

saure PAT-Isovariante
pI 5,06 – 5,07 (Immobiline)                                    AL 310/5

basische PAT-Isovariante
pI 5,32 (Immobiline)                                           AL 310/3

**Abbildung 3: Elektrophoretische Charakterisierung der thiolabhängigen PAT aus Penicillium chrysogenum P2/ATCC 48271**

Gradienten SDS–Polyacrylamidgelelektrophorese und isoeletrische Fokussierung auf Ampholine (pH 3,5 – 9,5) bzw. Immobiline (pH 4,5 – 6,5) von AL 310/Chromatofokussierung der affinitätschromatographisch hergestellten PAT-Enzympräparation AL 278/2 auf Mono P.

3a: Gradienten SDS–PAGE (8–20% T)

| Spur | Bezeichnung | |
|---|---|---|
| 1 | Einsatz 278/2 | |
| 2 | Low-MW-Std. 66, 45, 31, 22, 14 kD | |
| 3 | AL 310/7 | pH–Bereich von |
| 4 | AL 310/6 | 6.0 – 4.0 ( → ) |
| 5 | AL 310/5 | |
| 6 | AL 310/4 | A  25 mM bis-TRIS/HCl  pH 6,3 |
| 7 | AL 310/3 | |
| 8 | AL 310/2 | B  Polypuffer 74 10%ig pH 4.0 |
| 9 | AL 310/1 | |
| 10 | Low-MW-Std. 66, 45, 31, 22, 14 kD | |

3b: Ampholine (pH 3,5 – 9,5)

| Spur | Bezeichnung |
|---|---|
| 1 | pI-Std. Pharmacia von – nach +  (9.30; 8.65; 8.45; 8.15; 7.35; 6.85; 6.55; 5.85; 5.20; 4.55; 3.50) |
| 2 | AL 278/2 |
| 3 | Carboanhydrase neu hergestellt |

3c: Immobiline (pH 4,5 – 6,5)

| Spur | Bezeichnung | Herkunft |
|---|---|---|
| 1 | AL 278/2 | Affinitätschromatogr., 6-APA-AH-Seph. |
| 2 | AL 310/5 | |
| 3 | AL 310/4 | Chromatofokussierung |
| 4 | AL 310/3 | |
| 5 | Carboan-hydrase | |

**Abbildung 4: Restriktionskarte aus Penicillium chrysogenum, die das pat-Gen umfaßt.**

Restriktionskarten sind ungefähre Wiedergaben von Restriktionsschnittstellen in DNA–Molekülen. Die gezeichneten Abstände der Restriktionsschnittstellen sind proportional zu den tatsächlichen Abständen, aber die tatsächlich beobachteten Abstände können sich davon unterscheiden. Nicht alle Restriktionsschnittstellen sind angegeben, es können durchaus weitere Schnittstellen vorhanden sein.

1972

A

1

1592

B

1

ips

A = Sequenz aus den Beispielen 14 und 15

B = Sequenz aus dem Beispiel 18

1 kb

EcoRI

XhoI

BamHI

EcoRI

BamHI

SalI

SalI

EcoRI

XhoI

BamHI

SalI

EcoRI

XhoI

BamHI

SalI

BamHI

SalI

EcoRI

BamHI

## Abbildung 5: Anordnung und Sequenz der ausgewählten Oligonukleotidgemische

```
          5               10              15              20
ThrThrAlaTyrCysGlnLeuProAsnGlyAlaLeuGlnGlyGlnAsnTrpAspPhePheSer      Aminosäuresequenz Polypeptid 1


5'-ACNACNGCNTATTGTCAACTNCCNAATGGNGCNCTNCAAGGNCAAAATTGGGATTTTTTTAGT-3'
      G  G  GTTA      C      TTA       G. C      C  C  C  C
            G.                G                          TCN


3'-TGGTGACGGATAACAGTTGAAGGTTTACC.............GTTTTGACCCTAAAAAA-5'        Oligonukleotidgemisch 1
                                              C         G

                                              GTTTTAACCCTAAAAAA          Oligonukleotidgemisch 2
                                              C         G

                                              GTTTTGACCCTGAAAAA          Oligonukleotidgemisch 3
                                              C         G

                                              GTTTTAACCCTGAAAAA          Oligonukleotidgemisch 4
                                              C         G
```

EP 0 336 446 B1

Abbildung 6: Schematische Darstellung der Zusammenhänge zwischen den sequenzierten Bereichen (D,F,G), den Oligonukleotiden (C,E), dem Polypeptid 1 der PAT und der Sequenz des daraus abgeleiteten kodierenden DNA-Stranges (B).

D = Sequenz aus Beispiel 11.
F = Sequenz aus Beispiel 13.
G = Teil der Sequenz aus Beispiel 14 und 15

EP 0 336 446 B1

**Abbildung 7: Konstruktion des Plasmides pBC2001.**

Ausgehend von der DNA eines vollständigen pat–cDNA–Klons (oberste Zeile), werden die beiden EcoRI–Fragmente in den Vektor M13mp19 einkloniert. Mit Hilfe eines Oligonukleotides wird eine NcoI-Schnittstelle eingeführt. Das NcoI–HindIII–Fragment wird dann in den Expressionsvektor pKK233–2 eingebaut.

EcoRI, HindIII, NcoI markieren die Schnittstellen der entsprechenden Enzyme; mcs multiple Klonierungsstelle ("multiple cloning site") aus M13mp19; p trc trp–lac Fusionspromotor aus pKK233–2; rrnBT1T2 Transkriptionsterminator aus pKK233–2; bla Ampicillin–Resistenzgen aus pKK233–2.

**Abbildung 8: Das Plasmid pBC2002**

Die Konstruktion dieses Plasmides geht von pHS103 (Kolar et al.) aus, das eine Fusion des Aspergillus nidulans Promotors p gpd (Promotor des Glycerinaldehydphosphat-Dehydrogenase Gens) mit dem Phleomycin–Resistenzgen (ble) enthält. Das Ampicillin-Resistenzgen (bla) dient als Selektionsmarke in E.coli. In pHS103 wird das 4,8 kb Sall–Fragment mit dem Penicillium chrysogenum pat–Gen eingebaut. Sall, EcoRI sind die Schnittstellen der entsprechenden Restriktionsenzyme.

**Patentansprüche**
**Patentansprüche für folgende Vertragsstaaten : AT, BE, CH, DE, FR, GB, IT, LI, LU, NL, SE**

1. Eine DNA-Sequenz in gereinigter und isolierter Form, die für das Enzym Penicillinacyltransferase, das die folgende Sequenz aufweist, kodiert

```
                                    10                              20
         MetLeuHisIleLeuCysGlnGlyThrProPheGluIleGlyTyrGluHisGlySerAla
                                    30                              40
         AlaLysAlaValIleAlaArgSerIleAspPheAlaValAspLeuIleArgGlyLysThr
                                    50                              60
         LysLysThrAspGluGluLeuLysGlnValLeuSerGlnLeuGlyArgValIleGluGlu
                                    70                              80
         ArgTrpProLysTyrTyrGluGluIleArgGlyIleAlaLysGlyAlaGluArgAspVal
                                    90                             100
         SerGluIleValMetLeuAsnThrArgThrGluPheAlaTyrGlyLeuLysAlaAlaArg
                                   110                             120
         AspGlyCysThrThrAlaTyrCysGlnLeuProAsnGlyAlaLeuGlnGlyGlnAsnTrp
                                   130                             140
         AspPhePheSerAlaThrLysGluAsnLeuIleArgLeuThrIleArgGlnAlaGlyLeu
                                   150                             160
         ProThrIleLysPheIleThrGluAlaGlyIleIleGlyLysValGlyPheAsnSerAla
                                   170                             180
         GlyValAlaValAsnTyrAsnAlaLeuHisLeuGlnGlyLeuArgProThrGlyValPro
                                   190                             200
         SerHisIleAlaLeuArgIleAlaLeuGluSerThrSerProSerGlnAlaTyrAspArg
                                   210                             220
         IleValGluGlnGlyGlyMetAlaAlaSerAlaPheIleMetValGlyAsnGlyHisGlu
                                   230                             240
         AlaPheGlyLeuGluPheSerProThrSerIleArgLysGlnValLeuAspAlaAsnGly
                                   250                             260
         ArgMetValHisThrAsnHisCysLeuLeuGlnHisGlyLysAsnGluLysGluLeuAsp
                                   270                             280
         ProLeuProAspSerTrpAsnArgHisGlnArgMetGluPheLeuLeuAspGlyPheAsp
                                   290                             300
         GlyThrLysGlnAlaPheAlaGlnLeuTrpAlaAspGluAspAsnTyrProPheSerIle

                                   310                             320
         CysArgAlaTyrGluGluGlyLysSerArgGlyAlaThrLeuPheAsnIleIleTyrAsp
                                   330                             340
         HisAlaArgArgGluAlaThrValArgLeuGlyArgProThrAsnProAspGluMetPhe
                                   350
         ValMetArgPheAspGluGluAspGluArgSerAlaLeuAsnAlaArgLeu.
```

2. Eine DNA-Sequenz gemäss Anspruch 1, die folgende Sequenz umfasst:

```
        10        20        30        40        50        60        70
CTTGATCTCCCATCAGTGTCATCCTATCGTCCCAGATTCGTCGCTACCGCAATATAAATCTCACCATGCA
        80        90       100       110       120       130       140
CTTCCCCCTCCATGATCATCCCCACGACCCCCGTTTGTCATCTCCGTCAGCCCACGTCTCAGTTGTTTACCC
       150       160       170       180       190       200       210
ATCTTCCGACCCCCACCAGAAATGCTTCACATCCTCTCTCAACGCCACTCCCTTTGAAGTAAGTGCTGCAC
       220       230       240       250       260       270       280
TGAATACCAGATTTTTTTCCTTCTGAATCTTCCCGAGTTCTGACCTGATCCACATCGGCTACGAACATCGCT
       290       300       310       320       330       340       350
CTGCTCCCAAAGCCGTGATAGCCAGAAGCATTGACTTCCCCGTCGATCTCATCCGACGGAAAACGAACAA
       360       370       380       390       400       410       420
GACGGACGAAGACGCCTTAAACACGGTACTCTCCCAACTGCGGCCCCGTGATCGACGAAACATCGCCCCAAATAC
       430       440       450       460       470       480       490
TACGACCACATTCGCGGTGAGTCCCACTTCGGTCTTTCCTACATTTTCTGCACCAATGCTGACCGATGAC
       500       510       520       530       540       550       560
CCCCCAAAAACCACGTATTCCAAAGCGCCCCTGAACGCCGATGTCTCCCAGATTGTCATCCTTAATACCCCC
       570       580       590       600       610       620       630
ACGGAATTTGCATACCGGCTCAAGCCAGCCCGTGATCGCCTGCACCACTGCCTATTGTCAACTTCCAAATG
       640       650       660       670       680       690       700
GACCCCTCGAGCGCCCAAAACTGGGATGTACGTTAAGAGATTTTTACCTCCTCATTTTATTCCATCGAATTT
       710       720       730       740       750       760       770
CCCCCGACTAATTTCGTTGTTCAAGTTCTTTTCTCCCACCAAAGACAACCTGATCCCGGTTAACGATCCGT
       780       790       800       810       820       830       840
CACGCCCGACTTCCCACCATCAAATTCATAACCGAGCGCTCGAATCATCGGGAACGGTTCGGATTTAACAGTG
       850       860       870       880       890       900       910
CCGCCCGGTCGCCCGTCAATTACAACGCCCTTCACCTTCAACGTCTTCCACCCACCCGAGTTCCTTCCCATAT
       920       930       940       950       960       970       980
TCCCCTCCGCATAGCCGCTCGAAACCACTTCTCCTTCCCACGCCCTATGACCCGATCGTCGACCAACGCCGA
       990      1000      1010      1020      1030      1040      1050
ATCCCCCGCCAGCCGCTTTTTATCATCGTCGCGCAATCGGCCACCGAGCGCATTTCGTTTGGAATTCTCCCCCCACCA
      1060      1070      1080      1090      1100      1110      1120
GCCATCCGAAAGCCAGGTGCTCGACGCCGAAT.CTACCGATCGTGCCACACCAACCACTGCTTGCCTTCAGCACCG
      1130      1140      1150      1160      1170      1180      1190
CAAAAATGAGAAAGACCTCGATCCCTTACCCGACTCATCGGAATCCCCACCACCGTATCGAGTTCCTCCTC
      1200      1210      1220      1230      1240      1250      1260
GACCGGTTCGACGGCACCAAACACGGCATTTGCCCCAGCTCTCGGCCCGACGAAGACAATTATCCCTTTACCA
      1270      1280      1290      1300      1310      1320      1330
TCTGCCGCCCTTACGACGGAGGGCAAGAGCCAGAGCGCCCGACTCTGTTCAATATCATCTACGACCATGCCCCG
      1340      1350      1360      1370      1380      1390      1400
TAGAGACCCAACGGTGCCGGCTTCGCCCGGCCCGACCAACCCTGATCGAGATGTTTGTCATCCCGGTTTGACGAG
      1410      1420      1430      1440      1450      1460      1470
GACGGACCAGAGGTCTGCCCTCAACCCCAGCGCTTTGAAGGCTCTTCATGACGGACGCCCAATCGCATCTTTTGTA
      1480      1490      1500      1510      1520      1530      1540
TGTACCTTCAACCGACTCCGTCTTCACTTCTTCGCCCCGCACTGCCTACCGTTTGTACCATCTGACTCATA
      1550      1560      1570      1580      1590      1600      1610
TAAATGTCTAGCCCCTACCTACACTATACCTAAGGGACACAACCGTACAGTGCATTAACGTACGGCCCCTAT
      1620      1630      1640      1650      1660      1670      1680
AGTACCCCGATCTCTAGATAGAACATTTAGTAGACATTACGATCGCCTAACTAATTTAACTTGACCATTCT
      1690      1700      1710      1720      1730      1740      1750
CCCCTTCATATTGATTTTCATCCATTATACACTCTTAATCGTTTCCCCGGTAGAACGCCGATATATATACCAC
```

45

```
            1760       1770       1780       1790       1800       1810       1820
CATACCCTGTCGACAACAGCCGCTTCCCGTCTGCTTGCCCCTACTTAACCTATATATTCTACACGCCCAAT
            1830       1840       1850       1860       1870       1880       1890
ACTCAATGTCCCCTTAGCCACCTAACCCCCACTCTACCGTAAGTCCCGCGTCATATACGTCAGAAGTCTTAA
            1900       1910       1920       1930       1940       1950       1960
GACTCAAGACACCATATCACCCGTTACCCTCCACCGTACCTACTACCTTCAATATCACTCTTTCACGATG
            1970
CACACGGTCGAC.
```

3. Eine DNA-Sequenz gemäss Anspruch 1 oder 2, welche die für die Transkription und Translation des Penicillium chrysogenum PAT Gens notwendigen Sequenzen umfasst.

4. Eine DNA-Sequenz gemäss Anspruch 3, worin die Sequenzen, die für die Transkription und Translation des Penicillium chrysogenum PAT Gens notwendig sind, die Sequenzen zwischen dem Isopenicillin N synthetase Gen und dem Penicillium chrysogenum PAT Gen sind.

5. Ein Protein, für welches die gemäss einem der Ansprüche 1 bis 4 beanspruchte DNA-Sequenz kodiert, in gereinigter und isolierter Form.

6. Vektor, der eine DNA-Sequenz, die für das Enzym Penicillinacyltransferase (PAT) kodiert, enthält, worin das Enzym Penicillinacyltransferase die folgende Sequenz aufweist:

```
                            10                                    20
          MetLeuHisIleLeuCysGlnGlyThrProPheGluIleGlyTyrGluHisGlySerAla
                            30                                    40
          AlaLysAlaValIleAlaArgSerIleAspPheAlaValAspLeuIleArgGlyLysThr
                            50                                    60
          LysLysThrAspGluGluLeuLysGlnValLeuSerGlnLeuGlyArgValIleGluGlu
                            70                                    80
          ArgTrpProLysTyrTyrGluGluIleArgGlyIleAlaLysGlyAlaGluArgAspVal
                            90                                   100
          SerGluIleValMetLeuAsnThrArgThrGluPheAlaTyrGlyLeuLysAlaAlaArg
                           110                                   120
          AspGlyCysThrThrAlaTyrCysGlnLeuProAsnGlyAlaLeuGlnGlyGlnAsnTrp
                           130                                   140
          AspPhePheSerAlaThrLysGluAsnLeuIleArgLeuThrIleArgGlnAlaGlyLeu
                           150                                   160
          ProThrIleLysPheIleThrGluAlaGlyIleIleGlyLysValGlyPheAsnSerAla
                           170                                   180
          GlyValAlaValAsnTyrAsnAlaLeuHisLeuGlnGlyLeuArgProThrGlyValPro
```

```
                                  190                              200
         SerHisIleAlaLeuArgIleAlaLeuGluSerThrSerProSerGlnAlaTyrAspArg
                                  210                              220
         IleValGluGlnGlyGlyMetAlaAlaSerAlaPheIleMetValGlyAsnGlyHisGlu
                                  230                              240
         AlaPheGlyLeuGluPheSerProThrSerIleArgLysGlnValLeuAspAlaAsnGly
                                  250                              260
         ArgMetValHisThrAsnHisCysLeuLeuGlnHisGlyLysAsnGluLysGluLeuAsp
                                  270                              280
         ProLeuProAspSerTrpAsnArgHisGlnArgMetGluPheLeuLeuAspGlyPheAsp
                                  290                              300
         GlyThrLysGlnAlaPheAlaGlnLeuTrpAlaAspGluAspAsnTyrProPheSerIle

                                  310                              320
         CysArgAlaTyrGluGluGlyLysSerArgGlyAlaThrLeuPheAsnIleIleTyrAsp
                                  330                              340
         HisAlaArgArgGluAlaThrValArgLeuGlyArgProThrAsnProAspGluMetPhe
                                  350
         ValMetArgPheAspGluGluAspGluArgSerAlaLeuAsnAlaArgLeu.
```

7. Vektor gemäss Anspruch 6, worin die DNA-Sequenz, die für das Enzym Penicillinacyltransferase (PAT) kodiert, die folgende Sequenz umfasst:

```
       10       20       30       40       50       60       70
CTTGATGTCCCATCAGTCTCATGCTATCGTCCCAGATTGGTCGCTACGGCAATATAAATCTCACCATGCA
       80       90      100      110      120      130      140
CTTCCCCCTGCATGATCATCCCCACGACGCCCGTTTGTCATCTCCGTCAGCCACGTCTCAGTTGTTTACCC
      150      160      170      180      190      200      210
ATCTTCCGACCCCCACCACGAAATGCTTCACATCCTCTGTCAAGCCACTCCCTTTGAAGTAAGTCCTGCAC
      220      230      240      250      260      270      280
TGAATACCAGATTTTTTTCCTTCTGAATCTTCCGAGTTCTGACCTGATCCAGATCGGCTACGAACATCGCT
      290      300      310      320      330      340      350
CTCCTCCCAAAGCCGTGATAGCCCAGAAGCCATTGACTTCGCCCGTCGATCTCATCCGACGGAAAACGAAGAA
      360      370      380      390      400      410      420
GACGGACGAAGAGCTTAAACACGTACTCTCGCAACTGGGCCCGCCGTGATCGACGAAAGATCGGCCCAAATAC
      430      440      450      460      470      480      490
TACGACGACATTCGCCGGTCAGTCGCCACTTCGGTCTTTCCTACATTTTCTGCACCAATGCTGACCGATGAC
      500      510      520      530      540      550      560
CCCCCGAAAAACCAGGTATTGCAAAGGGCGCCTGAACGCGCATGTCTCGCAGATTGTCATGCTTAATACCCCC
      570      580      590      600      610      620      630
ACGCAATTTCCATACGGGCTCAACGCCACCCCGTCATGGCTCCACCACTGCCTATTGTCAACTTCCAAATG
      640      650      660      670      680      690      700
GACCCCTCCAGCGCCCAAAACTCGGATCTACGTTAACGACATTTTACCTCCTCATTTTATTCCATCGAATTT
      710      720      730      740      750      760      770
GCCCCCACTAATTTCGTTGTTCAAGTTCTTTTTCTCCCACCAAACAGAACCTCATCCCCGTTAACGATCCGT
      780      790      800      810      820      830      840
CAGCCCCGACTTCCCACCATCAAAATTCATAACCGACGCTCGAATCATCCCGAACGTTCGATTTAACACTG
```

47

```
          850       860       870       880       890       900       910
CCGCGGTCCCCGTCAATTACAACGCCCTTCACCTTCAACGTCTTCGACCCACCGGAGTTCCTTCCCATAT
          920       930       940       950       960       970       980
TGCCCTCCGCATAGCCCTCGAAAGCACTTCTCCTTCCCACGCCTATGACCGGATCGTGGACCAACGCCGA
          990      1000      1010      1020      1030      1040      1050
ATGCCCGCCAGCGCCTTTTATCATGGTCGGGCAATGCGGCACGACGCATTTCGTTTGGAATTCTCCCCCACCA
         1060      1070      1080      1090      1100      1110      1120
GGATCCGAAAGCAGGTGCTCGACGCCAAT.GTAGGATCGTGGACACCAACCACTGCTTGCTTCACGACCGG
         1130      1140      1150      1160      1170      1180      1190
CAAAAATGAGAAAGACCTCGATCCCTTACCCGACTCATGGAATCGGCACCAGCGTATGGAGTTCCTGCTC
         1200      1210      1220      1230      1240      1250      1260
GACCCGTTCGACGGCACCAAACACGGCATTTGCCCAGCTCTGGGGCCGACGAACACAATTATGCCTTTAGGA
         1270      1280      1290      1300      1310      1320      1330
TGTGCCGGCCCTTACGACGAGGGCAAGACGAGAGCGGGGACTGTGTTCAATATCATGTAGGACCATGGCGGG
         1340      1350      1360      1370      1380      1390      1400
TAGACACGGAAGCGGTGGCGGGGCTTGGGCCCGCCCACCAACCGGTGATGAGGATGTTTGTGATGGGGGGTTTGACGAG
         1410      1420      1430      1440      1450      1460      1470
CACGACGAGAGGGTGTGGGGGGCTGTGAACGGGGCACGGGCTTTGAAGGGCTGTGTGTTGATGACGAGGGGAATGGATGTGCTTTTGTA
         1480      1490      1500      1510      1520      1530      1540
TGTAGGCTTGCAACCGACTGCGGTGCTTGACTTTTCTTGCGCGCGGCACTGGCCTACCGGTTTGTGTACCATGTGACTGCATA
         1550      1560      1570      1580      1590      1600      1610
TAAATGGTGCTAGGCCCCTAGCCTACACTATAGCGCTAAGGGGACAGGAAGCCGTAGAGTGCATTAACGTACCGCGCCTAT
         1620      1630      1640      1650      1660      1670      1680
AGTAGCCCCGATCTCTAGATAGAACATTTAGTAGAGCATTACGGATGCCCTAACTAATTTAACTTGACGCATTCT
         1690      1700      1710      1720      1730      1740      1750
CCCCTTGCATATTGATTTTGCATCCATTATAGCACTCTTAATGCCTTTGCCCGGGTAGAACCCGGNATATATAGGGAC
         1760      1770      1780      1790      1800      1810      1820
CATAGGCTGTGGAGAACAGGGCGCTTCCCGGTGCTGCTTGCCCGGTAGCTTAAGGCTATATATTGCTACACGCGCCAAT
         1830      1840      1850      1860      1870      1880      1890
ACTCAATGTGCCCCTTAGCGACCTAAGCGCCCCACTGCTAGCGGTAAGTGCCGGGCTGCATATAGCGTGAGAAGTGCTTAA
         1900      1910      1920      1930      1940      1950      1960
GACTCAAGACACGGATATGCACCGCGTTAGCCCTGCACCGGTAGCCTAGCTACCTTGCAATATGCACTGCTTTGCAGGATG
         1970
GACACGGTGGAG .
```

8. Vektor gemäss Anspruch 6 oder 7, der weitere Gene enthält, die für die Transkription und Translation des Penicillium chrysogenum PAT Gens notwendig sind.

9. Vektor gemäss einem der Ansprüche 6 bis 8, der weitere Gene enthält, die eine Selektion des Plasmides in β-lactamproduzierenden Mikroorganismen erlauben.

10. Vektor gemäss einem der Ansprüche 6 bis 9, der ein weiteres Gen enthält, das für das Enzym Isopenicillin N Synthetase kodiert.

11. Verfahren zur Herstellung des Enzyms Penicillinacylasetransferase (PAT), bestehend aus der Transformierung eines Wirtes mit Hilfe eines Vektoren gemäss einem der Ansprüche 6 bis 10 und der Isolierung der exprimierten PAT.

12. Zellen, enthaltend einen Vektor gemäss einem der Ansprüche 6 bis 10.

13. Verfahren zur Verbesserung der Herstellung eines Penicillins, dadurch gekennzeichnet, dass man eine Zelle, die Penicillin herstellt, mit einem Vektor gemäss einem der Ansprüche 6 bis 10 transformieren lässt, und das Penicillin isoliert.

**EP 0 336 446 B1**

**Patentansprüche für folgende Vertragsstaaten : ES, GR**

1. Verfahren zur Herstellung einer DNA-Sequenz, die für das Enzym Penicillinacyltransferase (PAT) kodiert, in gereinigter und isolierter Form, dadurch gekennzeichnet, dass man die DNA-Sequenz, die für das Enzym Penicillinacyltransferase, das die folgende Sequenz aufweist

```
                                              10                                    20
         MetLeuHisIleLeuCysGlnGlyThrProPheGluIleGlyTyrGluHisGlySerAla
                                              30                                    40
         AlaLysAlaValIleAlaArgSerIleAspPheAlaValAspLeuIleArgGlyLysThr
                                              50                                    60
         LysLysThrAspGluGluLeuLysGlnValLeuSerGlnLeuGlyArgValIleGluGlu
                                              70                                    80
         ArgTrpProLysTyrTyrGluGluIleArgGlyIleAlaLysGlyAlaGluArgAspVal
                                              90                                   100
         SerGluIleValMetLeuAsnThrArgThrGluPheAlaTyrGlyLeuLysAlaAlaArg
                                             110                                   120
         AspGlyCysThrThrAlaTyrCysGlnLeuProAsnGlyAlaLeuGlnGlyGlnAsnTrp
                                             130                                   140
         AspPhePheSerAlaThrLysGluAsnLeuIleArgLeuThrIleArgGlnAlaGlyLeu
                                             150                                   160
         ProThrIleLysPheIleThrGluAlaGlyIleIleGlyLysValGlyPheAsnSerAla
                                             170                                   180
         GlyValAlaValAsnTyrAsnAlaLeuHisLeuGlnGlyLeuArgProThrGlyValPro
                                             190                                   200
         SerHisIleAlaLeuArgIleAlaLeuGluSerThrSerProSerGlnAlaTyrAspArg
                                             210                                   220
         IleValGluGlnGlyGlyMetAlaAlaSerAlaPheIleMetValGlyAsnGlyHisGlu
                                             230                                   240
         AlaPheGlyLeuGluPheSerProThrSerIleArgLysGlnValLeuAspAlaAsnGly
                                             250                                   260
         ArgMetValHisThrAsnHisCysLeuLeuGlnHisGlyLysAsnGluLysGluLeuAsp
                                             270                                   280
         ProLeuProAspSerTrpAsnArgHisGlnArgMetGluPheLeuLeuAspGlyPheAsp
                                             290                                   300
         GlyThrLysGlnAlaPheAlaGlnLeuTrpAlaAspGluAspAsnTyrProPheSerIle

                                             310                                   320
         CysArgAlaTyrGluGluGlyLysSerArgGlyAlaThrLeuPheAsnIleIleTyrAsp
                                             330                                   340
         HisAlaArgArgGluAlaThrValArgLeuGlyArgProThrAsnProAspGluMetPhe
                                             350
         ValMetArgPheAspGluGluAspGluArgSerAlaLeuAsnAlaArgLeu ,
```

durch rekombinante Methoden isolieren lässt und die isolierte DNA-Sequenz reinigen lässt.

2. Verfahren gemäss Anspruch 1, worin DNA-Sequenz die folgende Sequenz umfasst:

49

```
         10        20        30        40        50        60        70
GTTCATCTCCCATCAGTCTCATCCTATCGTCCCAGATTCGTCGCTACCGCAATATAAATCTCACCATCCA
         80        90       100       110       120       130       140
CTTCCCCCTCCATCATCATCCCCAGGACCCCCGTTTGTCATCTCCGTCAGCCACGTCTCAGTTGTTTACCC
        150       160       170       180       190       200       210
ATCTTCCGACCCCCAGCAGAAATCCTTCACATCCTCTGTCAACGCACTCCCTTTCAAGTAAGTCCTCCAC
        220       230       240       250       260       270       280
TCAATACCACGATTTTTTTCCTTCTCAATCTTCCGAGTTCTCACCTGATCCACATCGGCTACGAACATCCCT
        290       300       310       320       330       340       350
CTCCTCCCAAAGCCCGTGATAGCCACAAGCCATTCACTTCCCCCGTCCATCTCATCCGACGGCAAAACGAACAA
        360       370       380       390       400       410       420
CACGGACGAAGACCTTAAACAGGTACTCTCCCAACTCCCCCGCCGTGATCCACGAAAGATCCCCCAAATAC
        430       440       450       460       470       480       490
TACCACGACATTCCCCGTCAGTCCCACTTCCGTCTTTCCTACATTTTCTGCACCAATCCTCACCGATCAC
        500       510       520       530       540       550       560
CCCCCAAAAACCACCTATTCCAAACGCCCCTCAACCCCATCTCTCCCAGATTGTCATCCTTAATACCCCC
        570       580       590       600       610       620       630
ACGCAATTTCCATACCGCCCTCAAGCCAGCCCCGTCATCGCTCCACCACTCCCTATTGTCAACTTCCAAATG
        640       650       660       670       680       690       700
CAGCCCTCCAGCCCCAAAACTCCGATGTACGTTAAGACATTTTACCTCCTCATTTTATTCCATCCAATTT
        710       720       730       740       750       760       770
CCCCCCGACTAATTTGGTTCTTCAAGTTCTTTTCTCCCACCAAAGAGAACCTCATCCCGTTAACCATCCGT
        780       790       800       810       820       830       840
CAGCCCGGACTTCCCACCATCAAATTCATAACCGACGCTCGAATCATCCCGAACGTTCCATTTAACAGTG
        850       860       870       880       890       900       910
CGCCCGTCCCCCGTCAATTACAACGCCCCTTCACCTTCAACGTCTTCGACCCACCCGAGTTCCTTCCCATAT
        920       930       940       950       960       970       980
TGCCCTCCGCATAGCCCTCGAAAGCACTTCTCCTTCCCAGCCCTATGACCCGATCGTCGAGCAACGCCCGA
        990      1000      1010      1020      1030      1040      1050
ATCCCCGCCAGCGCCTTTTATCATGGTCGGGCAATCGGCACGAGCCATTTCGTTTGGAATTCTCCCCCACCA
        1060      1070      1080      1090      1100      1110      1120
CCATCCGAAAGCAGGTCCTCCACCCGAAT .CTACCATCCTCCACACCAACCACTCCTTGCTTCACCACCG
        1130      1140      1150      1160      1170      1180      1190
CAAAAATCACAAAGACCTCGATCCCCTTACCCCGACTCATCGAATCCCCACCACCGTATCGAGTTCCTCCTC
        1200      1210      1220      1230      1240      1250      1260
CACCCCGTTCGACGCCACCAAACAGCCATTTCGCCCACCTCTCGCCCCGACGAAGACAATTATCCCTTTACCA
        1270      1280      1290      1300      1310      1320      1330
TCTGCCGCCCTTACGACGACCGCCAACAGCCAGACCCGCCACTCTCTGTTCAATATCATCTACCACCATCCCCC
        1340      1350      1360      1370      1380      1390      1400
TAGACACCCAACCGTCCCGCCTTCCCCCGCCCACCAACCCTCATCACATCTTTCTCATCCCGTTTCACCAC
        1410      1420      1430      1440      1450      1460      1470
CACCACCAGACGTCTCCCCCTCAACCCCACCCTTTCAACCCTCTTCATCACCACCCAATCCATCTTTTTGTA
        1480      1490      1500      1510      1520      1530      1540
TCTACCTTCAACCGACTCCGTCTTCACTTCTTCCCCCCGCACTCCCTACCCGTTTGTACCATCTCACTCATA
        1550      1560      1570      1580      1590      1600      1610
TAAATCTCTACCCCCTACCTACACTATACCTAACGGAGACAACCGTACAGTCATTAACGTACCCCCCTAT
```

50

```
        1620      1630      1640      1650      1660      1670      1680
AGTACCCCGATCTCTAGATAGAACATTTAGTAGAGATTACCATCCCTAACTAATTTAACTTGACCATTGT
        1690      1700      1710      1720      1730      1740      1750
CCCGTTCATATTGATTTTCATCCATTATACACTCTTAATCGTTTCCCCGTAGAACCCGNATATATACGAC
        1760      1770      1780      1790      1800      1810      1820
CATAGCGTGTGGAGAACAGCCCTTCCCGTCTGCTTGCCCGTACTTAACCTATATATTCTACACCGCCAAT
        1830    · 1840      1850      1860      1870      1880      1890
ACTCAATGTGCCCTTAGCACCTAACCGCCACTCTACCGTAAGTGCCCGTCATATACGTCAGAAGTCTTAA
        1900      1910      1920      1930      1940      1950      1960
GACTGAAGACAGGATATCACCGCGTTACCCTGCACCGTACCTACTACCTTCAATATCACTCTTTCAGGATG
        1970
GACAGCGTCGAC.
```

3. Verfahren gemäss Anspruch 1 oder 2, worin die DNA-Sequenz die für die Transkription und Translation des Penicillium chrysogenum PAT Gens notwendigen Sequenzen umfasst.

4. Verfahren gemäss Anspruch 3, worin die Sequenzen, die für die Transkription und Translation des Penicillium chrysogenum PAT Gens notwendig sind, die Sequenzen zwischen dem Isopenicillin N synthetase Gen und dem Penicillium chrysogenum PAT Gen sind.

5. Verfahren zur Herstellung eines Vektors, der eine DNA-Sequenz, die für das Enzym Penicillinacyltransferase (PAT) kodiert, enthält, dadurch gekennzeichnet, dass die DNA-Sequenz, die für das Enzym Penicillinacyltransferase kodiert, die folgende Sequenz aufweist:

```
                              10                              20
        MetLeuHisIleLeuCysGlnGlyThrProPheGluIleGlyTyrGluHisGlySerAla
                              30                              40
        AlaLysAlaValIleAlaArgSerIleAspPheAlaValAspLeuIleArgGlyLysThr
                              50                              60
        LysLysThrAspGluGluLeuLysGlnValLeuSerGlnLeuGlyArgValIleGluGlu
                              70                              80
        ArgTrpProLysTyrTyrGluGluIleArgGlyIleAlaLysGlyAlaGluArgAspVal
                              90                              100
        SerGluIleValMetLeuAsnThrArgThrGluPheAlaTyrGlyLeuLysAlaAlaArg
                              110                             120
        AspGlyCysThrThrAlaTyrCysGlnLeuProAsnGlyAlaLeuGlnGlyGlnAsnTrp
                              130                             140
        AspPhePheSerAlaThrLysGluAsnLeuIleArgLeuThrIleArgGlnAlaGlyLeu
                              150                             160
        ProThrIleLysPheIleThrGluAlaGlyIleIleGlyLysValGlyPheAsnSerAla
                              170                             180
        GlyValAlaValAsnTyrAsnAlaLeuHisLeuGlnGlyLeuArgProThrGlyValPro
                              190                             200
        SerHisIleAlaLeuArgIleAlaLeuGluSerThrSerProSerGlnAlaTyrAspArg
```

51

EP 0 336 446 B1

                                    210                                    220
IleValGluGlnGlyGlyMetAlaAlaSerAlaPheIleMetValGlyAsnGlyHisGlu
                                    230                                    240
AlaPheGlyLeuGluPheSerProThrSerIleArgLysGlnValLeuAspAlaAsnGly
                                    250                                    260
ArgMetValHisThrAsnHisCysLeuLeuGlnHisGlyLysAsnGluLysGluLeuAsp
                                    270                                    280
ProLeuProAspSerTrpAsnArgHisGlnArgMetGluPheLeuLeuAspGlyPheAsp
                                    290                                    300
GlyThrLysGlnAlaPheAlaGlnLeuTrpAlaAspGluAspAsnTyrProPheSerIle

                                    310                                    320
CysArgAlaTyrGluGluGlyLysSerArgGlyAlaThrLeuPheAsnIleIleTyrAsp
                                    330                                    340
HisAlaArgArgGluAlaThrValArgLeuGlyArgProThrAsnProAspGluMetPhe
                                    350
ValMetArgPheAspGluGluAspGluArgSerAlaLeuAsnAlaArgLeu,

durch rekombinante Methoden mit einem Vektor verbinden lässt.

**6.** Vektor gemäss Anspruch 5, worin die DNA-Sequenz, die für das Enzym Penicillinacyltransferase (PAT) kodiert, die folgende Sequenz umfasst:

```
        10        20        30        40        50        60        70
GTTGATGTCCCATCAGTGTCATGCTATCGTCCCAGATTGGTCGCTACCGCAATATAAATCTCACCATGCA
        80        90       100       110       120       130       140
GTTCCCCCTGCATGATCATCCCCAGGACGCCGTTTGTCATCTCCGTCAGCCACGTCTCAGTTGTTTACCC
       150       160       170       180       190       200       210
ATCTTCCGACCCCGCAGCAGAAATGCTTCACATCCTCTGTCAACGCACTCCCTTTGAAGTAAGTGCTGCAC
       220       230       240       250       260       270       280
TGAATACCAGATTTTTTTCCTTCTGAATCTTCCGAGTTCTGACCTGATCCAGATCGGCTACGAACATCGCT
       290       300       310       320       330       340       350
CTGCTCCCAAAGCCCGTGATACCCAGAAGCATTGACTTCGCCCGTCGATCTCATCCGAGCGAAAACGAACAA
       360       370       380       390       400       410       420
GACGGACCAACAGCCTTAAACACGGTACTCTCCCAACTGGCGCCCCGTGATCGACGAAAGATCGGCCCAAATAC
       430       440       450       460       470       480       490
TACGACGACATTCGCCGGCTCAGTGCCCACTTCGGTCTTTCCTACATTTTCTGGCACCAATGGTGACCGATGAC
       500       510       520       530       540       550       560
CCCGGAAAAACCAGGTATTGCAAAGGGCGCCTGAACGGCCATGTGTCTCCGAGATTGTCATGGTTAATACCCGC
       570       580       590       600       610       620       630
ACGGAATTTGCATACCGGCTCAAGGCCAGGCCCGTGATGGCTGCACCACTGCCTATTGTCAACTTCCAAATG
       640       650       660       670       680       690       700
CAGCCCTGCAGCGGCCCAAAAACTGGGATGTACGTTAAGAGATTTTACCTGCTCATTTTATTGCATCCAATTT
       710       720       730       740       750       760       770
CGCGCCGACTAATTTTGGTTGTTGTTCAAGTTTCTTTTCTGCCCACCAAACACAACCTGATCGCGGTTAACGATGCGT
       780       790       800       810       820       830       840
CAGGCCCCGACTTCCCCACCATCAAAATTGCATAACCGGAGCCTCGAATCATCGCGGAACGTTCGGATTTAACACTG
```

52

EP 0 336 446 B1

```
        850        860        870        880        890        900        910
CCCCCGTCGCCCGTCAATTACAACGCCCTTCACCTTCAAGGTCTTCGACCCACCCGAGTTCCTTCGCATAT
        920        930        940        950        960        970        980
TGCCCTCCCCATACCGGCTCCAAACCACTTCTCCTTCCCACCCCTATGACCCGATCGTCCAGCAACGCCCGA
        990       1000       1010       1020       1030       1040       1050
ATGCCCCGCACCCCTTTTATCATGGTCGGCAATCGGCACGACGCATTTCGTTTGGAATTCTCCCCCACCA
       1060       1070       1080       1090       1100       1110       1120
CCATCCCAAACCACGTGCTCCACCCCGAAT·.GTACGATCGTCCACACCAACCACTGCTTGCTTCACCACCG
       1130       1140       1150       1160       1170       1180       1190
CAAAAATGACAAAGACCTCCATCCCTTACCCGACTCATCGAATCCCCACCAGCCGTATCGAGTTCCTCCTC
       1200       1210       1220       1230       1240       1250       1260
CACCCGTTCCACCCCACCAAACACGCATTTCCCCAGCTCTCGCGCCGACCAAGACAATTATCCCTTTACCA
       1270       1280       1290       1300       1310       1320       1330
TCTCCCGCCCTTACGACGACGGCAAGAGCACAGCGCGCGACTCTGTTCAATATCATCTACGACCATCCCCG
       1340       1350       1360       1370       1380       1390       1400
TAGACACCCAACCGTGCCCCCTTGCCCCGGCCGACCAACCCTGATGAGATGTTTGTCATCCCCGTTTCACCAG

       1410       1420       1430       1440       1450       1460       1470
CACGACGACACGTCTGCCCTCAACCCCACCCCTTTGAAGCCTCTTCATGACGACCCAATCCATCTTTTGTA
       1480       1490       1500       1510       1520       1530       1540
TGTACCTTCAACCGACTCCGTCTTCACTTCTTCCCCCGCACTGCCTACCGTTTGTACCATCTGACTCATA
       1550       1560       1570       1580       1590       1600       1610
TAAATGTCTAGCCCCTACCTACACTATACCTAACGGACAGAACCGTACAGTGATTAACGTACGCCCCTAT
       1620       1630       1640       1650       1660       1670       1680
AGTACCCCGATCTCTAGATAGAACATTTAGTACACATTACGATCCCTAACTAATTTAACTTGACCATTGT
       1690       1700       1710       1720       1730       1740       1750
CCCGTTCATATTCATTTTCATCCATTATACACTCTTAATCGTTTCCCCGTACAACCCCGATATATACCAC
       1760       1770       1780       1790       1800       1810       1820
CATACCGTCTCGAGAACAGCCCTTCCCCGTCTCCTTCGCCCGTACTTAACCTATATATTCTACACCGCCAAT
       1830        1840       1850       1860       1870       1880       1890
ACTCAATCTCCCCTTACCACCTAACCCGCACTCTACCCTAAGTCCCGCGTCATATACCTCAGAAGTCTTAA
       1900       1910       1920       1930       1940       1950       1960
CACTCAAGACACGATATCACCCCGTTACCCTCCACCGTACCTACTACCTTCAATATCACTCTTTCACGATC
       1970
CACAGCCTCCAC·
```

7. Verfahren gemäss Anspruch 5 oder 6, worin der Vektor weitere Gene enthält, die für die Transkription und Translation des Penicillium chrysogenum PAT Gens notwendig sind.

8. Verfahren gemäss einem der Ansprüche 5 bis 7 worin der Vektor weitere Gene enthält, die eine Selektion des Plasmides in $\beta$-lactamproduzierenden Mikroorganismen erlauben.

9. Verfahren gemäss einem der Ansprüche 5 bis 8, worin der Vektor ein weiteres Gen enthält, das für das Enzym Isopenicillin N Synthetase kodiert.

10. Verfahren zur Herstellung des Enzyms Penicillinacylasetransferase (PAT), bestehend aus der Transformierung eines Wirtes mit Hilfe eines Vektoren wie definiert in einem der Ansprüche 6 bis 9 und der Isolierung der exprimierten PAT.

11. Verfahren zur Herstellung von Zellen, die einen Vektor wie definiert in einem der Ansprüche 5 bis 9 enthalten, dadurch gekennzeichnet, dass man die Zellen mit einem Vektor wie definiert in einem der Ansprüche 5 bis 9 transformieren lässt.

53

12. Verfahren zur Verbesserung der Herstellung eines Penicillins, dadurch gekennzeichnet dass man eine Zelle, die Penicillin herstellt, mit einem Vektor wie definiert in einem der Ansprüche 5 bis 9 transformieren lässt, und das Penicillin isoliert.

**Claims**

**Claims for the following Contracting States : AT, BE, CH, DE, FR, GB, IT, LI, LU, NL, SE**

1.  A DNA sequence in purified and isolated form that codes for the enzyme penicillin acyl transferase which has the following sequence:

```
                                10                              20
        MetLeuHisIleLeuCysGlnGlyThrProPheGluIleGlyTyrGluHisGlySerAla
                                30                              40
        AlaLysAlaValIleAlaArgSerIleAspPheAlaValAspLeuIleArgGlyLysThr
                                50                              60
        LysLysThrAspGluGluLeuLysGlnValLeuSerGlnLeuGlyArgValIleGluGlu
                                70                              80
        ArgTrpProLysTyrTyrGluGluIleArgGlyIleAlaLysGlyAlaGluArgAspVal
                                90                              100
        SerGluIleValMetLeuAsnThrArgThrGluPheAlaTyrGlyLeuLysAlaAlaArg
                                110                             120
        AspGlyCysThrThrAlaTyrCysGlnLeuProAsnGlyAlaLeuGlnGlyGlnAsnTrp
                                130                             140
        AspPhePheSerAlaThrLysGluAsnLeuIleArgLeuThrIleArgGlnAlaGlyLeu
                                150                             160
        ProThrIleLysPheIleThrGluAlaGlyIleIleGlyLysValGlyPheAsnSerAla
                                170                             180
        GlyValAlaValAsnTyrAsnAlaLeuHisLeuGlnGlyLeuArgProThrGlyValPro
                                190                             200
        SerHisIleAlaLeuArgIleAlaLeuGluSerThrSerProSerGlnAlaTyrAspArg
                                210                             220
        IleValGluGlnGlyGlyMetAlaAlaSerAlaPheIleMetValGlyAsnGlyHisGlu
                                230                             240
        AlaPheGlyLeuGluPheSerProThrSerIleArgLysGlnValLeuAspAlaAsnGly
                                250                             260
        ArgMetValHisThrAsnHisCysLeuLeuGlnHisGlyLysAsnGluLysGluLeuAsp
                                270                             280
        ProLeuProAspSerTrpAsnArgHisGlnArgMetGluPheLeuLeuAspGlyPheAsp
                                290                             300
        GlyThrLysGlnAlaPheAlaGlnLeuTrpAlaAspGluAspAsnTyrProPheSerIle

                                310                             320
        CysArgAlaTyrGluGluGlyLysSerArgGlyAlaThrLeuPheAsnIleIleTyrAsp
                                330                             340
        HisAlaArgArgGluAlaThrValArgLeuGlyArgProThrAsnProAspGluMetPhe
                                350
        ValMetArgPheAspGluGluAspGluArgSerAlaLeuAsnAlaArgLeu.
```

2.  A DNA sequence according to claim 1 which includes the following sequence:

```
        10        20        30        40        50        60        70
CTTCATCTCCCATCAGTGTCATCCTATCGTCCCAGATTCGTCCCTACCGCAATATAAATCTCACCATGCA
        80        90       100       110       120       130       140
GTTCCCCCTCCATGATCATCCCCAGGACGCCCGTTTGTCATCTCCGTCACCCACGTCTCAGTTGTTTACCC
       150       160       170       180       190       200       210
ATCTTCCGACCCCCAGCAGAAATGCTTCACATCCTCTGTCAACGCACTCCCTTTGAAGTAAGTGCTGCAC
       220       230       240       250       260       270       280
TGAATACCAGATTTTTTCCTTCTGAATCTTCCCAGTTCTGACCTGATCCAGATCGCCTACCAACATCCCT
       290       300       310       320       330       340       350
CTCCTCCCAAACCCGTCATAGCCACAAGCATTGACTTCGCCGTCGATCTCATCCCAGCGCAAAACGAAGAA
       360       370       380       390       400       410       420
CACCGACCAAGCACCTTAAACAGGTACTCTCCCAACTCGCGCCCCGTGATCCACGAAAGATCGCCCCAAATAC
       430       440       450       460       470       480       490
TACGACCACATTCGCCGTGACTCCCACTTCGGTCTTTCCTACATTTTCTCCACCAATGCTCACCGATGAC
       500       510       520       530       540       550       560
CCCCCAAAAACCACGTATTCCAAACGCCCCTGAACGCGATGTCTCCCAGATTGTCATCCTTAATACCCCC
       570       580       590       600       610       620       630
ACGCAATTTCCATACCCGCTCAACGCCACCCCGTGATCGCCTGCACCACTGCCTATTGTCAACTTCCAAATG
       640       650       660       670       680       690       700
GACCCCTCCAGCGCCCAAAACTGGGATGTACGTTAAGACATTTTACCTCCTCATTTTATTCCATCCAATTT
       710       720       730       740       750       760       770
GCCCCGACTAATTTCGTTGTTCAAGTTCTTTTCTGCCCACCAAAGAGAACCTGATCCCCGTTAACGATCCGT
       780       790       800       810       820       830       840
CACCCCCGACTTCCCACCATCAAATTCATAACCGACGCTGGAATCATCGGGAACGTTGGATTTAACACTG
       850       860       870       880       890       900       910
CCCCCCTCCCCCGTCAATTACAACGCCCTTCACCTTCAACGTCTTCGACCCACCCGAGTTCCTTCGCATAT
       920       930       940       950       960       970       980
TGCCCTCCGCATAGCCGCTCCAAACCACTTCTCCTTCCCAGCCCTATCACCGGATCGTCGAGCAAGCCCGA
       990      1000      1010      1020      1030      1040      1050
ATCCCCGCCACCGCTTTTATCATCGTGCGCCCAATCCCCACCACGCCATTTCGTTTGGAATTCTCCCCCACCA
      1060      1070      1080      1090      1100      1110      1120
GCCATCCCAAACCACGTGCTCCACCCCGAAT.GTACCATCGTCCACACCAACCACTGCTTGCTTCACCACCG
      1130      1140      1150      1160      1170      1180      1190
CAAAAATCAGAAAGACCTCGATCCCTTACCCGACTCATCCAATCCCCACCACCGTATCGACTTCCTCCTC
      1200      1210      1220      1230      1240      1250      1260
GACCCGTTCGACGCCACCAAACAGGCATTTCCCCAGCTCTCGCGCCCGACGAAGACAATTATCCCTTTACCA
      1270      1280      1290      1300      1310      1320      1330
TCTCCCGCCCTTACGACGAGCGCAAGAGCAGAGCGCCGACTCTGTTCAATATCATCTACGACCATCCCCG
      1340      1350      1360      1370      1380      1390      1400
TACACACCCAACGGTCCGGCTTGCCCGGCCCACCAACCCTGATGACATCTTTGTCATCCCGTTTCACGAG
      1410      1420      1430      1440      1450      1460      1470
CACCACCGAGACCTCTCCCCTCAACCCCAGCCTTTCAAGCCTCTTCATGACGACCCAATCCATCTTTTCTA
      1480      1490      1500      1510      1520      1530      1540
TGTACCTTCAACCGACTCCGTCTTCACTTCTTCGCCCCCCACTCCCTACCGTTTGTACCATCTGACTCATA
      1550      1560      1570      1580      1590      1600      1610
TAAATCTCTAGCCCCCTACCTACACTATACCTAAGGGACAGAACCGTACAGTCATTAACCTACCGGCCCTAT
```

```
       1620      1630      1640      1650      1660      1670      1680
AGTACCCCGATCTCTAGATAGAACATTTAGTAGAGATTACGATCCCTAACTAATTTAACTTGACCATTGT
       1690      1700      1710      1720      1730      1740      1750
CCCCTTCATATTGATTTTCATCCATTATACACTCTTAATCGTTTCCCCGGTAGAACCCCGATATATACGAC
       1760      1770      1780      1790      1800      1810      1820
CATAGGGTGTGGAGAACAGCGCTTCCCGTCTGCTTGGCCCGTACTTAACCTATATATTCTACACGGCCCAAT
       1830      1840      1850      1860      1870      1880      1890
ACTCAATGTGCCCCTTAGCACCTAACCGCCACTCTACCGGTAAGTGCCGCGTCATATACGTGAGAAGTCTTAA
       1900      1910      1920      1930      1940      1950      1960
GACTGAAGACACGATATCACGCCGTTACCCTGGACCGTACCTACTACCTTCAATATCACTCTTTCACGATG
       1970
GACAGCGTGGAG.
```

3. A DNA sequence according to claim 1 or 2 which includes the sequences necessary for the transcription and translation of the Penicillium chrysogenum PAT gene.

4. A DNA sequence according to claim 3 in which the sequences which are necessary for the transcription and translation of the Penicillium chrysogenum PAT gene are the sequences between the Isopenicillin N-synthetase gene and the Pencillium chrysogenum PAT gene,

5. A protein which is coded for by a DNA sequence according to any one of claims 1 to 4, in purified an isolated form.

6. A vector which contains a DNA sequence that codes for the enzyme penicillin acyltransferase (PAT); the enzyme penicillin acyltransferase having the following sequence:

```
                                 10                                       20
        MetLeuHisIleLeuCysGlnGlyThrProPheGluIleGlyTyrGluHisGlySerAla
                                 30                                       40
        AlaLysAlaValIleAlaArgSerIleAspPheAlaValAspLeuIleArgGlyLysThr
                                 50                                       60
        LysLysThrAspGluGluLeuLysGlnValLeuSerGlnLeuGlyArgValIleGluGlu
                                 70                                       80
        ArgTrpProLysTyrTyrGluGluIleArgGlyIleAlaLysGlyAlaGluArgAspVal
                                 90                                      100
        SerGluIleValMetLeuAsnThrArgThrGluPheAlaTyrGlyLeuLysAlaAlaArg
                                110                                      120
        AspGlyCysThrThrAlaTyrCysGlnLeuProAsnGlyAlaLeuGlnGlyGlnAsnTrp
                                130                                      140
        AspPhePheSerAlaThrLysGluAsnLeuIleArgLeuThrIleArgGlnAlaGlyLeu
```

```
                         150                                          160
   ProThrIleLysPheIleThrGluAlaGlyIleIleGlyLysValGlyPheAsnSerAla
                         170                                          180
   GlyValAlaValAsnTyrAsnAlaLeuHisLeuGlnGlyLeuArgProThrGlyValPro
                         190                                          200
   SerHisIleAlaLeuArgIleAlaLeuGluSerThrSerProSerGlnAlaTyrAspArg
                         210                                          220
   IleValGluGlnGlyGlyMetAlaAlaSerAlaPheIleMetValGlyAsnGlyHisGlu
                         230                                          240
   AlaPheGlyLeuGluPheSerProThrSerIleArgLysGlnValLeuAspAlaAsnGly
                         250                                          260
   ArgMetValHisThrAsnHisCysLeuLeuGlnHisGlyLysAsnGluLysGluLeuAsp
                         270                                          280
   ProLeuProAspSerTrpAsnArgHisGlnArgMetGluPheLeuLeuAspGlyPheAsp
                         290                                          300
   GlyThrLysGlnAlaPheAlaGlnLeuTrpAlaAspGluAspAsnTyrProPheSerIle


                         310                                          320
   CysArgAlaTyrGluGluGlyLysSerArgGlyAlaThrLeuPheAsnIleIleTyrAsp
                         330                                          340
   HisAlaArgArgGluAlaThrValArgLeuGlyArgProThrAsnProAspGluMetPhe
                         350
   ValMetArgPheAspGluGluAspGluArgSerAlaLeuAsnAlaArgLeu.
```

7. A vector according to claim 6 in which the DNA sequence that codes for the enzyme penicillin acyltransferase (PAT), includes the following sequence:

```
         10        20        30        40        50        60        70
   GTTGATGTCCCATCACTCTCATCCTATCCTCCCAGATTCGTCGCCTACCGCAATATAAATCTCACCATGCA
         80        90       100       110       120       130       140
   CTTCCCCCTCCATGATCATCCCCACGACCGCCGTTTGTCATCTCCGTCACCCACGTCTCAGTTGTTTACCC
        150       160       170       180       190       200       210
   ATCTTCCGACCCCCACCAGAAATGCTTCACATCCTCTGTCAAGCCACTCCCTTTGAAGTAAGTGCTGCAC
        220       230       240       250       260       270       280
   TGAATACCAGATTTTTTTCCTTCTGAATCTTCCGAGTTCTCACCTGATCCAGATCGCCTACCAACATGCCT
        290       300       310       320       330       340       350
   CTCCTGCCAAAGCCCGTGATAGCCACAAGCATTGACTTCGCCGTCGATCTCATCCGAGCGAAAACGAAGAA
        360       370       380       390       400       410       420
   CACGGCACGAACAGCTTAAACACGTACTCTCCCAACTGCCGCCCCCCGATCCACGAAAGATCCCCCAAATAC
        430       440       450       460       470       480       490
   TACCACGACATTCGCCCGTGAGTGCCCACTTCGGTGTCTTTCCTACATTTTCTCCACCAATGCTGACCGATCAC
```

```
       500        510        520        530        540        550        560
CCCCCAAAAACCACCTATTCCAAACCCCCCTCAACCCCATCTCTCCCAGATTCTCATCCTTAATACCCCC
       570        580        590        600        610        620        630
ACCCAATTTCCATACCCCCTCAACCCACCCCCTCATCCCTCCACCACTCCCTATTCTCAACTTCCAAATC
       640        650        660        670        680        690        700
CACCCCTCCACCCCCAAAACTCCGATCTACCTTAAGAGATTTTACCTCCTCATTTTATTCCATCCAATTT
       710        720        730        740        750        760        770
CCCCCCACTAATTTCGTTCTTCAAGTTCTTTTCTCCCACCAAAGACAAGCTCATCCCCCTTAACCATCCCT
       780        790        800        810        820        830        840
CACCCCCGACTTCCCACCATCAAATTCATAACCCACCCTCCAATCATCCCGAACCTTCCATTTAACACTC
       850        860        870        880        890        900        910
CCCCCCTCCCCCTCAATTACAACCCCCTTCACCTTCAACCTCTTCCACCCACCCCAGTTCCTTCCCATAT
       920        930        940        950        960        970        980
TCCCCTCCCCATACCCCTCCAAACCACTTCTCCTTCCCACCCCTATCACCCCATCCTCCACCAACCCCCA
       990       1000       1010       1020       1030       1040       1050
ATCCCCCCCAGCCCCTTTTATCATCCTCCCCCAATCCCCACCACCCATTTCCTTTCCAATTCTCCCCCCACCA
      1060       1070       1080       1090       1100       1110       1120
CCATCCCAAACCACCTCCTCCACCCCCAAT·.CTACCATCCTCCACACCAACCACTCCTTCCTTCACCACCC
      1130       1140       1150       1160       1170       1180       1190
CAAAAATCACAAAGACCTCCATCCCTTACCCCACTCATCCAATCCCCACCACCCTATCCACTTCCTCCTC
      1200       1210       1220       1230       1240       1250       1260
CACCCCTTCCACCCCACCCAAACACCCATTTCCCCACCTCTCCCCCCACCAACACAATTATCCCTTTACCCA
      1270       1280       1290       1300       1310       1320       1330
TCTCCCCCCCTTACCACCACCCCCAAGACCAGACCCCCCACTCTCTTCAATATCATCTACCACCATCCCCCC
      1340       1350       1360       1370       1380       1390       1400
TACACACCCAACCCTCCCCCCTTCCCCCCCCCCACCAACCCTCATCACATCTTTCTCATCCCCTTTCACCAC
      1410       1420       1430       1440       1450       1460       1470
CACCACCACACCTCTCCCCTCAACCCCACCCTTTCAAGCCTCTTCATCACCACCCAATCCATCTTTTCTA
      1480       1490       1500       1510       1520       1530       1540
TCTACCTTCAACCCACTCCCTCTTCACTTCTTCCCCCCGCACTCCCTACCCGTTTCTACCATCTCACTCATA
      1550       1560       1570       1580       1590       1600       1610
TAAATCTCTACCCCCTACCTACACTATACCTAACCCACACAACCCTACACTCATTAACCTACCCCCCTAT
      1620       1630       1640       1650       1660       1670       1680
ACTACCCCCATCTCTACATACAACATTTAGTACACATTACCATCCCTAACTAATTTAACTTCACCATTCT
      1690       1700       1710       1720       1730       1740       1750
CCCCCTTCATATTCATTTTCATCCATTATACACTCTTAATCCTTTCCCCCGTACAACCCCCNATATATACCAC
      1760       1770       1780       1790       1800       1810       1820
CATACCCTCTCCACAACACCCCTTCCCCCTCTCCTTCCCCCTACTTAACCTATATATTCTACACCCCCAAT
      1830       1840       1850       1860       1870       1880       1890
ACTCAATCTCCCCTTACCACCTAACCCCCACTCTACCCTAACTCCCCCGTCATATACCTCACAACTCTTAA
      1900       1910       1920       1930       1940       1950       1960
CACTCAAGACACCATATCACCCCGTTACCCTCCACCCTACCTACTACCTTCAATATCACTCTTTCACCATC
      1970
CACACCCTCCAC ●
```

**8.** A vector according to claim 6 or claim 7 which contains further genes that are necessary for the transcription and translation of the Penicillium chrysogenum PAT gene.

**9.** A Vector according to any one of claims 6 to 8 which contains further genes that permit the selection of the plasmid in $\beta$-lactam producing microorganisms.

10. A vector according to any one of claims 6 to 9 which contains a further gene that codes for the enzyme Isopenicillin N synthetase.

11. A process for the production of the enzyme penicillin acyltransferase (PAT), comprising transforming a host with a vector according to any one of claims 6 to 10 and isolating the expressed PAT.

12. Cells which contain a vector according to any one of claims 6 to 10.

13. A process for improving the production of penicillin characterized in that a penicillin producing cell is transformed with a vector according to any one of claims 6 to 10, and the penicillin is isolated.

**Claims for the following Contracting States : ES, GR**

1. A process for the production of a DNA sequence that codes for the enzyme penicillin acyltransferase (PAT), in purified and isolated form, characterized in that a DNA sequence which codes for the enzyme penicillin acyltransferase having the following sequence:

```
                         10                            20
     MetLeuHisIleLeuCysGlnGlyThrProPheGluIleGlyTyrGluHisGlySerAla
                         30                            40
     AlaLysAlaValIleAlaArgSerIleAspPheAlaValAspLeuIleArgGlyLysThr
                         50                            60
     LysLysThrAspGluGluLeuLysGlnValLeuSerGlnLeuGlyArgValIleGluGlu
                         70                            80
     ArgTrpProLysTyrTyrGluGluIleArgGlyIleAlaLysGlyAlaGluArgAspVal
                         90                           100
     SerGluIleValMetLeuAsnThrArgThrGluPheAlaTyrGlyLeuLysAlaAlaArg
                        110                           120
     AspGlyCysThrThrAlaTyrCysGlnLeuProAsnGlyAlaLeuGlnGlyGlnAsnTrp
                        130                           140
     AspPhePheSerAlaThrLysGluAsnLeuIleArgLeuThrIleArgGlnAlaGlyLeu
                        150                           160
     ProThrIleLysPheIleThrGluAlaGlyIleIleGlyLysValGlyPheAsnSerAla
                        170                           180
     GlyValAlaValAsnTyrAsnAlaLeuHisLeuGlnGlyLeuArgProThrGlyValPro
                        190                           200
     SerHisIleAlaLeuArgIleAlaLeuGluSerThrSerProSerGlnAlaTyrAspArg
                        210                           220
     IleValGluGlnGlyGlyMetAlaAlaSerAlaPheIleMetValGlyAsnGlyHisGlu
                        230                           240
     AlaPheGlyLeuGluPheSerProThrSerIleArgLysGlnValLeuAspAlaAsnGly
                        250                           260
     ArgMetValHisThrAsnHisCysLeuLeuGlnHisGlyLysAsnGluLysGluLeuAsp
                        270                           280
     ProLeuProAspSerTrpAsnArgHisGlnArgMetGluPheLeuLeuAspGlyPheAsp
                        290                           300
     GlyThrLysGlnAlaPheAlaGlnLeuTrpAlaAspGluAspAsnTyrProPheSerIle

                        310                           320
     CysArgAlaTyrGluGluGlyLysSerArgGlyAlaThrLeuPheAsnIleIleTyrAsp
                        330                           340
     HisAlaArgArgGluAlaThrValArgLeuGlyArgProThrAsnProAspGluMetPhe
                        350
     ValMetArgPheAspGluGluAspGluArgSerAlaLeuAsnAlaArgLeu ,
```

is isolated using recombinant methods and the isolated DNA sequence is purified.

2. A process according to claim 1 in which the DNA sequence includes the following sequence:

```
          10        20        30        40        50        60        70
CTTCATCTCCCATCACTCTCATCCTATCCTCCCAGATTCGTCGCTACCCCAATATAAATCTCACCATGCA
          80        90       100       110       120       130       140
CTTCCCCCTCCATCATCATCCCCAGCACCCCGTTTGTCATCTCCGTCAGCCACGTCTCAGTTGTTTACCC
         150       160       170       180       190       200       210
ATCTTCCGACCCCGCAGCAGAAATCCTTCACATCCTCTGTCAACGCACTCCCTTTGAAGTAAGTCCTCCAC
         220       230       240       250       260       270       280
TGAATACCACATTTTTTCCTTCTGAATCTTCCGAGTTCTGACCTGATCCACATCGCCTACGAACATCCCT
         290       300       310       320       330       340       350
CTCCTCCCAAAGCCCGTCATAGCCCAGAAGCCATTGACTTCCCCGTCCATCTCATCCGACGGCAAAACGAAGAA
         360       370       380       390       400       410       420
GACGGACCAAGCAGCCTTAAACACGGTACTCTCCCAACTGCCCCCCGTGATCCACGAAAGATCCCCCAAATAC
         430       440       450       460       470       480       490
TACCAGCACATTCCCCGTCAGTCCCACTTCCGTCTTTCCTACATTTTCTCCACCAATCCTCACCGATCAC
         500       510       520       530       540       550       560
CCCCCAAAAACCACCTATTCCAAACCCCCCTCAACCCCATCTCTCCGAGATTGTCATCCTTAATACCCCC
         570       580       590       600       610       620       630
ACGCAATTTCCATACCCCCTCAAGCCAGCCCGTGATCGCCTCCACCACTCCCTATTGTCAACTTCCAAATG
         640       650       660       670       680       690       700
GACCCCTCCAGCCCCAAAACTCCGATGTACCTTAAGACATTTTACCTCCTCATTTTATTCCATCCAATTT
         710       720       730       740       750       760       770
CCCCCCACTAATTTCGTTCTTCAAGTTCTTTTCTCCCACCAAAGACAACCTCATCCCCGTTAACCATCCGT
         780       790       800       810       820       830       840
CACCCCCGACTTCCCACCATCAAATTCATAACCGACGCCTCCAATCATCCCGAACGTTCCGATTTAACAGTG
         850       860       870       880       890       900       910
CCCCCGTCGCCCGTCAATTACAACCCCCTTCACCTTCAACGTCTTCGACCCACCCGAGTTCCTTCCCATAT
         920       930       940       950       960       970       980
TCCCCTCCCCATACCGCTCCAAACCACTTCTCCTTCCCACCCCTATGACCGGATCCTCCAGCAACCCCCA
         990      1000      1010      1020      1030      1040      1050
ATCCCCGCCAGCCCTTTTATCATCGTCGCCAATCGCCACGACCCATTTCGTTTGCAATTCTCCCCCCACCA
        1060      1070      1080      1090      1100      1110      1120
CCATCCGAAACCACGTGCTCCACCCCAAT.GTAGGATCGTCCACACCAACCACTCCTTGCTTCAGCACCG
        1130      1140      1150      1160      1170      1180      1190
CAAAAATCAGAAAGACCTCGATCCCTTACCCGACTCATCGAATCGCCACCACCGTATCGACTTCCTCCTC
        1200      1210      1220      1230      1240      1250      1260
GACCCGTTCCACCCCACCAAACACGCATTTCCCCAGCCTCTCGCCCCGACGAAGACAATTATCCCTTTACCA
        1270      1280      1290      1300      1310      1320      1330
TCTCCCGCCCCTTACGAGGACGCGCAAGAGCAGAGCGCCCCACTCTGTTCAATATCATCTACGACCATCGCCCC
        1340      1350      1360      1370      1380      1390      1400
TACAGACCCCAACCGTCCCGCCTTCGCCCGCCCGACCAACCCTCATCACATCTTTGTCATCCCGTTTGACGAG
```

```
            1410      1420      1430      1440      1450      1460      1470
CACCACGACGACGTCTGCCCTCAACGCCACGCCTTTGAAGCCTCTTCATGACGACCCAATCCATCTTTTCTA
            1480      1490      1500      1510      1520      1530      1540
TCTACCTTCAACCGACTCCGTCTTCACTTCTTCGCCCCGCACTCCCTACCGTTTGTACCATCTGACTCATA
            1550      1560      1570      1580      1590      1600      1610
TAAATGTCTACCCCCTACCTACACTATACCTAACGGACGAGAACCGTACAGTCATTAACGTACCCGCCTAT

            1620      1630      1640      1650      1660      1670      1680
AGTACCCCGATCTCTAGATACAACATTTAGTAGACGATTACGATCCCTAACTAATTTAACTTGACCATTGT
            1690      1700      1710      1720      1730      1740      1750
CCCCTTCATATTCGATTTTCATCCATTATACACTCTTAATCGTTTCCCCGTACAACCCCGNATATATACGAC
            1760      1770      1780      1790      1800      1810      1820
CATAGCGTGTGGAGAACACGGCTTCCCGTCTGCTTGGCCCGTACTTAACCTATATATTCTACACCGCCAAT
            1830      1840      1850      1860      1870      1880      1890
ACTCAATGTCCCCTTACCACCTAACCGCCACTCTACGGTAAGTGCGCGTGATATACGTGACAAGTCTTAA
            1900      1910      1920      1930      1940      1950      1960
GACTGAAGACAGGATATCACGCCGTTACCCTCCACCGTACCTACTACCTTCAATATCACTCTTTCACGATG
            1970
CACACCGTCCAC.
```

3.  A process according to claim 1 or claim 2 in which the DNA-sequence includes the sequences necessary for the transcription and translation of the Penicillium chrysogenum PAT gene.

4.  A process according to claim 3 in which the sequences that are necessary for the transcription and translation of the Penicillium chrysogenum PAT gene are the sequences between the Isopenicillium N synthetase gene and the Penicillium chrysogenum PAT gene.

5.  A process for the production of a vector that contains a DNA sequence that codes for the enzyme penicillin acyltransferase (PAT), characterized in that the DNA sequence that codes for the enzyme penicillin acyltransferase having the following sequence:

```
                                          10                                    20
              MetLeuHisIleLeuCysGlnGlyThrProPheGluIleGlyTyrGluHisGlySerAla
                                          30                                    40
              AlaLysAlaValIleAlaArgSerIleAspPheAlaValAspLeuIleArgGlyLysThr
                                          50                                    60
              LysLysThrAspGluGluLeuLysGlnValLeuSerGlnLeuGlyArgValIleGluGlu
                                          70                                    80
              ArgTrpProLysTyrTyrGluGluIleArgGlyIleAlaLysGlyAlaGluArgAspVal
                                          90                                   100
              SerGluIleValMetLeuAsnThrArgThrGluPheAlaTyrGlyLeuLysAlaAlaArg
                                         110                                   120
              AspGlyCysThrThrAlaTyrCysGlnLeuProAsnGlyAlaLeuGlnGlyGlnAsnTrp
                                         130                                   140
              AspPhePheSerAlaThrLysGluAsnLeuIleArgLeuThrIleArgGlnAlaGlyLeu

                                         150                                   160
              ProThrIleLysPheIleThrGluAlaGlyIleIleGlyLysValGlyPheAsnSerAla
                                         170                                   180
              GlyValAlaValAsnTyrAsnAlaLeuHisLeuGlnGlyLeuArgProThrGlyValPro
                                         190                                   200
              SerHisIleAlaLeuArgIleAlaLeuGluSerThrSerProSerGlnAlaTyrAspArg

                                         210                                   220
              IleValGluGlnGlyGlyMetAlaAlaSerAlaPheIleMetValGlyAsnGlyHisGlu
                                         230                                   240
              AlaPheGlyLeuGluPheSerProThrSerIleArgLysGlnValLeuAspAlaAsnGly
                                         250                                   260
              ArgMetValHisThrAsnHisCysLeuLeuGlnHisGlyLysAsnGluLysGluLeuAsp
                                         270                                   280
              ProLeuProAspSerTrpAsnArgHisGlnArgMetGluPheLeuLeuAspGlyPheAsp
                                         290                                   300
              GlyThrLysGlnAlaPheAlaGlnLeuTrpAlaAspGluAspAsnTyrProPheSerIle

                                         310                                   320
              CysArgAlaTyrGluGluGlyLysSerArgGlyAlaThrLeuPheAsnIleIleTyrAsp
                                         330                                   340
              HisAlaArgArgGluAlaThrValArgLeuGlyArgProThrAsnProAspGluMetPhe
                                         350
              ValMetArgPheAspGluGluAspGluArgSerAlaLeuAsnAlaArgLeu,
```

is combined with a vector by recombinant methods.

6. A vector according to claim 5 in which the DNA sequence that codes for the enzyme penicillin acyltransferase (PAT) includes the following sequence:

```
          10        20        30        40        50        60        70
GTTGATGTCCCATCAGTGTCATGCTATGGTCCCAGATTGGTGCCTACCGCAATATAAATCTCACCATGCCA
          80        90       100       110       120       130       140
GTTCCCCCTGCATGATCATCCCCAGGACGCCCGTTTGTCATCTCCGTCAGCCACGTGTCTCAGTTGTTTACCC
         150       160       170       180       190       200       210
ATCTTCCCACCCCCAGCAGAAATGCTTCACATGCCTGTGTCAACGCACTCCCTTTGAAGTAAGTGCCTGCCAC
         220       230       240       250       260       270       280
TGAATACCAGATTTTTTTCCTTCTGAATGTTCCGAGTTGTGACCTGATGCCAGATGGCGCTACGAACATGGCCT
         290       300       310       320       330       340       350
GTGCCTGCCCAAAGCCCGTGATAGCCCAGAAGCATTGACTTCGCCGGTGCATGTGATGCCGAGGCGAAAACGAAGAA
         360       370       380       390       400       410       420
GACGGACCAAGAGCCTTAAACACGGTACTGTGTCCCCAACTGGGGCCCCGTGATGGGAGCCAAAGATGCCGCCCCCAAATAC
         430       440       450       460       470       480       490
TACGAGGGACATTCCGGGCTCAGTGCCCACTTCCGTGTGTTTCCTACATTTTGTCTCCACCAATGGCTGCACCCGATGAC
         500       510       520       530       540       550       560
CCCCCGAAAAACCAGGTATTGCCAAAGGGCCGGCTGAACGGCCATGTGTGTCTCCCAGATTGTGTCATGGCCTTAATACCCCC
         570       580       590       600       610       620       630
ACCCAATTTGCCATACCGGGCTCAAGGCCAGGCCCCGTGATGGCCCTGCCACCACTGGCCCTATTGCTCAACTTGCCAAATG
         640       650       660       670       680       690       700
GAGCCCCTCCAGCGCCCCAAAAACTGCGGATGTGATGCGTTAAGAGAGCATTTTTACCTCCTCATTTTTATTGCCATGCCAATTT
         710       720       730       740       750       760       770
CCGGCCCACTAATTTGCGTTGTTGTTCAAGTTGTCTTTTTGCTGCCCACCAAAGAGAAGCCTGATGCCCCGTTAACCATGCCCGT
         780       790       800       810       820       830       840
CACGGCCCCGACTTGCCCACCATGCAAATTGCATAACCGACGGCTGCCAATCATGCGCGAACGTTGCCATTTAACAGTG
         850       860       870       880       890       900       910
CCGGCCGGTGCCCCGTGCAATTACAACGGCCCCTTGCACCTTGCAACGTGCTTGCGACCCACCCGAGTTGCCTTGCCCATAT
         920       930       940       950       960       970       980
TGCCCCTGCCCATACCGGCTGGAAAGCCACTTGCTGCCTTGCCCAGGCCCCTATGACCGGGATGCGTGGGAGCAACGGCCGGA
         990      1000      1010      1020      1030      1040      1050
ATGCCCCGCCCACCGCCTTTTTATCATGGTGCGGGCCAATGGGGCCACGGACGGCATTTGGTTTGGCAATTGCTGCCCCCCCACCA
        1060      1070      1080      1090      1100      1110      1120
CCATGCCCCAAAGGCCAGGTGCTGCCACGGCCCAAT.GTAGGATGGTGCCACACCAACCCACTGCCTTGCCTTGCACCACGGG
        1130      1140      1150      1160      1170      1180      1190
CAAAAAATGCAGAAAGAGCCTGCCATGCCCTTACCCCCGACTGCATGGCAAATGCCCCACCACCGGTATGGGAGTTGCCTGCCTGC
        1200      1210      1220      1230      1240      1250      1260
GACCGCGTTGCCACGCCCACCAAAACACGGCATTTGCCCCACGCTGCTGCCGGCCCGACGAAGACAAATTATGCCCTTTACCA
        1270      1280      1290      1300      1310      1320      1330
TGCTGCCGCCGCCTTACGACGGAGGGCAAGAGCGCAGAGCGCCCCGACTGCTGCTTGCAATATGCATGCTACGGACCATGCCCCG
        1340      1350      1360      1370      1380      1390      1400
TACGAGAGCCCAACGCTGCCCCGCTTGCCCCGGCCCACGCAACCCTGCATCGACATGCTTTGCTGCATGCCCGTTTGCACCAG
        1410      1420      1430      1440      1450      1460      1470
CAGCGACGCAGAGGGTGTGTGCGGGCTCAACGCCCACGGCCTTTGAAGGCCTGCTTGCATGACGGCACGCCAATGGCCATGCTTTTTGTA
        1480      1490      1500      1510      1520      1530      1540
TGTACGGCTTGCAACGGGACTGGCCGTGTGTGTTGCACTTGCTTGCGGCCCCGGCACTGGCCCTACCGGTTTGGTACCATGCTGGACTGCATA
        1550      1560      1570      1580      1590      1600      1610
TAAATGTGCTAGGCCCCCTACCTACACTATACCTAAGGGACGAGCAAACCCTAGAGTGCATTAACGGTACGGGCCCTAT
```

```
         1620      1630      1640      1650      1660      1670      1680
AGTACCCCGATCTCTAGATACAACATTTACTAGACATTACGATCCCTAACTAATTTAACTTGACCATTCT
         1690      1700      1710      1720      1730      1740      1750
CCCCTTCATATTCATTTTCATCCATTATACACTCTTAATCGTTTCCCCGTAGAACCCCGNATATATACGAC
         1760      1770      1780      1790      1800      1810      1820
CATACCGTCTCGAGAACAGCCCTTCCCGTCTGCTTGCCCGTACTTAACCTATATATTCTACACGCCCAAT
         1830      1840      1850      1860      1870      1880      1890
ACTCAATCTGCCCCTTACCACCTAACCGGCACTCTACGGTAAGTGCCCGTCATATACGTGACAAGTCTTAA
         1900      1910      1920      1930      1940      1950      1960
GACTGAACACACGATATCACGCCGTTACCCTGCACCGTACCTACTACCTTCAATATCACTCTTTCACGATG
         1970
GACACGGTCGAC.
```

7. A process according to claim 5 or claim 6 in which the vector contains further genes that are necessary for transcription and translation of the Penicillium chrysogenum PAT gene.

8. A process according to any one of claims 5 to 7 in which the vector contains further genes that permit the selection of the plasmid in $\beta$-lactam producing microorganisms.

9. A process according to any one of claims 5 to 8 in which the vector contains a further gene that codes for the enzyme Isopenicillium N synthetase.

10. A process for the production of the enzyme penicillin acyltransferase (PAT) comprising transforming the host with a vector as defined in any one of claims 6 to 9 and isolating the expressed PAT.

11. A process for the production of cells that contain a vector as defined in any one of claims 5 to 9, characterized in that the cells are transformed with a vector as defined in any one of claims 5 to 9.

12. A process for improving the production of penicillin characterized in that a penicillin producing cell is transformed with a vector as defined in any one of claims 5 to 9, and the penicillin is isolated.

**Revendications**
**Revendications pour les Etats contractants suivants : AT, BE, CH, DE, FR, GB, IT, LI, LU, NL, SE**

1. Séquence d'ADN sous forme purifiée et isolée, codant pour l'enzyme pénicillinacyltransférase qui présente la séquence suivante :

```
                                   10                              20
    MetLeuHisIleLeuCysGlnGlyThrProPheGluIleGlyTyrGluHisGlySerAla
                                   30                              40
    AlaLysAlaValIleAlaArgSerIleAspPheAlaValAspLeuIleArgGlyLysThr
                                   50                              60
    LysLysThrAspGluGluLeuLysGlnValLeuSerGlnLeuGlyArgValIleGluGlu
                                   70                              80
    ArgTrpProLysTyrTyrGluGluIleArgGlyIleAlaLysGlyAlaGluArgAspVal
                                   90                             100
    SerGluIleValMetLeuAsnThrArgThrGluPheAlaTyrGlyLeuLysAlaAlaArg
                                  110                             120
    AspGlyCysThrThrAlaTyrCysGlnLeuProAsnGlyAlaLeuGlnGlyGlnAsnTrp
                                  130                             140
    AspPhePheSerAlaThrLysGluAsnLeuIleArgLeuThrIleArgGlnAlaGlyLeu
                                  150                             160
    ProThrIleLysPheIleThrGluAlaGlyIleIleGlyLysValGlyPheAsnSerAla
                                  170                             180
    GlyValAlaValAsnTyrAsnAlaLeuHisLeuGlnGlyLeuArgProThrGlyValPro
                                  190                             200
    SerHisIleAlaLeuArgIleAlaLeuGluSerThrSerProSerGlnAlaTyrAspArg
                                  210                             220
    IleValGluGlnGlyGlyMetAlaAlaSerAlaPheIleMetValGlyAsnGlyHisGlu
                                  230                             240
    AlaPheGlyLeuGluPheSerProThrSerIleArgLysGlnValLeuAspAlaAsnGly
                                  250                             260
    ArgMetValHisThrAsnHisCysLeuLeuGlnHisGlyLysAsnGluLysGluLeuAsp
                                  270                             280
    ProLeuProAspSerTrpAsnArgHisGlnArgMetGluPheLeuLeuAspGlyPheAsp
                                  290                             300
    GlyThrLysGlnAlaPheAlaGlnLeuTrpAlaAspGluAspAsnTyrProPheSerIle

                                  310                             320
    CysArgAlaTyrGluGluGlyLysSerArgGlyAlaThrLeuPheAsnIleIleTyrAsp
                                  330                             340
    HisAlaArgArgGluAlaThrValArgLeuGlyArgProThrAsnProAspGluMetPhe
                                  350
    ValMetArgPheAspGluGluAspGluArgSerAlaLeuAsnAlaArgLeu.
```

2.  Séquence d'ADN selon la revendication 1, comprenant la séquence suivante :

```
          10        20        30        40        50        60        70
CTTCATCTCCCATCAGTGTCATCCTATCCTCCCACATTCCTCCCTACCCCAATATAAATCTCACCATCCA
          80        90       100       110       120       130       140
CTTCCCCCTCCATCATCATCCCCACGACCCCCTTTGTCATCTCCGTCACCCACCTCTCAGTTCTTTACCC
         150       160       170       180       190       200       210
ATCTTCCCACCCCCACCACAAATCCTTCACATCCTCTGTCAACCCACTCCCTTTCAACTAACTCCTCCAC
         220       230       240       250       260       270       280
TCAATACCACATTTTTTTCCTTCTCAATCTTCCCAGTTCTCACCTCATCCACATCCCCTACCAACATCCCT
         290       300       310       320       330       340       350
CTCCTCCCAAACCCGTCATACCCACAACCATTCACTTCCCCGTCGATCTCATCCCACCGAAAACGAACAA
         360       370       380       390       400       410   -   420
CACCCACCAACACCTTAAACACGTACTCTCCCAACTCCCCCCCGTCATCCACCAAAGATCCCCCAAATAC
         430       440       450       460       470       480       490
TACCACCACATTCCCCGTCACTCCCACTTCCGTCTTTCCTACATTTTCTCCACCAATCCTCACCCATCAC
         500       510       520       530       540       550       560
CCCCCAAAAACCACCTATTCCAAACCCCCCTCAACCCGATCTCTCCCACATTGTCATCCTTAATACCCCC
         570       580       590       600       610       620       630
ACCCAATTTCCATACCCCCTCAACCCACCCCGTCATCCCTCCACCACTCCCTATTCTCAACTTCCAAATC
         640       650       660       670       680       690       700
CACCCCTCCAGCCCCAAAACTCCGATGTACCTTAACACATTTTACCTCCTCATTTTATTCCATCCAATTT
         710       720       730       740       750       760       770
CCCCCCACTAATTTCGTTCTTCAAGTTCTTTTCTCCCACCAAAGACAACCTCATCCCCGTTAACCATCCGT
         780       790       800       810       820       830       840
CACCCCCGACTTCCCACCATCAAATTCATAACCCACCCTCCAATCATCCCCAACCTTCCATTTAACAGTG
         850       860       870       880       890       900       910
CCCCCGTCCCCCGTCAATTACAACCCCCTTCACCTTCAACCTCTTCCACCCACCCCAGTTCCTTCCCATAT
         920       930       940       950       960       970       980
TCCCCTCCCCATACCCCTCCAAACCACTTCTCCTTCCCACCCCTATCACCCCATCGTCCACCAACCCCCGA
         990      1000      1010      1020      1030      1040      1050
ATCCCCCCCACCCCTTTTATCATCCTCCCCAATCCCCACCACCCATTTCGTTTCCAATTCTCCCCCCACCA
        1060      1070      1080      1090      1100      1110      1120
CCATCCCAAACCACGTCCTCCACCCCCAAT.CTACCATCCTCCACACCAACCACTCCTTCCTTCACCACCG
        1130      1140      1150      1160      1170      1180      1190
CAAAAATCACAAAGACCTCCATCCCTTACCCCACTCATCCAATCCCCACCACCGTATCCAGTTCCTCCTC
        1200      1210      1220      1230      1240      1250      1260
CACCCGTTCCACCCCACCAAACACCCATTTCCCCACCTCTCCCCCCACCAACACAATTATCCCTTTACCA
        1270      1280      1290      1300      1310      1320      1330
TCTCCCCCCCTTACCACCACCCCAACAGCACACCCCCCACTCTCTTCAATATCATCTACCACCATCCCCG
        1340      1350      1360      1370      1380      1390      1400
TACACACCCAACCCTCCCCGCTTCCCCCCCCCACCAACCCTCATCACATCTTTCTCATCCCGTTTCACGAC

        1410      1420      1430      1440      1450      1460      1470
CACCACCACACCTCTCCCCTCAACCCCCACCCTTTCAACCCTCTTCATCACCACCCAATCCATCTTTTGTA
        1480      1490      1500      1510      1520      1530      1540
TCTACCTTCAACCCACTCCGTCTTCACTTCTTCCCCCCCCACTCCCTACCGTTTGTACCATCTCACTCATA
        1550      1560      1570      1580      1590      1600      1610
TAAATCTCTACCCCCTACCTACACTATACCTAAACGCACACAACCGTACACTCATTAACCTACCCCCCTAT
```

66

```
        1620      1630      1640      1650      1660      1670      1680
AGTACCCCGATCTCTAGATAGAACATTTAGTAGAGATTACCATCCCTAACTAATTTAACTTGACCATTGT
        1690      1700      1710      1720      1730      1740    ·  1750
CCCGTTCATATTCATTTTCATCCATTATACACTCTTAATCGTTTCCCCGTACAACCCCGATATATACCAC
        1760      1770      1780      1790      1800      1810      1820
CATACCGTCTCCAGAACACCCCTTCCCGTCTCCTTCCCCGTACTTAACCTATATATTCTACACCCCCAAT
        1830    ·  1840      1850      1860      1870      1880      1890
ACTCAATCTCCCCTTACCACCTAACCCCCACTCTACCGTAAGTCCGCCGTCATATACGTCACAACTCTTAA
        1900      1910      1920      1930      1940      1950      1960
CACTCAAGACACCATATCACGCCGTTACCCTCCACCGTACCTACTACCTTCAATATCACTCTTTCACCATC
        1970
CACACCGTCCAC.
```

**3.** Séquence d'ADN selon la revendication 1 ou 2, comprenant les séquences requises pour la transcription et la traduction du gène PAT de <u>Penicillium chrysogenum</u>.

**4.** Séquence d'ADN selon la revendication 3, dans laquelle les séquences qui sont requises pour la transcription et la traduction du gène PAT de <u>Penicillium chrysogenum</u> sont les séquences entre le gène de l'isopénicilline N synthétase et le gène PAT de <u>Penicillium</u> <u>chrysogenum</u>.

**5.** Protéine qui est codée par une séquence d'ADN selon l'une quelconque des revendications 1 à 4, sous forme purifiée et isolée.

**6.** Vecteur contenant une séquence d'ADN codant pour l'enzyme pénicillinacyltransférase (PAT), l'enzyme pénicillinacyltransférase présentant la séquence suivante :

```
                              10                                    20
        MetLeuHisIleLeuCysGlnGlyThrProPheGluIleGlyTyrGluHisGlySerAla
                              30                                    40
        AlaLysAlaValIleAlaArgSerIleAspPheAlaValAspLeuIleArgGlyLysThr
                              50                                    60
        LysLysThrAspGluGluLeuLysGlnValLeuSerGlnLeuGlyArgValIleGluGlu
                              70                                    80
        ArgTrpProLysTyrTyrGluGluIleArgGlyIleAlaLysGlyAlaGluArgAspVal
                              90                                    100
        SerGluIleValMetLeuAsnThrArgThrGluPheAlaTyrGlyLeuLysAlaAlaArg
                              110                                   120
        AspGlyCysThrThrAlaTyrCysGlnLeuProAsnGlyAlaLeuGlnGlyGlnAsnTrp
                              130                                   140
        AspPhePheSerAlaThrLysGluAsnLeuIleArgLeuThrIleArgGlnAlaGlyLeu
                              150                                   160
        ProThrIleLysPheIleThrGluAlaGlyIleIleGlyLysValGlyPheAsnSerAla
                              170                                   180
        GlyValAlaValAsnTyrAsnAlaLeuHisLeuGlnGlyLeuArgProThrGlyValPro
```

```
                    190                                    200
SerHisIleAlaLeuArgIleAlaLeuGluSerThrSerProSerGlnAlaTyrAspArg
                    210                                    220
IleValGluGlnGlyGlyMetAlaAlaSerAlaPheIleMetValGlyAsnGlyHisGlu
                    230                                    240
AlaPheGlyLeuGluPheSerProThrSerIleArgLysGlnValLeuAspAlaAsnGly
                    250                                    260
ArgMetValHisThrAsnHisCysLeuLeuGlnHisGlyLysAsnGluLysGluLeuAsp
                    270                                    280
ProLeuProAspSerTrpAsnArgHisGlnArgMetGluPheLeuLeuAspGlyPheAsp
                    290                                    300
GlyThrLysGlnAlaPheAlaGlnLeuTrpAlaAspGluAspAsnTyrProPheSerIle

                    310                                    320
CysArgAlaTyrGluGluGlyLysSerArgGlyAlaThrLeuPheAsnIleIleTyrAsp
                    330                                    340
HisAlaArgArgGluAlaThrValArgLeuGlyArgProThrAsnProAspGluMetPhe
                    350
ValMetArgPheAspGluGluAspGluArgSerAlaLeuAsnAlaArgLeu.
```

7. Vecteur selon la revendication 6, dans lequel la séquence d'ADN, codant pour l'enzyme pénicillinacyltransférase (PAT), comprend la séquence suivante :

```
        10        20        30        40        50        60        70
GTTCATCTCCCATCAGTCTCATCCTATCGTCCCACATTCGTCCCTACCGCAATATAAATCTCACCATCCA
        80        90       100       110       120       130       140
GTTCCCCCTCCATCATCATCCCCACCACCCCGTTTGTCATCTCCGTCACCCACGTCTCAGTTGTTTACCC
       150       160       170       180       190       200       210
ATCTTCCCACCCCCACCACAAATCCTTCACATCCTCTGTCAACCCACTCCCTTTGAAGTAAGTCCTCCAC
       220       230       240       250       260       270       280
TGAATACCAGATTTTTTTCCTTCTGAATCTTCCGAGTTCTCACCTGATCCACATCCCCTACCAACATCCCT
       290       300       310       320       330       340       350
CTCCTCCCAAACCCGTCATAGCCACAACCATTGACTTCCCCGTCCATCTCATCCCACCGAAAACGAAGAA
       360       370       380       390       400       410       420
GACCGACCAAGACCTTAAACACGTACTCTCCCAACTCCCCCCCCGTGATCGACCAAACATCCCCCAAATAC
       430       440       450       460       470       480       490
TACCACCACATTCCCCGTCAGTCCCACTTCCGTCTTTCCTACATTTTCTGCACCAATCCTGACCCATCAC
       500       510       520       530       540       550       560
CCCCCAAAAACCACGTATTCCAAAGCCCCCCTGAACCCCATGTCTCCGAGATTGTCATCCTTAATACCCCC
       570       580       590       600       610       620       630
ACCCAATTTCCATACCCCCTCAACCCACCCCCTCATCCCTCCACCACTCCCTATTGTCAACTTCCAAATC
       640       650       660       670       680       690       700
CACCCCTCCAGCCCCCAAAACTCCGATGTACGTTAAGACATTTTACCTCCTCATTTTATTCCATCCAATTT
       710       720       730       740       750       760       770
CCCCCCACTAATTTCGTTGTTCAAGTTCTTTTTCTCCCACCAAACAGAACCTCATCCCCGTTAACCATCCGT
       780       790       800       810       820       830       840
CACCCCCGACTTCCCCACCATCAAATTCATAACCGACCCTCCAATCATCCCGAACGTTCGATTTAACACTC
```

68

```
      850       860       870       880          890       900       910
CCCCCGTCCCCGTCAATTACAACCCCCTTCACCTTCAACGTCTTCGACCCACCCGAGTTCCTTCCCATAT
      920       930       940       950          960       970       980
TCCCCTCCCCATACCCCTCCAAACCACTTCTCCTTCCCACCCCTATCACCCGATCGTCGACCAACCCCGA
      990      1000      1010      1020         1030      1040      1050
ATCCCCCCCACCCCTTTTATCATCGTCGCCCAATCGGCCACCACCCATTTCGTTTCCAATTCTCCCCCACCA
     1060      1070      1080      1090         1100      1110      1120
CCATCCCAAACCACGTCCTCCACGCCAAT.CTACGATCGTCCACACCAACCACTCCTTCCTTCACCACCG
     1130      1140      1150      1160         1170      1180      1190
CAAAAATCACAAACACCTCCATCCCTTACCGCACTCATCGAATCCCCACCACCGTATCGAGTTCCTCCTC
     1200      1210      1220      1230         1240      1250      1260
CACCCCTTCCACCCCACCAAACACCCATTTCCCCACCTCTCGCCCCGACCAACACAATTATCCCTTTACCA
     1270      1280      1290      1300         1310      1320      1330
TCTCCCCCCCTTACCACCCACGCCCAACAGCAGACGCCCCGACTCTCGTTCAATATCATCTACCACCATCCCCG
     1340      1350      1360      1370         1380      1390      1400
TACACACCCAACCGTCCCGCCTTCGCCCCGCCCACCAACCCTCATCACATCTTTGTCATCCCGTTTCACCAG
     1410      1420      1430      1440         1450      1460      1470
CACCACCACACGTCTCCCGCTCAACCCCCACCCTTTCAACCCTCTTCATCACCACCCAATCCATCTTTTCTA
     1480      1490      1500      1510         1520      1530      1540
TGTACCTTCAACCCACTCCCTCTTCACTTCTTCCCCCCCACTCCCTACCGTTTGTACCATCTCACTCATA
     1550      1560      1570      1580         1590      1600      1610
TAAATCTCTACCCCCTACCTACACTATACCTAACGGACACAACCGTACACTCATTAACCTACCGCCCCTAT
     1620      1630      1640      1650         1660      1670      1680
AGTACCCCGATCTCTACATACAACATTTACTACACACATTACGATCCCTAACTAATTTAACTTCACCATTCT
     1690      1700      1710      1720         1730      1740      1750
CCCGTTCATATTCATTTTCATCCATTATACACTCTTAATCGTTTCCCCGTACAACCCCGATATATACCAC
     1760      1770      1780      1790         1800      1810      1820
CATACCGTGTCGAGAACACCCCTTCCCCGTCTCCTTGCCCCGTACTTAACCTATATATTCTACACCCCCAAT
     1830      1840      1850      1860         1870      1880      1890
ACTCAATGTCCCCTTACCACCTAACCCCCACTCTACCGTAAGTCCCGGTCATATACGTCAGAAGTCTTAA
     1900      1910      1920      1930         1940      1950      1960
CACTCAACACACCATATCACCCCGTTACCCTCCACCCTACCTACTACCTTCAATATCACTCTTTCACCATC
     1970
CACACCCTCCAC .
```

8. Vecteur selon la revendication 6 ou 7, contenant d'autres gènes qui sont requis pour la tanscription et la traduction du gène PAT de Penicillium chrysogenum.

9. Vecteur selon l'une quelconque des revendications 6 à 8, contenant d'autres gènes qui permettent une sélection du plasmide dans des microorganismes producteurs de β-lactames.

10. Vecteur selon l'une quelconque des revendications 6 à 9, contenant un autre gène qui code pour l'enzyme isopénicilline N synthétase.

11. Procédé pour la production de l'enzyne pénicillinacyltransférase (PAT), consistant en la transformation d'un hôte à l'aide d'un vecteur selon l'une quelconque des revendications 6 à 10 et l'isolement de la PAT exprimée.

12. Cellule contenant un vecteur selon l'une quelconque des revendications 6 à 10.

13. Procédé pour l'amélioration de la production d'une pénicilline, caractérisé en ce qu'on transforme une cellule productrice de pénicilline avec un vecteur selon l'une quelconque des revendications 6 à 10, et on isole la pénicilline.

**Revendications pour les Etats contractants suivants : ES, GR**

1. Procédé pour la production d'une séquence d'ADN codant pour l'enzyme pénicillinacyltransférase (PAT), sous forme purifiée et isolée, caractérisé en ce qu'on isole par des méthodes de recombinaison la séquence d'ADN qui code pour l'enzyme pénicillinacyltransférase présentant la séquence suivante :

```
                           10                           20
        MetLeuHisIleLeuCysGlnGlyThrProPheGluIleGlyTyrGluHisGlySerAla
                           30                           40
        AlaLysAlaValIleAlaArgSerIleAspPheAlaValAspLeuIleArgGlyLysThr
                           50                           60
        LysLysThrAspGluGluLeuLysGlnValLeuSerGlnLeuGlyArgValIleGluGlu
                           70                           80
        ArgTrpProLysTyrTyrGluGluIleArgGlyIleAlaLysGlyAlaGluArgAspVal
                           90                           100
        SerGluIleValMetLeuAsnThrArgThrGluPheAlaTyrGlyLeuLysAlaAlaArg
                           110                          120
        AspGlyCysThrThrAlaTyrCysGlnLeuProAsnGlyAlaLeuGlnGlyGlnAsnTrp
                           130                          140
        AspPhePheSerAlaThrLysGluAsnLeuIleArgLeuThrIleArgGlnAlaGlyLeu
                           150                          160
        ProThrIleLysPheIleThrGluAlaGlyIleIleGlyLysValGlyPheAsnSerAla
                           170                          180
        GlyValAlaValAsnTyrAsnAlaLeuHisLeuGlnGlyLeuArgProThrGlyValPro
                           190                          200
        SerHisIleAlaLeuArgIleAlaLeuGluSerThrSerProSerGlnAlaTyrAspArg
                           210                          220
        IleValGluGlnGlyGlyMetAlaAlaSerAlaPheIleMetValGlyAsnGlyHisGlu
                           230                          240
        AlaPheGlyLeuGluPheSerProThrSerIleArgLysGlnValLeuAspAlaAsnGly
                           250                          260
        ArgMetValHisThrAsnHisCysLeuLeuGlnHisGlyLysAsnGluLysGluLeuAsp
                           270                          280
        ProLeuProAspSerTrpAsnArgHisGlnArgMetGluPheLeuLeuAspGlyPheAsp
                           290                          300
        GlyThrLysGlnAlaPheAlaGlnLeuTrpAlaAspGluAspAsnTyrProPheSerIle

                           310                          320
        CysArgAlaTyrGluGluGlyLysSerArgGlyAlaThrLeuPheAsnIleIleTyrAsp
                           330                          340
        HisAlaArgArgGluAlaThrValArgLeuGlyArgProThrAsnProAspGluMetPhe
                           350
        ValMetArgPheAspGluGluAspGluArgSerAlaLeuAsnAlaArgLeu ,
```

et on purifie la séquence d'ADN isolée.

2. Procédé selon la revendication 1, dans lequel la séquence d'ADN comprend la séquence suivante :

```
        10        20        30        40        50        60        70
CTTCATCTCCCATCAGTCTCATCCTATCGTCCCAGATTCGTCCCTACCCCAATATAAATCTCACCATCCA
        80        90       100       110       120       130       140
CTTCCCCCTCCATCATCATCCCCACGACCCCGTTTGTCATCTCCGTCACCCACGTCTCAGTTGTTTACCC
       150       160       170       180       190       200       210
ATCTTCCCGACCCCCAGCAGAAATCCTTCACATCCTCTGTCAACGCACTCCCTTTGAAGTAAGTCCTGCAC
       220       230       240       250       260       270       280
TGAATACCACATTTTTTTCCTTCTCAATCTTCCGAGTTCTCACCTGATCCACATCGCCTACGAACATCCCT
       290       300       310       320       330       340       350
CTCCTCCCAAACCCGTCATACCCACAAGCATTGACTTCGCCCGTCGATCTCATCCGACGGAAAACGAACAA
       360       370       380       390       400       410       420
CACGGACCAACACCTTAAACACGTACTCTCCCAACTCCCCCCCGTCATCGACGAAACATCCCCCAAATAC
       430       440       450       460       470       480       490
TACGACGACATTCCCCGTCAGTCCCACTTCCGTCTTTCCTACATTTTCTCCACCAATCCTCACCGATCAC
       500       510       520       530       540       550       560
CCCCCAAAAACCACGTATTCCAAACGCCCCCTCAACCCGATCTCTCCCAGATTGTCATCCTTAATACCCCG
       570       580       590       600       610       620       630
ACGCAATTTCCATACCCCCTCAACGCCAGCCCCGTCATGCCTCCACCACTCCCTATTGTCAACTTCCAAATG
       640       650       660       670       680       690       700
CACCCCTCCAGCCCCAAAACTCGGATCTACGTTAAGACATTTTACCTCCTCATTTTATTCCATCCAATTT
       710       720       730       740       750       760       770
CCCCCCGACTAATTTCGTTGTTCAAGTTCTTTTTCTCCCACCAAAGACAACCTCATCCCCGTTAACCATCCCT
       780       790       800       810       820       830       840
CACCCCCGACTTCCCACCATCAAATTCATAACCGACCCTCCAATCATCCCGAACGTTCCATTTAACAGTG
       850       860       870       880       890       900       910
CCCCCGTCCCCCGTCAATTACAACGCCCCTTCACCTTCAACGTCTTCGACCCACCCCAGTTCCTTCCCATAT
       920       930       940       950       960       970       980
TCCCCTCCGCATACCCCTCGAAAGCACTTCTCCTTCCCACCCCTATCACCCGATCGTCGACCAACGCCCA
       990      1000      1010      1020      1030      1040      1050
ATCCCCCCCACCCCTTTTATCATCGTCGCCCAATCGCCACGAGCCATTTCGTTTCCAATTCTCCCCCCACCA
      1060      1070      1080      1090      1100      1110      1120
CCATCCCAAACCACGTCCTCCACCCCGAAT.GTACCATCGTCCACACCAACCACTCCTTCCTTCACCACCG
      1130      1140      1150      1160      1170      1180      1190
CAAAAATCAGAAAGACCTCCATCCCTTACCGGACTCATCCAATCCCCACCACCGTATCGAGTTCCTCCTC
      1200      1210      1220      1230      1240      1250      1260
CACCCCTTCCACCGCCACCAAACACGCATTTCCCCACCTCTCGCCCCACGAACACAATTATCCCTTTACCA
      1270      1280      1290      1300      1310      1320      1330
TCTGCCCCCCCTTACGACGACGGGCAAGAGCCAGACGCCCGACTCTGTTCAATATCATCTACGACCATCCCCG
      1340      1350      1360      1370      1380      1390      1400
TACACACCCAACCGTCCCCCTTCCCCCCCCCCACCAACCCTCATCACATCTTTCTCATCCCGTTTCACCAC
      1410      1420      1430      1440      1450      1460      1470
CACCACGACACGTCTCCCCTCAACGCCCACCCTTTCAACCCTCTTCATGACGACCCAATCCATCTTTTGTA
      1480      1490      1500      1510      1520      1530      1540
TCTACCTTCAACCCACTCCGTCTTCACTTCTTCCCCCCGCACTCCCTACCGTTTGTACCATCTCACTCATA
      1550      1560      1570      1580      1590      1600      1610
TAAATCTCTACCCCCTACCTACACTATACCTAACGCAGACAACCGTACAGTCATTAACGTACGCCCCTAT
```

71

```
      1620      1630      1640      1650      1660      1670      1680
AGTACCCCCATCTCTAGATAGAACATTTAGTAGACATTACGATCCCTAACTAATTTAACTTCACCATTGT
      1690      1700      1710      1720      1730      1740      1750
CCCGTTCATATTCATTTTCATCCATTATACACTCTTAATCGTTTCCCCGTACAACCCGNATATATACCAC
      1760      1770      1780      1790      1800      1810      1820
CATACCGTGTCGAGAACACCCCTTCCCCGTCTCCTTGCCCCGTACTTAACCTATATATTCTACACCCCCAAT
      1830      1840      1850      1860      1870      1880      1890
ACTCAATGTCCCCTTACCACCTAAGCCCCACTCTACCGTAAGTCCCCGTCATATACGTCAGAACTCTTAA
      1900      1910      1920      1930      1940      1950      1960
CACTCAAGACACCATATCACCCCGTTACCCTCCACCGTACCTACTACCTTCAATATCACTCTTTCACCATC
      1970
CACACCGTCCAC.
```

**3.** Procédé selon la revendication 1 ou 2, dans lequel la séquence d'ADN comprend les séquences requises pour la transcription et la traduction du gène PAT de Pénicillium chrysogenum.

**4.** Procédé selon la revendication 3, dans lequel les séquences qui sont requises pour la transcription et la traduction du gène PAT de Penicillium chrysogenum sont les séquences entre le gène de l'isopénicilline N synthétase et le gène PAT de Penicillium chrysogenum.

**5.** Procédé pour la production d'un vecteur qui contient une séquence d'ADN qui code pour l'enzyme pénicillinacyltransférase (PAT), caractérisé en ce que la séquence d'ADN qui code pour l'enzyme pénicillinacyltransférase présentant la séquence suivante :

```
                      10                                      20
    MetLeuHisIleLeuCysGlnGlyThrProPheGluIleGlyTyrGluHisGlySerAla
                      30                                      40
    AlaLysAlaValIleAlaArgSerIleAspPheAlaValAspLeuIleArgGlyLysThr
                      50                                      60
    LysLysThrAspGluGluLeuLysGlnValLeuSerGlnLeuGlyArgValIleGluGlu
                      70                                      80
    ArgTrpProLysTyrTyrGluGluIleArgGlyIleAlaLysGlyAlaGluArgAspVal
                      90                                      100
    SerGluIleValMetLeuAsnThrArgThrGluPheAlaTyrGlyLeuLysAlaAlaArg
                      110                                     120
    AspGlyCysThrThrAlaTyrCysGlnLeuProAsnGlyAlaLeuGlnGlyGlnAsnTrp
                      130                                     140
    AspPhePheSerAlaThrLysGluAsnLeuIleArgLeuThrIleArgGlnAlaGlyLeu
                      150                                     160
    ProThrIleLysPheIleThrGluAlaGlyIleIleGlyLysValGlyPheAsnSerAla
                      170                                     180
    GlyValAlaValAsnTyrAsnAlaLeuHisLeuGlnGlyLeuArgProThrGlyValPro
                      190                                     200
    SerHisIleAlaLeuArgIleAlaLeuGluSerThrSerProSerGlnAlaTyrAspArg
```

72

```
                              210                                      220
         IleValGluGlnGlyGlyMetAlaAlaSerAlaPheIleMetValGlyAsnGlyHisGlu
                              230                                      240
         AlaPheGlyLeuGluPheSerProThrSerIleArgLysGlnValLeuAspAlaAsnGly
                              250                                      260
         ArgMetValHisThrAsnHisCysLeuLeuGlnHisGlyLysAsnGluLysGluLeuAsp
                              270                                      280
         ProLeuProAspSerTrpAsnArgHisGlnArgMetGluPheLeuLeuAspGlyPheAsp
                              290                                      300
         GlyThrLysGlnAlaPheAlaGlnLeuTrpAlaAspGluAspAsnTyrProPheSerIle

                              310                                      320
         CysArgAlaTyrGluGluGlyLysSerArgGlyAlaThrLeuPheAsnIleIleTyrAsp
                              330                                      340
         HisAlaArgArgGluAlaThrValArgLeuGlyArgProThrAsnProAspGluMetPhe
                              350
         ValMetArgPheAspGluGluAspGluArgSerAlaLeuAsnAlaArgLeu,
```

est liée à un vecteur par des méthodes de recombinaison.

6. Vecteur selon la revendication 5, dans lequel la séquence d'ADN, qui code pour l'enzyme pénicillinacyltransférase (PAT), comprend la séquence suivante :

```
      10        20        30        40        50        60        70
GTTGATGTCCCATCAGTGTCATGCTATCGTCCCAGATTCGTCCCTACCCCAATATAAATCTCACCATCCA
      80        90        100       110       120       130       140
GTTCCCCCTCCATGATCATCCCCACGACCCCGTTTGTCATCTCCGTCACCCACGTCTCAGTTGTTTACCC
      150       160       170       180       190       200       210
ATCTTCCGACCCCCACCAGAAATGCTTCACATCCTCTGTCAACCCACTCCCTTTCAAGTAAGTGCTCCAC
      220       230       240       250       260       270       280
TGAATACCAGATTTTTTTCCTTCTGAATCTTCCGAGTTCTCACCTGATCCACATCGCCTACCAACATCCCT
      290       300       310       320       330       340       350
CTCCTCCCAAACCCGTGATACCCAGAACCATTGACTTCCCCGTCGATCTCATCCGACGCAAAACGAAGAA
      360       370       380       390       400       410       420
GACGGACCAACAGCCTTAAACACGTACTCTCCCAACTCCCCCCGCGTGATCGACCAAAGATCCCCCAAATAC
      430       440       450       460       470       480       490
TACGACGACATTCCCCGTGAGTGCCCACTTCCGTCTTTCCTACATTTTTCTCCACCAATGCTGACCGATGAC
      500       510       520       530       540       550       560
CCCCGAAAAACCACGTATTCGAAAGCGCCCCTGAACCCCATGTCTCCCAGATTGTCATCGTTAATACCCCG
      570       580       590       600       610       620       630
ACCGAATTTCCATACCGCCTCAACCCAGCCCCGTCATCGCTGCACCACTCCCTATTGTCAACTTCCAAATG
      640       650       660       670       680       690       700
CACCCCTCCACGCCCCAAAACTCGGATGTACGTTAAGACATTTTACCTCCTCATTTTATTCCATCCAATTT
      710       720       730       740       750       760       770
CCCCCCACTAATTTCGTTGTTCAAGTTCTTTTTCTCCCACCAAACAGAACCTGATCCCGTTAACGATCCGT
```

```
           780        790        800        810        820        830        840
CACCCCCGACTTCCCACCATCAAATTCATAACCGACCCTCGAATCATCCCCAACCTTCCATTTAACAGTC


           850        860        870        880        890        900        910
CCCCCGGTCCCCGTCAATTACAACCCCCTTCACCTTCAACGTCTTCGACCCACCCGAGTTCCTTCCCATAT
           920        930        940        950        960        970        980
TCCCCTCCGCATACCCCTCGAAACCACTTCTCCTTCCCACCCCTATGACCCGATCGTCGACCAACCCCGA
           990       1000       1010       1020       1030       1040       1050
ATCCCCCCCACCCGCTTTTATCATCGTCGCCCAATCGCCCACCACCCATTTCGTTTCGAATTCTCCCCCACCA
          1060       1070       1080       1090       1100       1110       1120
GCATCCCAAACCACGTCCTCCACCCCAAT.GTACCATCGTCCACACCAACCACTCCTTCCTTCACCACCG
          1130       1140       1150       1160       1170       1180       1190
CAAAAATCACAAAGACCTCGATCCCTTACCCGACTCATCGAATCCCCACCACCGTATCCACTTCCTCCTC
          1200       1210       1220       1230       1240       1250       1260
CACCCGTTCCACCCCACCAAACACGCATTTCCCCACCTCTCCCCCCGACCAACACAATTATCCCTTTACCA
          1270       1280       1290       1300       1310       1320       1330
TCTCCCCCCCTTACCACCGACCCCAAGACCCAGACCCCCCGACTCTGTTCAATATCATCTACCACCATCCCCG
          1340       1350       1360       1370       1380       1390       1400
TACACACCCAACCGTCCCCCCTTCGCCCCGCCCACCAACCCTCATCACGATCTTTCTCATCCCCTTTCACCAG


          1410       1420       1430       1440       1450       1460       1470
CACCACCACACGTCTCCCCTCAACCCCACCCTTTCAACCCTCTTCATCACCACCCAATCCATCTTTTGTA
          1480       1490       1500       1510       1520       1530       1540
TGTACCTTCAACCGACTCCGTCTTCACTTCTTCCCCCCCACTCCCTACCGTTTCTACCATCTCACTCATA
          1550       1560       1570       1580       1590       1600       1610
TAAATCTCTACCCCCTACCTACACTATACCTAACGGACACAACCGTACAGTCATTAACGTACCCCCCTAT
          1620       1630       1640       1650       1660       1670       1680
AGTACCCCGATCTCTAGATAGAACATTTAGTAGAGATTACCATCCCTAACTAATTTAACTTCACCATTCT
          1690       1700       1710       1720       1730       1740       1750
CCCGTTCATATTCATTTTCATCCATTATACACTCTTAATCGTTTCCCCGTACAACCCCGATATATACCAC
          1760       1770       1780       1790       1800       1810       1820
CATACCGTCTCGACAACACCCCTTCCCGTCTCCTTCCCCGTACTTAACCTATATATTCTACACCCCCAAT
          1830       1840       1850       1860       1870       1880       1890
ACTCAATCTCCCCTTACCACCTAACCCCCACTCTACCGTAAGTCCCCGTCATATACGTCAGAAGTCTTAA
          1900       1910       1920       1930       1940       1950       1960
GACTCAACACACCATATCACCCCGTTACCCTCCACCGTACCTACTACCTTCAATATCACTCTTTCACCATG
          1970
CACACCGTCCAC•
```

**7.** Procédé selon la revendication 5 ou 6, dans lequel le vecteur contient d'autres gènes qui sont puis pour la transcription et la traduction du gène PAT de Penicillium chrysogenum.

**8.** Procédé selon l'une quelconque des revendications 5 à 7, dans lequel le vecteur contient d'autres gènes qui permettent une sélection du plasmide dans des micro-organismes producteurs de β-lactames.

**9.** Procédé selon l'une quelconque des revendications 5 à 8, dans lequel le vecteur contient un autre gène qui code pour l'enzyme isopénicilline N synthétase.

**10.** Procédé pour la production de l'enzyme pénicillinacyltransférase (PAT), consistant en la transformation d'un hôte à l'aide d'un vecteur tel que défini à l'une quelconque des revendications 6 à 9 et l'isolement de la PAT exprimée.

11. Procédé pour la production de cellules contenant un vecteur tel que défini à l'une quelconque des revendications 5 à 9, caractérisé en ce qu'on transforme les cellules avec un vecteur tel que défini à l'une quelconque des revendications 5 à 9.

12. Procédé pour l'amélioration de la production d'une pénicilline, caractérisé en ce qu'on transforme une cellule productrice de pénicilline avec un vecteur tel que défini à l'une quelconque des revendications 5 à 9, et on isole la pénicilline.